# EUROPEAN PATENT SPECIFICATION

(11) **EP 1 912 677 B1**
(45) Date of publication and mention of the grant of the patent: **02.10.2013**
(21) Application number: 06785283.0
(22) Date of filing: 20.06.2006
(51) Int. Cl.: A61K 47/48, A61P 35/00

(54) **PSMA antibody-drug conjugates**
PSMA-Antikörper-Arzneimittel-Konjugate
Conjugués anticorps PSMA-médicament

(30) Priority: 20.06.2005 US 692399 P; 14.04.2006 US 792360 P
(43) Date of publication of application: 23.04.2008
(73) Proprietor: Psma Development Company, L.L.C., Tarrytown, New York 10591 (US)
(72) Inventor: MA, Dangshe, Millwood, NY 10546 (US); MADDON, Paul, J., Scarsdale, NY 10583 (US); OLSON, William, C., Ossining, NY 10562 (US); DORONINA, Svetlana, O., Snohomish, WA 98296 (US); TOKI, Brian, E., Shoreline, WA 98155 (US); SENTER, Peter, D., Seattle, WA 98115 (US)
(74) Representative: Jump, Timothy John Simon
(86) International application number: PCT/US2006/024182
(87) International publication number: WO 2007/002222

(56) References cited:
- EP-A2- 0 624 377
- WO-A-2005/001038
- WO-A2-03/034903
- WO-A2-2004/067564
- DORONINA S O ET AL: "Development of potent monoclonal antibody auristatin conjugates for cancer therapy" NATURE BIOTECHNOLOGY, NATURE PUBLISHING GROUP, NEW YORK, NY, US, vol. 21, no. 7, July 2003 (2003-07), pages 778-784, XP002280966 ISSN: 1087-0156 cited in the application
- DORONINA S ET AL: "IMMUNOCONJUGATES COMPRISED OF DRUGS WITH IMPAIRED CELLULAR PERMEABILITY: A NEW APPROACH TO TARGETED THERAPY", ABSTRACTS OF PAPERS. ACS NATIONAL MEETING, XX, XX, vol. 228, no. PART 01, 1 August 2004 (2004-08-01), page U908, XP009066098,
- FRANCISCO JOSEPH A ET AL: "cAC10-vcMMAE, an anti-CD30-monomethyl auristatin E conjugate with potent and selective antitumor activity.", BLOOD 15 AUG 2003 LNKD- PUBMED:12714494, vol. 102, no. 4, 15 August 2003 (2003-08-15), pages 1458-1465, ISSN: 0006-4971
- BHASKAR V ET AL: "E-selectin up-regulation allows for targeted drug delivery in prostate cancer", CANCER RESEARCH, AMERICAN ASSOCIATION FOR CANCER REREARCH, US, vol. 63, no. 19, 1 October 2003 (2003-10-01), pages 6387-6394, XP002280964, ISSN: 0008-5472
- D. P. PETRYLAK, P. W. KANTOFF, Y. ROTSHTEYN, R. J. ISRAEL, W. C. OLSON, T. RAMAKRISHNA, S. MORRIS: "Prostate-specific membrane antigen antibody drug conjugate (PSMA ADC): A phase I trial in taxane-refractory prostate cancer.", J CLIN ONCOL, no. 7, 158 ,
- NAGATA SATOSHI ET AL: "Cell membrane-specific epitopes on CD30: Potentially superior targets for immunotherapy", PROCEEDINGS OF THE NATIONAL ACADEMY OF SCIENCES, NATIONAL ACADEMY OF SCIENCES, US, vol. 102, no. 22, 1 May 2005 (2005-05-01), pages 7946-7951, XP002436245, ISSN: 0027-8424, DOI: 10.1073/PNAS.0502975102

### SEQUENCE LISTING

<110> PSMA DEVELOPMENT COMPANY, L.L.C.
   MA, Dangshe
   MADDON, Paul J.
   OLSON, William C.
   DORONINA, Svetlana
   TOKI, Brian
   SENTER, Peter
<120> PSMA ANTIBODY-DRUG CONJUGATES
<130> P0741.70008W000
<150> US 60/792,360
   <151> 2006-04-14
<150> US 60/692,399
   <151> 2005-06-20
<160> 33
<170> PatentIn version 3.1
<210> 1
   <211> 750
   <212> PRT
   <213> Homo sapiens
<400> 1
   Met Trp Asn Leu Leu His Glu Thr Asp Ser Ala Val Ala Thr Ala Arg
   1               5                   10                  15
   Arg Pro Arg Trp Leu Cys Ala Gly Ala Leu Val Leu Ala Gly Gly Phe
               20                  25                  30
   Phe Leu Leu Gly Phe Leu Phe Gly Trp Phe Ile Lys Ser Ser Asn Glu
           35                  40                  45
   Ala Thr Asn Ile Thr Pro Lys His Asn Met Lys Ala Phe Leu Asp Glu
       50                  55                  60
   Leu Lys Ala Glu Asn Ile Lys Lys Phe Leu Tyr Asn Phe Thr Gln Ile
   65                  70                  75                  80
   Pro His Leu Ala Gly Thr Glu Gln Asn Phe Gln Leu Ala Lys Gln Ile
                   85                  90                  95
   Gln Ser Gln Trp Lys Glu Phe Gly Leu Asp Ser Val Glu Leu Ala His
               100                 105                 110
   Tyr Asp Val Leu Leu Ser Tyr Pro Asn Lys Thr His Pro Asn Tyr Ile
           115                 120                 125
   Ser Ile Ile Asn Glu Asp Gly Asn Glu Ile Phe Asn Thr Ser Leu Phe
       130                 135                 140
   Glu Pro Pro Pro Pro Gly Tyr Glu Asn Val Ser Asp Ile Val Pro Pro
   145                 150                 155                 160
   Phe Ser Ala Phe Ser Pro Gln Gly Met Pro Glu Gly Asp Leu Val Tyr
                   165                 170                 175
   Val Asn Tyr Ala Arg Thr Glu Asp Phe Phe Lys Leu Glu Arg Asp Met
               180                 185                 190
   Lys Ile Asn Cys Ser Gly Lys Ile Val Ile Ala Arg Tyr Gly Lys Val
           195                 200                 205
   Phe Arg Gly Asn Lys Val Lys Asn Ala Gln Leu Ala Gly Ala Lys Gly
       210                 215                 220
   Val Ile Leu Tyr Ser Asp Pro Ala Asp Tyr Phe Ala Pro Gly Val Lys
   225                 230                 235                 240
   Ser Tyr Pro Asp Gly Trp Asn Leu Pro Gly Gly Gly Val Gln Arg Gly
                   245                 250                 255
   Asn Ile Leu Asn Leu Asn Gly Ala Gly Asp Pro Leu Thr Pro Gly Tyr
               260                 265                 270
   Pro Ala Asn Glu Tyr Ala Tyr Arg Arg Gly Ile Ala Glu Ala Val Gly
           275                 280                 285
   Leu Pro Ser Ile Pro Val His Pro Ile Gly Tyr Tyr Asp Ala Gln Lys
       290                 295                 300
   Leu Leu Glu Lys Met Gly Gly Ser Ala Pro Pro Asp Ser Ser Trp Arg
   305 310 315 320
   Gly Ser Leu Lys Val Pro Tyr Asn Val Gly Pro Gly Phe Thr Gly Asn
   325 330 335
   Phe Ser Thr Gln Lys Val Lys Met His Ile His Ser Thr Asn Glu Val
   340 345 350
   Thr Arg Ile Tyr Asn Val Ile Gly Thr Leu Arg Gly Ala Val Glu Pro
   355 360 365
   Asp Arg Tyr Val Ile Leu Gly Gly His Arg Asp Ser Trp Val Phe Gly
   370 375 380
   Gly Ile Asp Pro Gln Ser Gly Ala Ala Val Val His Glu Ile Val Arg
   385 390 395 400
   Ser Phe Gly Thr Leu Lys Lys Glu Gly Trp Arg Pro Arg Arg Thr Ile
   405 410 415
   Leu Phe Ala Ser Trp Asp Ala Glu Glu Phe Gly Leu Leu Gly Ser Thr
   420 425 430
   Glu Trp Ala Glu Glu Asn Ser Arg Leu Leu Gln Glu Arg Gly Val Ala
   435 440 445
   Tyr Ile Asn Ala Asp Ser Ser Ile Glu Gly Asn Tyr Thr Leu Arg Val
   450 455 460
   Asp Cys Thr Pro Leu Met Tyr Ser Leu Val His Asn Leu Thr Lys Glu
   465 470 475 480
   Leu Lys Ser Pro Asp Glu Gly Phe Glu Gly Lys Ser Leu Tyr Glu Ser
   485 490 495
   Trp Thr Lys Lys Ser Pro Ser Pro Glu Phe Ser Gly Met Pro Arg Ile
   500 505 510
   Ser Lys Leu Gly Ser Gly Asn Asp Phe Glu Val Phe Phe Gln Arg Leu
   515 520 525
   Gly Ile Ala Ser Gly Arg Ala Arg Tyr Thr Lys Asn Trp Glu Thr Asn
   530 535 540
   Lys Phe Ser Gly Tyr Pro Leu Tyr His Ser Val Tyr Glu Thr Tyr Glu
   545 550 555 560
   Leu Val Glu Lys Phe Tyr Asp Pro Met Phe Lys Tyr His Leu Thr Val
   565 570 575
   Ala Gln Val Arg Gly Gly Met Val Phe Glu Leu Ala Asn Ser Ile Val
   580 585 590
   Leu Pro Phe Asp Cys Arg Asp Tyr Ala Val Val Leu Arg Lys Tyr Ala
   595 600 605
   Asp Lys Ile Tyr Ser Ile Ser Met Lys His Pro Gln Glu Met Lys Thr
   610 615 620
   Tyr Ser Val Ser Phe Asp Ser Leu Phe Ser Ala Val Lys Asn Phe Thr
   625 630 635 640
   Glu Ile Ala Ser Lys Phe Ser Glu Arg Leu Gln Asp Phe Asp Lys Ser
   645 650 655
   Asn Pro Ile Val Leu Arg Met Met Asn Asp Gln Leu Met Phe Leu Glu
   660 665 670
   Arg Ala Phe Ile Asp Pro Leu Gly Leu Pro Asp Arg Pro Phe Tyr Arg
   675 680 685
   His Val Ile Tyr Ala Pro Ser Ser His Asn Lys Tyr Ala Gly Glu Ser
   690 695 700
   Phe Pro Gly Ile Tyr Asp Ala Leu Phe Asp Ile Glu Ser Lys Val Asp
   705 710 715 720
   Pro Ser Lys Ala Trp Gly Glu Val Lys Arg Gln Ile Tyr Val Ala Ala
   725 730 735
   Phe Thr Val Gln Ala Ala Ala Glu Thr Leu Ser Glu Val Ala
   740 745 750
<210> 2
   <211> 7570
   <212> DNA
   <213> Artificial Sequence
<220>
   <223> Plasmid
<400> 2
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   ggtaccaagc ttggatctca ccatggagtt gggactgcgc tggggcttcc tcgttgctct 960
   tttaagaggt gtccagtgtc aggtgcaatt ggtggagtct gggggaggcg tggtccagcc 1020
   tgggaggtcc ctgagactct cctgtgcagc gtctggattc gccttcagta gatatggcat 1080
   gcactgggtc cgccaggctc caggcaaggg gctggagtgg gtggcagtta tatggtatga 1140
   tggaagtaat aaatactatg cagactccgt gaagggccga ttcaccatct ccagagacaa 1200
   ttccaagaac acgcagtatc tgcaaatgaa cagcctgaga gccgaggaca cggctgtgta 1260
   ttactgtgcg agaggcggtg acttcctcta ctactactat tacggtatgg acgtctgggg 1320
   ccaagggacc acggtcaccg tctcctcagc ctccaccaag ggcccatcgg tcttccccct 1380
   ggcaccctct agcaagagca cctctggggg cacagcggcc ctgggctgcc tggtcaagga 1440
   ctacttcccc gaaccggtga cggtgtcgtg gaactcaggc gccctgacca gcggcgtgca 1500
   caccttcccg gctgtcctac agtcctcagg actctactcc ctcagcagcg tggtgaccgt 1560
   gccctccagc agcttgggca cccagaccta catctgcaac gtgaatcaca agcccagcaa 1620
   caccaaggtg gacaagagag ttggtgagag gccagcacag ggagggaggg tgtctgctgg 1680
   aagccaggct cagcgctcct gcctggacgc atcccggcta tgcagtccca gtccagggca 1740
   gcaaggcagg ccccgtctgc ctcttcaccc ggaggcctct gcccgcccca ctcatgctca 1800
   gggagagggt cttctggctt tttccccagg ctctgggcag gcacaggcta ggtgccccta 1860
   acccaggccc tgcacacaaa ggggcaggtg ctgggctcag acctgccaag agccatatcc 1920
   gggaggaccc tgcccctgac ctaagcccac cccaaaggcc aaactctcca ctccctcagc 1980
   tcggacacct tctctcctcc cagattccag taactcccaa tcttctctct gcagagccca 2040
   aatcttgtga caaaactcac acatgcccac cgtgcccagg taagccagcc caggcctcgc 2100
   cctccagctc aaggcgggac aggtgcccta gagtagcctg catccaggga caggccccag 2160
   ccgggtgctg acacgtccac ctccatctct tcctcagcac ctgaactcct ggggggaccg 2220
   tcagtcttcc tcttcccccc aaaacccaag gacaccctca tgatctcccg gacccctgag 2280
   gtcacatgcg tggtggtgga cgtgagccac gaagaccctg aggtcaagtt caactggtac 2340
   gtggacggcg tggaggtgca taatgccaag acaaagccgc gggaggagca gtacaacagc 2400
   acgtaccgtg tggtcagcgt cctcaccgtc ctgcaccagg actggctgaa tggcaaggag 2460
   tacaagtgca aggtctccaa caaagccctc ccagccccca tcgagaaaac catctccaaa 2520
   gccaaaggtg ggacccgtgg ggtgcgaggg ccacatggac agaggccggc tcggcccacc 2580
   ctctgccctg agagtgaccg ctgtaccaac ctctgtccct acagggcagc cccgagaacc 2640
   acaggtgtac accctgcccc catcccggga ggagatgacc aagaaccagg tcagcctgac 2700
   ctgcctggtc aaaggcttct atcccagcga catcgccgtg gagtgggaga gcaatgggca 2760
   gccggagaac aactacaaga ccacgcctcc cgtgctggac tccgacggct ccttcttcct 2820
   ctatagcaag ctcaccgtgg acaagagcag gtggcagcag gggaacgtct tctcatgctc 2880
   cgtgatgcat gaggctctgc acaaccacta cacgcagaag agcctctccc tgtctccggg 2940
   taaatgagaa ttcctcgagt ctagagggcc cgtttaaacc cgctgatcag cctcgactgt 3000
   gccttctagt tgccagccat ctgttgtttg cccctccccc gtgccttcct tgaccctgga 3060
   aggtgccact cccactgtcc tttcctaata aaatgaggaa attgcatcgc attgtctgag 3120
   taggtgtcat tctattctgg ggggtggggt ggggcaggac agcaaggggg aggattggga 3180
   agacaatagc aggcatgctg gggatgcggt gggctctatg gcttctgagg cggaaagaac 3240
   cagctggggc tctagggggt atccccacgc gccctgtagc ggcgcattaa gcgcggcggg 3300
   tgtggtggtt acgcgcagcg tgaccgctac acttgccagc gccctagcgc ccgctccttt 3360
   cgctttcttc ccttcctttc tcgccacgtt cgccggcttt ccccgtcaag ctctaaatcg 3420
   gggcatccct ttagggttcc gatttagtgc tttacggcac ctcgacccca aaaaacttga 3480
   ttagggtgat ggttcacgta gtgggccatc gccctgatag acggtttttc gccctttgac 3540
   gttggagtcc acgttcttta atagtggact cttgttccaa actggaacaa cactcaaccc 3600
   tatctcggtc tattcttttg atttataagg gattttgggg atttcggcct attggttaaa 3660
   aaatgagctg atttaacaaa aatttaacgc gaattaattc tgtggaatgt gtgtcagtta 3720
   gggtgtggaa agtccccagg ctccccaggc aggcagaagt atgcaaagca tgcatctcaa 3780
   ttagtcagca accaggtgtg gaaagtcccc aggctcccca gcaggcagaa gtatgcaaag 3840
   catgcatctc aattagtcag caaccatagt cccgccccta actccgccca tcccgcccct 3900
   aactccgccc agttccgccc attctccgcc ccatggctga ctaatttttt ttatttatgc 3960
   agaggccgag gccgcctctg cctctgagct attccagaag tagtgaggag gcttttttgg 4020
   aggcctaggc ttttgcaaaa agctcccggg agcttgtata tccattttcg gatctgatca 4080
   gcacgtgatg aaaaagcctg aactcaccgc gacgtctgtc gagaagtttc tgatcgaaaa 4140
   gttcgacagc gtctccgacc tgatgcagct ctcggagggc gaagaatctc gtgctttcag 4200
   cttcgatgta ggagggcgtg gatatgtcct gcgggtaaat agctgcgccg atggtttcta 4260
   caaagatcgt tatgtttatc ggcactttgc atcggccgcg ctcccgattc cggaagtgct 4320
   tgacattggg gaattcagcg agagcctgac ctattgcatc tcccgccgtg cacagggtgt 4380
   cacgttgcaa gacctgcctg aaaccgaact gcccgctgtt ctgcagccgg tcgcggaggc 4440
   catggatgcg atcgctgcgg ccgatcttag ccagacgagc gggttcggcc cattcggacc 4500
   gcaaggaatc ggtcaataca ctacatggcg tgatttcata tgcgcgattg ctgatcccca 4560
   tgtgtatcac tggcaaactg tgatggacga caccgtcagt gcgtccgtcg cgcaggctct 4620
   cgatgagctg atgctttggg ccgaggactg ccccgaagtc cggcacctcg tgcacgcgga 4680
   tttcggctcc aacaatgtcc tgacggacaa tggccgcata acagcggtca ttgactggag 4740
   cgaggcgatg ttcggggatt cccaatacga ggtcgccaac atcttcttct ggaggccgtg 4800
   gttggcttgt atggagcagc agacgcgcta cttcgagcgg aggcatccgg agcttgcagg 4860
   atcgccgcgg ctccgggcgt atatgctccg cattggtctt gaccaactct atcagagctt 4920
   ggttgacggc aatttcgatg atgcagcttg ggcgcagggt cgatgcgacg caatcgtccg 4980
   atccggagcc gggactgtcg ggcgtacaca aatcgcccgc agaagcgcgg ccgtctggac 5040
   cgatggctgt gtagaagtac tcgccgatag tggaaaccga cgccccagca ctcgtccgag 5100
   ggcaaaggaa tagcacgtgc tacgagattt cgattccacc gccgccttct atgaaaggtt 5160
   gggcttcgga atcgttttcc gggacgccgg ctggatgatc ctccagcgcg gggatctcat 5220
   gctggagttc ttcgcccacc ccaacttgtt tattgcagct tataatggtt acaaataaag 5280
   caatagcatc acaaatttca caaataaagc atttttttca ctgcattcta gttgtggttt 5340
   gtccaaactc atcaatgtat cttatcatgt ctgtataccg tcgacctcta gctagagctt 5400
   ggcgtaatca tggtcatagc tgtttcctgt gtgaaattgt tatccgctca caattccaca 5460
   caacatacga gccggaagca taaagtgtaa agcctggggt gcctaatgag tgagctaact 5520
   cacattaatt gcgttgcgct cactgcccgc tttccagtcg ggaaacctgt cgtgccagct 5580
   gcattaatga atcggccaac gcgcggggag aggcggtttg cgtattgggc gctcttccgc 5640
   ttcctcgctc actgactcgc tgcgctcggt cgttcggctg cggcgagcgg tatcagctca 5700
   ctcaaaggcg gtaatacggt tatccacaga atcaggggat aacgcaggaa agaacatgtg 5760
   agcaaaaggc cagcaaaagg ccaggaaccg taaaaaggcc gcgttgctgg cgtttttcca 5820
   taggctccgc ccccctgacg agcatcacaa aaatcgacgc tcaagtcaga ggtggcgaaa 5880
   cccgacagga ctataaagat accaggcgtt tccccctgga agctccctcg tgcgctctcc 5940
   tgttccgacc ctgccgctta ccggatacct gtccgccttt ctcccttcgg gaagcgtggc 6000
   gctttctcaa tgctcacgct gtaggtatct cagttcggtg taggtcgttc gctccaagct 6060
   gggctgtgtg cacgaacccc ccgttcagcc cgaccgctgc gccttatccg gtaactatcg 6120
   tcttgagtcc aacccggtaa gacacgactt atcgccactg gcagcagcca ctggtaacag 6180
   gattagcaga gcgaggtatg taggcggtgc tacagagttc ttgaagtggt ggcctaacta 6240
   cggctacact agaaggacag tatttggtat ctgcgctctg ctgaagccag ttaccttcgg 6300
   aaaaagagtt ggtagctctt gatccggcaa acaaaccacc gctggtagcg gtggtttttt 6360
   tgtttgcaag cagcagatta cgcgcagaaa aaaaggatct caagaagatc ctttgatctt 6420
   ttctacgggg tctgacgctc agtggaacga aaactcacgt taagggattt tggtcatgag 6480
   attatcaaaa aggatcttca cctagatcct tttaaattaa aaatgaagtt ttaaatcaat 6540
   ctaaagtata tatgagtaaa cttggtctga cagttaccaa tgcttaatca gtgaggcacc 6600
   tatctcagcg atctgtctat ttcgttcatc catagttgcc tgactccccg tcgtgtagat 6660
   aactacgata cgggagggct taccatctgg ccccagtgct gcaatgatac cgcgagaccc 6720
   acgctcaccg gctccagatt tatcagcaat aaaccagcca gccggaaggg ccgagcgcag 6780
   aagtggtcct gcaactttat ccgcctccat ccagtctatt aattgttgcc gggaagctag 6840
   agtaagtagt tcgccagtta atagtttgcg caacgttgtt gccattgcta caggcatcgt 6900
   ggtgtcacgc tcgtcgtttg gtatggcttc attcagctcc ggttcccaac gatcaaggcg 6960
   agttacatga tcccccatgt tgtgcaaaaa agcggttagc tccttcggtc ctccgatcgt 7020
   tgtcagaagt aagttggccg cagtgttatc actcatggtt atggcagcac tgcataattc 7080
   tcttactgtc atgccatccg taagatgctt ttctgtgact ggtgagtact caaccaagtc 7140
   attctgagaa tagtgtatgc ggcgaccgag ttgctcttgc ccggcgtcaa tacgggataa 7200
   taccgcgcca catagcagaa ctttaaaagt gctcatcatt ggaaaacgtt cttcggggcg 7260
   aaaactctca aggatcttac cgctgttgag atccagttcg atgtaaccca ctcgtgcacc 7320
   caactgatct tcagcatctt ttactttcac cagcgtttct gggtgagcaa aaacaggaag 7380
   gcaaaatgcc gcaaaaaagg gaataagggc gacacggaaa tgttgaatac tcatactctt 7440
   cctttttcaa tattattgaa gcatttatca gggttattgt ctcatgagcg gatacatatt 7500
   tgaatgtatt tagaaaaata aacaaatagg ggttccgcgc acatttcccc gaaaagtgcc 7560
   acctgacgtc 7570
<210> 3
   <211> 7597
   <212> DNA
   <213> Artificial Sequence
<220>
   <223> Plasmid
<400> 3
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   ggtaccaagc ttggatctca ccatggggtc aaccgccatc ctcaccatgg agttggggct 960
   gcgctgggtt ctcctcgttg ctcttttaag aggtgtccag tgtcaggtgc agctggtgga 1020
   gtctggggga ggcgtggtcc agcctgggag gtccctgaga ctctcctgtg cagcgtctgg   1080
   attcaccttc agtaactatg tcatgcactg ggtccgccag gctccaggca aggggctgga   1140
   gtgggtggca attatatggt atgatggaag taataaatac tatgcagact ccgtgaaggg   1200
   ccgattcacc atctccagag acaattccaa gaacacgctg tatctgcaaa tgaacagcct   1260
   gagagccgag gacacggctg tgtattactg tgcgggtgga tataactgga actacgagta   1320
   ccactactac ggtatggacg tctggggcca agggaccacg gtcaccgtct cctcagcctc   1380
   caccaagggc ccatcggtct tccccctggc accctctagc aagagcacct ctgggggcac   1440
   agcggccctg ggctgcctgg tcaaggacta cttccccgaa ccggtgacgg tgtcgtggaa   1500
   ctcaggcgcc ctgaccagcg gcgtgcacac cttcccggct gtcctacagt cctcaggact   1560
   ctactccctc agcagcgtgg tgaccgtgcc ctccagcagc ttgggcaccc agacctacat   1620
   ctgcaacgtg aatcacaagc ccagcaacac caaggtggac aagagagttg gtgagaggcc   1680
   agcacaggga gggagggtgt ctgctggaag ccaggctcag cgctcctgcc tggacgcatc   1740
   ccggctatgc agtcccagtc cagggcagca aggcaggccc cgtctgcctc ttcacccgga   1800
   ggcctctgcc cgccccactc atgctcaggg agagggtctt ctggcttttt ccccaggctc   1860
   tgggcaggca caggctaggt gcccctaacc caggccctgc acacaaaggg gcaggtgctg   1920
   ggctcagacc tgccaagagc catatccggg aggaccctgc ccctgaccta agcccacccc   1980
   aaaggccaaa ctctccactc cctcagctcg gacaccttct ctcctcccag attccagtaa   2040
   ctcccaatct tctctctgca gagcccaaat cttgtgacaa aactcacaca tgcccaccgt   2100
   gcccaggtaa gccagcccag gcctcgccct ccagctcaag gcgggacagg tgccctagag   2160
   tagcctgcat ccagggacag gccccagccg ggtgctgaca cgtccacctc catctcttcc   2220
   tcagcacctg aactcctggg gggaccgtca gtcttcctct tccccccaaa acccaaggac   2280
   accctcatga tctcccggac ccctgaggtc acatgcgtgg tggtggacgt gagccacgaa   2340
   gaccctgagg tcaagttcaa ctggtacgtg gacggcgtgg aggtgcataa tgccaagaca   2400
   aagccgcggg aggagcagta caacagcacg taccgtgtgg tcagcgtcct caccgtcctg   2460
   caccaggact ggctgaatgg caaggagtac aagtgcaagg tctccaacaa agccctccca   2520
   gcccccatcg agaaaaccat ctccaaagcc aaaggtggga cccgtggggt gcgagggcca   2580
   catggacaga ggccggctcg gcccaccctc tgccctgaga gtgaccgctg taccaacctc   2640
   tgtccctaca gggcagcccc gagaaccaca ggtgtacacc ctgcccccat cccgggagga   2700
   gatgaccaag aaccaggtca gcctgacctg cctggtcaaa ggcttctatc ccagcgacat   2760
   cgccgtggag tgggagagca atgggcagcc ggagaacaac tacaagacca cgcctcccgt   2820
   gctggactcc gacggctcct tcttcctcta tagcaagctc accgtggaca agagcaggtg   2880
   gcagcagggg aacgtcttct catgctccgt gatgcatgag gctctgcaca accactacac   2940
   gcagaagagc ctctccctgt ctccgggtaa atgagaattc ctcgagtcta gagggcccgt   3000
   ttaaacccgc tgatcagcct cgactgtgcc ttctagttgc cagccatctg ttgtttgccc   3060
   ctcccccgtg ccttccttga ccctggaagg tgccactccc actgtccttt cctaataaaa   3120
   tgaggaaatt gcatcgcatt gtctgagtag gtgtcattct attctggggg gtggggtggg   3180
   gcaggacagc aagggggagg attgggaaga caatagcagg catgctgggg atgcggtggg   3240
   ctctatggct tctgaggcgg aaagaaccag ctggggctct agggggtatc cccacgcgcc   3300
   ctgtagcggc gcattaagcg cggcgggtgt ggtggttacg cgcagcgtga ccgctacact   3360
   tgccagcgcc ctagcgcccg ctcctttcgc tttcttccct tcctttctcg ccacgttcgc   3420
   cggctttccc cgtcaagctc taaatcgggg catcccttta gggttccgat ttagtgcttt   3480
   acggcacctc gaccccaaaa aacttgatta gggtgatggt tcacgtagtg ggccatcgcc   3540
   ctgatagacg gtttttcgcc ctttgacgtt ggagtccacg ttctttaata gtggactctt   3600
   gttccaaact ggaacaacac tcaaccctat ctcggtctat tcttttgatt tataagggat   3660
   tttggggatt tcggcctatt ggttaaaaaa tgagctgatt taacaaaaat ttaacgcgaa   3720
   ttaattctgt ggaatgtgtg tcagttaggg tgtggaaagt ccccaggctc cccaggcagg   3780
   cagaagtatg caaagcatgc atctcaatta gtcagcaacc aggtgtggaa agtccccagg   3840
   ctccccagca ggcagaagta tgcaaagcat gcatctcaat tagtcagcaa ccatagtccc   3900
   gcccctaact ccgcccatcc cgcccctaac tccgcccagt tccgcccatt ctccgcccca   3960
   tggctgacta atttttttta tttatgcaga ggccgaggcc gcctctgcct ctgagctatt   4020
   ccagaagtag tgaggaggct tttttggagg cctaggcttt tgcaaaaagc tcccgggagc   4080
   ttgtatatcc attttcggat ctgatcagca cgtgatgaaa aagcctgaac tcaccgcgac   4140
   gtctgtcgag aagtttctga tcgaaaagtt cgacagcgtc tccgacctga tgcagctctc   4200
   ggagggcgaa gaatctcgtg ctttcagctt cgatgtagga gggcgtggat atgtcctgcg   4260
   ggtaaatagc tgcgccgatg gtttctacaa agatcgttat gtttatcggc actttgcatc   4320
   ggccgcgctc ccgattccgg aagtgcttga cattggggaa ttcagcgaga gcctgaccta   4380
   ttgcatctcc cgccgtgcac agggtgtcac gttgcaagac ctgcctgaaa ccgaactgcc   4440
   cgctgttctg cagccggtcg cggaggccat ggatgcgatc gctgcggccg atcttagcca   4500
   gacgagcggg ttcggcccat tcggaccgca aggaatcggt caatacacta catggcgtga   4560
   tttcatatgc gcgattgctg atccccatgt gtatcactgg caaactgtga tggacgacac   4620
   cgtcagtgcg tccgtcgcgc aggctctcga tgagctgatg ctttgggccg aggactgccc   4680
   cgaagtccgg cacctcgtgc acgcggattt cggctccaac aatgtcctga cggacaatgg 4740
   ccgcataaca gcggtcattg actggagcga ggcgatgttc ggggattccc aatacgaggt 4800
   cgccaacatc ttcttctgga ggccgtggtt ggcttgtatg gagcagcaga cgcgctactt 4860
   cgagcggagg catccggagc ttgcaggatc gccgcggctc cgggcgtata tgctccgcat 4920
   tggtcttgac caactctatc agagcttggt tgacggcaat ttcgatgatg cagcttgggc 4980
   gcagggtcga tgcgacgcaa tcgtccgatc cggagccggg actgtcgggc gtacacaaat 5040
   cgcccgcaga agcgcggccg tctggaccga tggctgtgta gaagtactcg ccgatagtgg 5100
   aaaccgacgc cccagcactc gtccgagggc aaaggaatag cacgtgctac gagatttcga 5160
   ttccaccgcc gccttctatg aaaggttggg cttcggaatc gttttccggg acgccggctg 5220
   gatgatcctc cagcgcgggg atctcatgct ggagttcttc gcccacccca acttgtttat 5280
   tgcagcttat aatggttaca aataaagcaa tagcatcaca aatttcacaa ataaagcatt 5340
   tttttcactg cattctagtt gtggtttgtc caaactcatc aatgtatctt atcatgtctg 5400
   tataccgtcg acctctagct agagcttggc gtaatcatgg tcatagctgt ttcctgtgtg 5460
   aaattgttat ccgctcacaa ttccacacaa catacgagcc ggaagcataa agtgtaaagc 5520
   ctggggtgcc taatgagtga gctaactcac attaattgcg ttgcgctcac tgcccgcttt 5580
   ccagtcggga aacctgtcgt gccagctgca ttaatgaatc ggccaacgcg cggggagagg 5640
   cggtttgcgt attgggcgct cttccgcttc ctcgctcact gactcgctgc gctcggtcgt 5700
   tcggctgcgg cgagcggtat cagctcactc aaaggcggta atacggttat ccacagaatc 5760
   aggggataac gcaggaaaga acatgtgagc aaaaggccag caaaaggcca ggaaccgtaa 5820
   aaaggccgcg ttgctggcgt ttttccatag gctccgcccc cctgacgagc atcacaaaaa 5880
   tcgacgctca agtcagaggt ggcgaaaccc gacaggacta taaagatacc aggcgtttcc 5940
   ccctggaagc tccctcgtgc gctctcctgt tccgaccctg ccgcttaccg gatacctgtc 6000
   cgcctttctc ccttcgggaa gcgtggcgct ttctcaatgc tcacgctgta ggtatctcag 6060
   ttcggtgtag gtcgttcgct ccaagctggg ctgtgtgcac gaaccccccg ttcagcccga 6120
   ccgctgcgcc ttatccggta actatcgtct tgagtccaac ccggtaagac acgacttatc 6180
   gccactggca gcagccactg gtaacaggat tagcagagcg aggtatgtag gcggtgctac 6240
   agagttcttg aagtggtggc ctaactacgg ctacactaga aggacagtat ttggtatctg 6300
   cgctctgctg aagccagtta ccttcggaaa aagagttggt agctcttgat ccggcaaaca 6360
   aaccaccgct ggtagcggtg gtttttttgt ttgcaagcag cagattacgc gcagaaaaaa 6420
   aggatctcaa gaagatcctt tgatcttttc tacggggtct gacgctcagt ggaacgaaaa 6480
   ctcacgttaa gggattttgg tcatgagatt atcaaaaagg atcttcacct agatcctttt 6540
   aaattaaaaa tgaagtttta aatcaatcta aagtatatat gagtaaactt ggtctgacag 6600
   ttaccaatgc ttaatcagtg aggcacctat ctcagcgatc tgtctatttc gttcatccat 6660
   agttgcctga ctccccgtcg tgtagataac tacgatacgg gagggcttac catctggccc 6720
   cagtgctgca atgataccgc gagacccacg ctcaccggct ccagatttat cagcaataaa 6780
   ccagccagcc ggaagggccg agcgcagaag tggtcctgca actttatccg cctccatcca 6840
   gtctattaat tgttgccggg aagctagagt aagtagttcg ccagttaata gtttgcgcaa 6900
   cgttgttgcc attgctacag gcatcgtggt gtcacgctcg tcgtttggta tggcttcatt 6960
   cagctccggt tcccaacgat caaggcgagt tacatgatcc cccatgttgt gcaaaaaagc 7020
   ggttagctcc ttcggtcctc cgatcgttgt cagaagtaag ttggccgcag tgttatcact 7080
   catggttatg gcagcactgc ataattctct tactgtcatg ccatccgtaa gatgcttttc 7140
   tgtgactggt gagtactcaa ccaagtcatt ctgagaatag tgtatgcggc gaccgagttg 7200
   ctcttgcccg gcgtcaatac gggataatac cgcgccacat agcagaactt taaaagtgct 7260
   catcattgga aaacgttctt cggggcgaaa actctcaagg atcttaccgc tgttgagatc 7320
   cagttcgatg taacccactc gtgcacccaa ctgatcttca gcatctttta ctttcaccag 7380
   cgtttctggg tgagcaaaaa caggaaggca aaatgccgca aaaaagggaa taagggcgac 7440
   acggaaatgt tgaatactca tactcttcct ttttcaatat tattgaagca tttatcaggg 7500
   ttattgtctc atgagcggat acatatttga atgtatttag aaaaataaac aaataggggt 7560
   tccgcgcaca tttccccgaa aagtgccacc tgacgtc 7597
<210> 4
   <211> 7579
   <212> DNA
   <213> Artificial Sequence
<220>
   <223> Plasmid
<400> 4
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   ggtaccaagc ttggatctca ccatggagtt gggacttagc tgggttttcc tcgttgctct 960
   tttaagaggt gtccagtgtc aggtccagct ggtggagtct gggggaggcg tggtccagcc 1020
   tgggaggtcc ctgagactct cctgtgcagc gtctggattc accttcagta gctatggcat 1080
   gcactgggtc cgccaggctc caggcaaggg gctggactgg gtggcaatta tttggcatga 1140
   tggaagtaat aaatactatg cagactccgt gaagggccga ttcaccatct ccagagacaa 1200
   ttccaagaag acgctgtacc tgcaaatgaa cagtttgaga gccgaggaca cggctgtgta 1260
   ttactgtgcg agagcttggg cctatgacta cggtgactat gaatactact tcggtatgga 1320
   cgtctggggc caagggacca cggtcaccgt ctcctcagcc tccaccaagg gcccatcggt 1380
   cttccccctg gcaccctcta gcaagagcac ctctgggggc acagcggccc tgggctgcct 1440
   ggtcaaggac tacttccccg aaccggtgac ggtgtcgtgg aactcaggcg ccctgaccag 1500
   cggcgtgcac accttcccgg ctgtcctaca gtcctcagga ctctactccc tcagcagcgt 1560
   ggtgaccgtg ccctccagca gcttgggcac ccagacctac atctgcaacg tgaatcacaa 1620
   gcccagcaac accaaggtgg acaagagagt tggtgagagg ccagcacagg gagggagggt 1680
   gtctgctgga agccaggctc agcgctcctg cctggacgca tcccggctat gcagtcccag 1740
   tccagggcag caaggcaggc cccgtctgcc tcttcacccg gaggcctctg cccgccccac 1800
   tcatgctcag ggagagggtc ttctggcttt ttccccaggc tctgggcagg cacaggctag 1860
   gtgcccctaa cccaggccct gcacacaaag gggcaggtgc tgggctcaga cctgccaaga 1920
   gccatatccg ggaggaccct gcccctgacc taagcccacc ccaaaggcca aactctccac 1980
   tccctcagct cggacacctt ctctcctccc agattccagt aactcccaat cttctctctg 2040
   cagagcccaa atcttgtgac aaaactcaca catgcccacc gtgcccaggt aagccagccc 2100
   aggcctcgcc ctccagctca aggcgggaca ggtgccctag agtagcctgc atccagggac 2160
   aggccccagc cgggtgctga cacgtccacc tccatctctt cctcagcacc tgaactcctg 2220
   gggggaccgt cagtcttcct cttcccccca aaacccaagg acaccctcat gatctcccgg 2280
   acccctgagg tcacatgcgt ggtggtggac gtgagccacg aagaccctga ggtcaagttc 2340
   aactggtacg tggacggcgt ggaggtgcat aatgccaaga caaagccgcg ggaggagcag 2400
   tacaacagca cgtaccgtgt ggtcagcgtc ctcaccgtcc tgcaccagga ctggctgaat 2460
   ggcaaggagt acaagtgcaa ggtctccaac aaagccctcc cagcccccat cgagaaaacc 2520
   atctccaaag ccaaaggtgg gacccgtggg gtgcgagggc cacatggaca gaggccggct 2580
   cggcccaccc tctgccctga gagtgaccgc tgtaccaacc tctgtcccta cagggcagcc 2640
   ccgagaacca caggtgtaca ccctgccccc atcccgggag gagatgacca agaaccaggt 2700
   cagcctgacc tgcctggtca aaggcttcta tcccagcgac atcgccgtgg agtgggagag 2760
   caatgggcag ccggagaaca actacaagac cacgcctccc gtgctggact ccgacggctc 2820
   cttcttcctc tatagcaagc tcaccgtgga caagagcagg tggcagcagg ggaacgtctt 2880
   ctcatgctcc gtgatgcatg aggctctgca caaccactac acgcagaaga gcctctccct 2940
   gtctccgggt aaatgagaat tcctcgagtc tagagggccc gtttaaaccc gctgatcagc 3000
   ctcgactgtg ccttctagtt gccagccatc tgttgtttgc ccctcccccg tgccttcctt 3060
   gaccctggaa ggtgccactc ccactgtcct ttcctaataa aatgaggaaa ttgcatcgca 3120
   ttgtctgagt aggtgtcatt ctattctggg gggtggggtg gggcaggaca gcaaggggga 3180
   ggattgggaa gacaatagca ggcatgctgg ggatgcggtg ggctctatgg cttctgaggc 3240
   ggaaagaacc agctggggct ctagggggta tccccacgcg ccctgtagcg gcgcattaag 3300
   cgcggcgggt gtggtggtta cgcgcagcgt gaccgctaca cttgccagcg ccctagcgcc 3360
   cgctcctttc gctttcttcc cttcctttct cgccacgttc gccggctttc cccgtcaagc 3420
   tctaaatcgg ggcatccctt tagggttccg atttagtgct ttacggcacc tcgaccccaa 3480
   aaaacttgat tagggtgatg gttcacgtag tgggccatcg ccctgataga cggtttttcg 3540
   ccctttgacg ttggagtcca cgttctttaa tagtggactc ttgttccaaa ctggaacaac 3600
   actcaaccct atctcggtct attcttttga tttataaggg attttgggga tttcggccta 3660
   ttggttaaaa aatgagctga tttaacaaaa atttaacgcg aattaattct gtggaatgtg 3720
   tgtcagttag ggtgtggaaa gtccccaggc tccccaggca ggcagaagta tgcaaagcat 3780
   gcatctcaat tagtcagcaa ccaggtgtgg aaagtcccca ggctccccag caggcagaag 3840
   tatgcaaagc atgcatctca attagtcagc aaccatagtc ccgcccctaa ctccgcccat 3900
   cccgccccta actccgccca gttccgccca ttctccgccc catggctgac taattttttt 3960
   tatttatgca gaggccgagg ccgcctctgc ctctgagcta ttccagaagt agtgaggagg 4020
   cttttttgga ggcctaggct tttgcaaaaa gctcccggga gcttgtatat ccattttcgg 4080
   atctgatcag cacgtgatga aaaagcctga actcaccgcg acgtctgtcg agaagtttct 4140
   gatcgaaaag ttcgacagcg tctccgacct gatgcagctc tcggagggcg aagaatctcg 4200
   tgctttcagc ttcgatgtag gagggcgtgg atatgtcctg cgggtaaata gctgcgccga 4260
   tggtttctac aaagatcgtt atgtttatcg gcactttgca tcggccgcgc tcccgattcc 4320
   ggaagtgctt gacattgggg aattcagcga gagcctgacc tattgcatct cccgccgtgc 4380
   acagggtgtc acgttgcaag acctgcctga aaccgaactg cccgctgttc tgcagccggt 4440
   cgcggaggcc atggatgcga tcgctgcggc cgatcttagc cagacgagcg ggttcggccc 4500
   attcggaccg caaggaatcg gtcaatacac tacatggcgt gatttcatat gcgcgattgc 4560
   tgatccccat gtgtatcact ggcaaactgt gatggacgac accgtcagtg cgtccgtcgc 4620
   gcaggctctc gatgagctga tgctttgggc cgaggactgc cccgaagtcc ggcacctcgt 4680
   gcacgcggat ttcggctcca acaatgtcct gacggacaat ggccgcataa cagcggtcat 4740
   tgactggagc gaggcgatgt tcggggattc ccaatacgag gtcgccaaca tcttcttctg 4800
   gaggccgtgg ttggcttgta tggagcagca gacgcgctac ttcgagcgga ggcatccgga 4860
   gcttgcagga tcgccgcggc tccgggcgta tatgctccgc attggtcttg accaactcta 4920
   tcagagcttg gttgacggca atttcgatga tgcagcttgg gcgcagggtc gatgcgacgc 4980
   aatcgtccga tccggagccg ggactgtcgg gcgtacacaa atcgcccgca gaagcgcggc 5040
   cgtctggacc gatggctgtg tagaagtact cgccgatagt ggaaaccgac gccccagcac 5100
   tcgtccgagg gcaaaggaat agcacgtgct acgagatttc gattccaccg ccgccttcta 5160
   tgaaaggttg ggcttcggaa tcgttttccg ggacgccggc tggatgatcc tccagcgcgg 5220
   ggatctcatg ctggagttct tcgcccaccc caacttgttt attgcagctt ataatggtta 5280
   caaataaagc aatagcatca caaatttcac aaataaagca tttttttcac tgcattctag 5340
   ttgtggtttg tccaaactca tcaatgtatc ttatcatgtc tgtataccgt cgacctctag 5400
   ctagagcttg gcgtaatcat ggtcatagct gtttcctgtg tgaaattgtt atccgctcac 5460
   aattccacac aacatacgag ccggaagcat aaagtgtaaa gcctggggtg cctaatgagt 5520
   gagctaactc acattaattg cgttgcgctc actgcccgct ttccagtcgg gaaacctgtc 5580
   gtgccagctg cattaatgaa tcggccaacg cgcggggaga ggcggtttgc gtattgggcg 5640
   ctcttccgct tcctcgctca ctgactcgct gcgctcggtc gttcggctgc ggcgagcggt 5700
   atcagctcac tcaaaggcgg taatacggtt atccacagaa tcaggggata acgcaggaaa 5760
   gaacatgtga gcaaaaggcc agcaaaaggc caggaaccgt aaaaaggccg cgttgctggc 5820
   gtttttccat aggctccgcc cccctgacga gcatcacaaa aatcgacgct caagtcagag 5880
   gtggcgaaac ccgacaggac tataaagata ccaggcgttt ccccctggaa gctccctcgt 5940
   gcgctctcct gttccgaccc tgccgcttac cggatacctg tccgcctttc tcccttcggg 6000
   aagcgtggcg ctttctcaat gctcacgctg taggtatctc agttcggtgt aggtcgttcg 6060
   ctccaagctg ggctgtgtgc acgaaccccc cgttcagccc gaccgctgcg ccttatccgg 6120
   taactatcgt cttgagtcca acccggtaag acacgactta tcgccactgg cagcagccac 6180
   tggtaacagg attagcagag cgaggtatgt aggcggtgct acagagttct tgaagtggtg 6240
   gcctaactac ggctacacta gaaggacagt atttggtatc tgcgctctgc tgaagccagt 6300
   taccttcgga aaaagagttg gtagctcttg atccggcaaa caaaccaccg ctggtagcgg 6360
   tggttttttt gtttgcaagc agcagattac gcgcagaaaa aaaggatctc aagaagatcc 6420
   tttgatcttt tctacggggt ctgacgctca gtggaacgaa aactcacgtt aagggatttt 6480
   ggtcatgaga ttatcaaaaa ggatcttcac ctagatcctt ttaaattaaa aatgaagttt 6540
   taaatcaatc taaagtatat atgagtaaac ttggtctgac agttaccaat gcttaatcag 6600
   tgaggcacct atctcagcga tctgtctatt tcgttcatcc atagttgcct gactccccgt 6660
   cgtgtagata actacgatac gggagggctt accatctggc cccagtgctg caatgatacc 6720
   gcgagaccca cgctcaccgg ctccagattt atcagcaata aaccagccag ccggaagggc 6780
   cgagcgcaga agtggtcctg caactttatc cgcctccatc cagtctatta attgttgccg 6840
   ggaagctaga gtaagtagtt cgccagttaa tagtttgcgc aacgttgttg ccattgctac 6900
   aggcatcgtg gtgtcacgct cgtcgtttgg tatggcttca ttcagctccg gttcccaacg 6960
   atcaaggcga gttacatgat cccccatgtt gtgcaaaaaa gcggttagct ccttcggtcc 7020
   tccgatcgtt gtcagaagta agttggccgc agtgttatca ctcatggtta tggcagcact 7080
   gcataattct cttactgtca tgccatccgt aagatgcttt tctgtgactg gtgagtactc 7140
   aaccaagtca ttctgagaat agtgtatgcg gcgaccgagt tgctcttgcc cggcgtcaat 7200
   acgggataat accgcgccac atagcagaac tttaaaagtg ctcatcattg gaaaacgttc 7260
   ttcggggcga aaactctcaa ggatcttacc gctgttgaga tccagttcga tgtaacccac 7320
   tcgtgcaccc aactgatctt cagcatcttt tactttcacc agcgtttctg ggtgagcaaa 7380
   aacaggaagg caaaatgccg caaaaaaggg aataagggcg acacggaaat gttgaatact 7440
   catactcttc ctttttcaat attattgaag catttatcag ggttattgtc tcatgagcgg 7500
   atacatattt gaatgtattt agaaaaataa acaaataggg gttccgcgca catttccccg 7560
   aaaagtgcca cctgacgtc 7579
<210> 5
   <211> 7558
   <212> DNA
   <213> Artificial Sequence
<220>
   <223> Plasmid
<400> 5
   gacggatcgg gagatctccc gatcccctat ggtcgactct cagtacaatc tgctctgatg      60
   ccgcatagtt aagccagtat ctgctccctg cttgtgtgtt ggaggtcgct gagtagtgcg     120
   cgagcaaaat ttaagctaca acaaggcaag gcttgaccga caattgcatg aagaatctgc     180
   ttagggttag gcgttttgcg ctgcttcgcg atgtacgggc cagatatacg cgttgacatt     240
   gattattgac tagttattaa tagtaatcaa ttacggggtc attagttcat agcccatata     300
   tggagttccg cgttacataa cttacggtaa atggcccgcc tggctgaccg cccaacgacc     360
   cccgcccatt gacgtcaata atgacgtatg ttcccatagt aacgccaata gggactttcc     420
   attgacgtca atgggtggac tatttacggt aaactgccca cttggcagta catcaagtgt     480
   atcatatgcc aagtacgccc cctattgacg tcaatgacgg taaatggccc gcctggcatt     540
   atgcccagta catgacctta tgggactttc ctacttggca gtacatctac gtattagtca     600
   tcgctattac catggtgatg cggttttggc agtacatcaa tgggcgtgga tagcggtttg     660
   actcacgggg atttccaagt ctccacccca ttgacgtcaa tgggagtttg ttttggcacc     720
   aaaatcaacg ggactttcca aaatgtcgta acaactccgc cccattgacg caaatgggcg     780
   gtaggcgtgt acggtgggag gtctatataa gcagagctct ctggctaact agagaaccca     840
   ctgcttactg gcttatcgaa attaatacga ctcactatag ggagacccaa gctggctaga     900
   ggtaccaagc ttggatccca ccatggggtc aaccgtcatc ctcgccctcc tcctggctgt     960
   tctccaagga gtctgtgccg aggtgcagct ggtgcagtct ggagcagagg tgaaaaagcc    1020
   cggggagtct ctgaagatct cctgtaaggg ttctggatac agctttacca gttactggat    1080
   cggctgggtg cgccagatgc ccgggaaagg cctggagtgg atggggatca tctatcctgg    1140
   tgactctgat accagataca gcccgtcctt ccaaggccag gtcaccatct cagccgacaa    1200
   gtccatcagc accgcctacc tgcagtggag cagcctgaag gcctcggaca ccgccatgta    1260
   ttactgtgcg agacggatgg cagcagctgg cccctttgac tactggggcc agggaaccct    1320
   ggtcaccgtc tcctcagcct ccaccaaggg cccatcggtc ttccccctgg caccctctag    1380
   caagagcacc tctgggggca cagcggccct gggctgcctg gtcaaggact acttccccga    1440
   accggtgacg gtgtcgtgga actcaggcgc cctgaccagc ggcgtgcaca ccttcccggc 1500
   tgtcctacag tcctcaggac tctactccct cagcagcgtg gtgaccgtgc cctccagcag 1560
   cttgggcacc cagacctaca tctgcaacgt gaatcacaag cccagcaaca ccaaggtgga 1620
   caagagagtt ggtgagaggc cagcacaggg agggagggtg tctgctggaa gccaggctca 1680
   gcgctcctgc ctggacgcat cccggctatg cagtcccagt ccagggcagc aaggcaggcc 1740
   ccgtctgcct cttcacccgg aggcctctgc ccgccccact catgctcagg gagagggtct 1800
   tctggctttt tccccaggct ctgggcaggc acaggctagg tgcccctaac ccaggccctg 1860
   cacacaaagg ggcaggtgct gggctcagac ctgccaagag ccatatccgg gaggaccctg 1920
   cccctgacct aagcccaccc caaaggccaa actctccact ccctcagctc ggacaccttc 1980
   tctcctccca gattccagta actcccaatc ttctctctgc agagcccaaa tcttgtgaca 2040
   aaactcacac atgcccaccg tgcccaggta agccagccca ggcctcgccc tccagctcaa 2100
   ggcgggacag gtgccctaga gtagcctgca tccagggaca ggccccagcc gggtgctgac 2160
   acgtccacct ccatctcttc ctcagcacct gaactcctgg ggggaccgtc agtcttcctc 2220
   ttccccccaa aacccaagga caccctcatg atctcccgga cccctgaggt cacatgcgtg 2280
   gtggtggacg tgagccacga agaccctgag gtcaagttca actggtacgt ggacggcgtg 2340
   gaggtgcata atgccaagac aaagccgcgg gaggagcagt acaacagcac gtaccgtgtg 2400
   gtcagcgtcc tcaccgtcct gcaccaggac tggctgaatg gcaaggagta caagtgcaag 2460
   gtctccaaca aagccctccc agcccccatc gagaaaacca tctccaaagc caaaggtggg 2520
   acccgtgggg tgcgagggcc acatggacag aggccggctc ggcccaccct ctgccctgag 2580
   agtgaccgct gtaccaacct ctgtccctac agggcagccc cgagaaccac aggtgtacac 2640
   cctgccccca tcccgggagg agatgaccaa gaaccaggtc agcctgacct gcctggtcaa 2700
   aggcttctat cccagcgaca tcgccgtgga gtgggagagc aatgggcagc cggagaacaa 2760
   ctacaagacc acgcctcccg tgctggactc cgacggctcc ttcttcctct atagcaagct 2820
   caccgtggac aagagcaggt ggcagcaggg gaacgtcttc tcatgctccg tgatgcatga 2880
   ggctctgcac aaccactaca cgcagaagag cctctccctg tctccgggta aatgagaatt 2940
   cctcgagtct agagggcccg tttaaacccg ctgatcagcc tcgactgtgc cttctagttg 3000
   ccagccatct gttgtttgcc cctcccccgt gccttccttg accctggaag gtgccactcc 3060
   cactgtcctt tcctaataaa atgaggaaat tgcatcgcat tgtctgagta ggtgtcattc 3120
   tattctgggg ggtggggtgg ggcaggacag caagggggag gattgggaag acaatagcag 3180
   gcatgctggg gatgcggtgg gctctatggc ttctgaggcg gaaagaacca gctggggctc 3240
   tagggggtat ccccacgcgc cctgtagcgg cgcattaagc gcggcgggtg tggtggttac 3300
   gcgcagcgtg accgctacac ttgccagcgc cctagcgccc gctcctttcg ctttcttccc 3360
   ttcctttctc gccacgttcg ccggctttcc ccgtcaagct ctaaatcggg gcatcccttt 3420
   agggttccga tttagtgctt tacggcacct cgaccccaaa aaacttgatt agggtgatgg 3480
   ttcacgtagt gggccatcgc cctgatagac ggtttttcgc cctttgacgt tggagtccac 3540
   gttctttaat agtggactct tgttccaaac tggaacaaca ctcaacccta tctcggtcta 3600
   ttcttttgat ttataaggga ttttggggat ttcggcctat tggttaaaaa atgagctgat 3660
   ttaacaaaaa tttaacgcga attaattctg tggaatgtgt gtcagttagg gtgtggaaag 3720
   tccccaggct ccccaggcag gcagaagtat gcaaagcatg catctcaatt agtcagcaac 3780
   caggtgtgga aagtccccag gctccccagc aggcagaagt atgcaaagca tgcatctcaa 3840
   ttagtcagca accatagtcc cgcccctaac tccgcccatc ccgcccctaa ctccgcccag 3900
   ttccgcccat tctccgcccc atggctgact aatttttttt atttatgcag aggccgaggc 3960
   cgcctctgcc tctgagctat tccagaagta gtgaggaggc ttttttggag gcctaggctt 4020
   ttgcaaaaag ctcccgggag cttgtatatc cattttcgga tctgatcagc acgtgatgaa 4080
   aaagcctgaa ctcaccgcga cgtctgtcga gaagtttctg atcgaaaagt tcgacagcgt 4140
   ctccgacctg atgcagctct cggagggcga agaatctcgt gctttcagct tcgatgtagg 4200
   agggcgtgga tatgtcctgc gggtaaatag ctgcgccgat ggtttctaca aagatcgtta 4260
   tgtttatcgg cactttgcat cggccgcgct cccgattccg gaagtgcttg acattgggga 4320
   attcagcgag agcctgacct attgcatctc ccgccgtgca cagggtgtca cgttgcaaga 4380
   cctgcctgaa accgaactgc ccgctgttct gcagccggtc gcggaggcca tggatgcgat 4440
   cgctgcggcc gatcttagcc agacgagcgg gttcggccca ttcggaccgc aaggaatcgg 4500
   tcaatacact acatggcgtg atttcatatg cgcgattgct gatccccatg tgtatcactg 4560
   gcaaactgtg atggacgaca ccgtcagtgc gtccgtcgcg caggctctcg atgagctgat 4620
   gctttgggcc gaggactgcc ccgaagtccg gcacctcgtg cacgcggatt tcggctccaa 4680
   caatgtcctg acggacaatg gccgcataac agcggtcatt gactggagcg aggcgatgtt 4740
   cggggattcc caatacgagg tcgccaacat cttcttctgg aggccgtggt tggcttgtat 4800
   ggagcagcag acgcgctact tcgagcggag gcatccggag cttgcaggat cgccgcggct 4860
   ccgggcgtat atgctccgca ttggtcttga ccaactctat cagagcttgg ttgacggcaa 4920
   tttcgatgat gcagcttggg cgcagggtcg atgcgacgca atcgtccgat ccggagccgg 4980
   gactgtcggg cgtacacaaa tcgcccgcag aagcgcggcc gtctggaccg atggctgtgt 5040
   agaagtactc gccgatagtg gaaaccgacg ccccagcact cgtccgaggg caaaggaata 5100
   gcacgtgcta cgagatttcg attccaccgc cgccttctat gaaaggttgg gcttcggaat 5160
   cgttttccgg gacgccggct ggatgatcct ccagcgcggg gatctcatgc tggagttctt 5220
   cgcccacccc aacttgttta ttgcagctta taatggttac aaataaagca atagcatcac 5280
   aaatttcaca aataaagcat ttttttcact gcattctagt tgtggtttgt ccaaactcat 5340
   caatgtatct tatcatgtct gtataccgtc gacctctagc tagagcttgg cgtaatcatg 5400
   gtcatagctg tttcctgtgt gaaattgtta tccgctcaca attccacaca acatacgagc 5460
   cggaagcata aagtgtaaag cctggggtgc ctaatgagtg agctaactca cattaattgc 5520
   gttgcgctca ctgcccgctt tccagtcggg aaacctgtcg tgccagctgc attaatgaat 5580
   cggccaacgc gcggggagag gcggtttgcg tattgggcgc tcttccgctt cctcgctcac 5640
   tgactcgctg cgctcggtcg ttcggctgcg gcgagcggta tcagctcact caaaggcggt 5700
   aatacggtta tccacagaat caggggataa cgcaggaaag aacatgtgag caaaaggcca 5760
   gcaaaaggcc aggaaccgta aaaaggccgc gttgctggcg tttttccata ggctccgccc 5820
   ccctgacgag catcacaaaa atcgacgctc aagtcagagg tggcgaaacc cgacaggact 5880
   ataaagatac caggcgtttc cccctggaag ctccctcgtg cgctctcctg ttccgaccct 5940
   gccgcttacc ggatacctgt ccgcctttct cccttcggga agcgtggcgc tttctcaatg 6000
   ctcacgctgt aggtatctca gttcggtgta ggtcgttcgc tccaagctgg gctgtgtgca 6060
   cgaacccccc gttcagcccg accgctgcgc cttatccggt aactatcgtc ttgagtccaa 6120
   cccggtaaga cacgacttat cgccactggc agcagccact ggtaacagga ttagcagagc 6180
   gaggtatgta ggcggtgcta cagagttctt gaagtggtgg cctaactacg gctacactag 6240
   aaggacagta tttggtatct gcgctctgct gaagccagtt accttcggaa aaagagttgg 6300
   tagctcttga tccggcaaac aaaccaccgc tggtagcggt ggtttttttg tttgcaagca 6360
   gcagattacg cgcagaaaaa aaggatctca agaagatcct ttgatctttt ctacggggtc 6420
   tgacgctcag tggaacgaaa actcacgtta agggattttg gtcatgagat tatcaaaaag 6480
   gatcttcacc tagatccttt taaattaaaa atgaagtttt aaatcaatct aaagtatata 6540
   tgagtaaact tggtctgaca gttaccaatg cttaatcagt gaggcaccta tctcagcgat 6600
   ctgtctattt cgttcatcca tagttgcctg actccccgtc gtgtagataa ctacgatacg 6660
   ggagggctta ccatctggcc ccagtgctgc aatgataccg cgagacccac gctcaccggc 6720
   tccagattta tcagcaataa accagccagc cggaagggcc gagcgcagaa gtggtcctgc 6780
   aactttatcc gcctccatcc agtctattaa ttgttgccgg gaagctagag taagtagttc 6840
   gccagttaat agtttgcgca acgttgttgc cattgctaca ggcatcgtgg tgtcacgctc 6900
   gtcgtttggt atggcttcat tcagctccgg ttcccaacga tcaaggcgag ttacatgatc 6960
   ccccatgttg tgcaaaaaag cggttagctc cttcggtcct ccgatcgttg tcagaagtaa 7020
   gttggccgca gtgttatcac tcatggttat ggcagcactg cataattctc ttactgtcat 7080
   gccatccgta agatgctttt ctgtgactgg tgagtactca accaagtcat tctgagaata 7140
   gtgtatgcgg cgaccgagtt gctcttgccc ggcgtcaata cgggataata ccgcgccaca 7200
   tagcagaact ttaaaagtgc tcatcattgg aaaacgttct tcggggcgaa aactctcaag 7260
   gatcttaccg ctgttgagat ccagttcgat gtaacccact cgtgcaccca actgatcttc 7320
   agcatctttt actttcacca gcgtttctgg gtgagcaaaa acaggaaggc aaaatgccgc 7380
   aaaaaaggga ataagggcga cacggaaatg ttgaatactc atactcttcc tttttcaata 7440
   ttattgaagc atttatcagg gttattgtct catgagcgga tacatatttg aatgtattta 7500
   gaaaaataaa caaatagggg ttccgcgcac atttccccga aaagtgccac ctgacgtc 7558
<210> 6
   <211> 7576
   <212> DNA
   <213> Artificial Sequence
<220>
   <223> Plasmid
<400> 6
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   ggtaccaagc ttggatctca ccatggagtt tgggctgtgc tggattttcc tcgttgctct 960
   tttaagaggt gtccagtgtc aggtgcagct ggtggagtct gggggaggcg tggtccagcc 1020
   tgggaggtcc ctgagactct cctgtgcagc ctctggattc accttcatta gctatggcat 1080
   gcactgggtc cgccaggctc caggcaaggg gctggagtgg gtggcagtta tatcatatga 1140
   tggaagtaat aaatactatg cagactccgt gaagggccga ttcaccatct ccagagacaa 1200
   ttccaagaac acgctgtatc tgcaaatgaa cagcctgaga gctgaggaca cggctgtgta 1260
   ttactgtgcg agagtattag tgggagcttt atattattat aactactacg ggatggacgt 1320
   ctggggccaa gggaccacgg tcaccgtctc ctcagcctcc accaagggcc catcggtctt 1380
   ccccctggca ccctctagca agagcacctc tgggggcaca gcggccctgg gctgcctggt 1440
   caaggactac ttccccgaac cggtgacggt gtcgtggaac tcaggcgccc tgaccagcgg 1500
   cgtgcacacc ttcccggctg tcctacagtc ctcaggactc tactccctca gcagcgtggt 1560
   gaccgtgccc tccagcagct tgggcaccca gacctacatc tgcaacgtga atcacaagcc 1620
   cagcaacacc aaggtggaca agagagttgg tgagaggcca gcacagggag ggagggtgtc 1680
   tgctggaagc caggctcagc gctcctgcct ggacgcatcc cggctatgca gtcccagtcc 1740
   agggcagcaa ggcaggcccc gtctgcctct tcacccggag gcctctgccc gccccactca 1800
   tgctcaggga gagggtcttc tggctttttc cccaggctct gggcaggcac aggctaggtg 1860
   cccctaaccc aggccctgca cacaaagggg caggtgctgg gctcagacct gccaagagcc 1920
   atatccggga ggaccctgcc cctgacctaa gcccacccca aaggccaaac tctccactcc 1980
   ctcagctcgg acaccttctc tcctcccaga ttccagtaac tcccaatctt ctctctgcag 2040
   agcccaaatc ttgtgacaaa actcacacat gcccaccgtg cccaggtaag ccagcccagg 2100
   cctcgccctc cagctcaagg cgggacaggt gccctagagt agcctgcatc cagggacagg 2160
   ccccagccgg gtgctgacac gtccacctcc atctcttcct cagcacctga actcctgggg 2220
   ggaccgtcag tcttcctctt ccccccaaaa cccaaggaca ccctcatgat ctcccggacc 2280
   cctgaggtca catgcgtggt ggtggacgtg agccacgaag accctgaggt caagttcaac 2340
   tggtacgtgg acggcgtgga ggtgcataat gccaagacaa agccgcggga ggagcagtac 2400
   aacagcacgt accgtgtggt cagcgtcctc accgtcctgc accaggactg gctgaatggc 2460
   aaggagtaca agtgcaaggt ctccaacaaa gccctcccag cccccatcga gaaaaccatc 2520
   tccaaagcca aaggtgggac ccgtggggtg cgagggccac atggacagag gccggctcgg 2580
   cccaccctct gccctgagag tgaccgctgt accaacctct gtccctacag ggcagccccg 2640
   agaaccacag gtgtacaccc tgcccccatc ccgggaggag atgaccaaga accaggtcag 2700
   cctgacctgc ctggtcaaag gcttctatcc cagcgacatc gccgtggagt gggagagcaa 2760
   tgggcagccg gagaacaact acaagaccac gcctcccgtg ctggactccg acggctcctt 2820
   cttcctctat agcaagctca ccgtggacaa gagcaggtgg cagcagggga acgtcttctc 2880
   atgctccgtg atgcatgagg ctctgcacaa ccactacacg cagaagagcc tctccctgtc 2940
   tccgggtaaa tgagaattcc tcgagtctag agggcccgtt taaacccgct gatcagcctc 3000
   gactgtgcct tctagttgcc agccatctgt tgtttgcccc tcccccgtgc cttccttgac 3060
   cctggaaggt gccactccca ctgtcctttc ctaataaaat gaggaaattg catcgcattg 3120
   tctgagtagg tgtcattcta ttctgggggg tggggtgggg caggacagca agggggagga 3180
   ttgggaagac aatagcaggc atgctgggga tgcggtgggc tctatggctt ctgaggcgga 3240
   aagaaccagc tggggctcta gggggtatcc ccacgcgccc tgtagcggcg cattaagcgc 3300
   ggcgggtgtg gtggttacgc gcagcgtgac cgctacactt gccagcgccc tagcgcccgc 3360
   tcctttcgct ttcttccctt cctttctcgc cacgttcgcc ggctttcccc gtcaagctct 3420
   aaatcggggc atccctttag ggttccgatt tagtgcttta cggcacctcg accccaaaaa 3480
   acttgattag ggtgatggtt cacgtagtgg gccatcgccc tgatagacgg tttttcgccc 3540
   tttgacgttg gagtccacgt tctttaatag tggactcttg ttccaaactg gaacaacact 3600
   caaccctatc tcggtctatt cttttgattt ataagggatt ttggggattt cggcctattg 3660
   gttaaaaaat gagctgattt aacaaaaatt taacgcgaat taattctgtg gaatgtgtgt 3720
   cagttagggt gtggaaagtc cccaggctcc ccaggcaggc agaagtatgc aaagcatgca 3780
   tctcaattag tcagcaacca ggtgtggaaa gtccccaggc tccccagcag gcagaagtat 3840
   gcaaagcatg catctcaatt agtcagcaac catagtcccg cccctaactc cgcccatccc 3900
   gcccctaact ccgcccagtt ccgcccattc tccgccccat ggctgactaa ttttttttat 3960
   ttatgcagag gccgaggccg cctctgcctc tgagctattc cagaagtagt gaggaggctt 4020
   ttttggaggc ctaggctttt gcaaaaagct cccgggagct tgtatatcca ttttcggatc 4080
   tgatcagcac gtgatgaaaa agcctgaact caccgcgacg tctgtcgaga agtttctgat 4140
   cgaaaagttc gacagcgtct ccgacctgat gcagctctcg gagggcgaag aatctcgtgc 4200
   tttcagcttc gatgtaggag ggcgtggata tgtcctgcgg gtaaatagct gcgccgatgg 4260
   tttctacaaa gatcgttatg tttatcggca ctttgcatcg gccgcgctcc cgattccgga 4320
   agtgcttgac attggggaat tcagcgagag cctgacctat tgcatctccc gccgtgcaca 4380
   gggtgtcacg ttgcaagacc tgcctgaaac cgaactgccc gctgttctgc agccggtcgc 4440
   ggaggccatg gatgcgatcg ctgcggccga tcttagccag acgagcgggt tcggcccatt 4500
   cggaccgcaa ggaatcggtc aatacactac atggcgtgat ttcatatgcg cgattgctga 4560
   tccccatgtg tatcactggc aaactgtgat ggacgacacc gtcagtgcgt ccgtcgcgca 4620
   ggctctcgat gagctgatgc tttgggccga ggactgcccc gaagtccggc acctcgtgca 4680
   cgcggatttc ggctccaaca atgtcctgac ggacaatggc cgcataacag cggtcattga 4740
   ctggagcgag gcgatgttcg gggattccca atacgaggtc gccaacatct tcttctggag 4800
   gccgtggttg gcttgtatgg agcagcagac gcgctacttc gagcggaggc atccggagct 4860
   tgcaggatcg ccgcggctcc gggcgtatat gctccgcatt ggtcttgacc aactctatca 4920
   gagcttggtt gacggcaatt tcgatgatgc agcttgggcg cagggtcgat gcgacgcaat 4980
   cgtccgatcc ggagccggga ctgtcgggcg tacacaaatc gcccgcagaa gcgcggccgt 5040
   ctggaccgat ggctgtgtag aagtactcgc cgatagtgga aaccgacgcc ccagcactcg 5100
   tccgagggca aaggaatagc acgtgctacg agatttcgat tccaccgccg ccttctatga 5160
   aaggttgggc ttcggaatcg ttttccggga cgccggctgg atgatcctcc agcgcgggga 5220
   tctcatgctg gagttcttcg cccaccccaa cttgtttatt gcagcttata atggttacaa 5280
   ataaagcaat agcatcacaa atttcacaaa taaagcattt ttttcactgc attctagttg 5340
   tggtttgtcc aaactcatca atgtatctta tcatgtctgt ataccgtcga cctctagcta 5400
   gagcttggcg taatcatggt catagctgtt tcctgtgtga aattgttatc cgctcacaat 5460
   tccacacaac atacgagccg gaagcataaa gtgtaaagcc tggggtgcct aatgagtgag 5520
   ctaactcaca ttaattgcgt tgcgctcact gcccgctttc cagtcgggaa acctgtcgtg 5580
   ccagctgcat taatgaatcg gccaacgcgc ggggagaggc ggtttgcgta ttgggcgctc 5640
   ttccgcttcc tcgctcactg actcgctgcg ctcggtcgtt cggctgcggc gagcggtatc 5700
   agctcactca aaggcggtaa tacggttatc cacagaatca ggggataacg caggaaagaa 5760
   catgtgagca aaaggccagc aaaaggccag gaaccgtaaa aaggccgcgt tgctggcgtt 5820
   tttccatagg ctccgccccc ctgacgagca tcacaaaaat cgacgctcaa gtcagaggtg 5880
   gcgaaacccg acaggactat aaagatacca ggcgtttccc cctggaagct ccctcgtgcg 5940
   ctctcctgtt ccgaccctgc cgcttaccgg atacctgtcc gcctttctcc cttcgggaag 6000
   cgtggcgctt tctcaatgct cacgctgtag gtatctcagt tcggtgtagg tcgttcgctc 6060
   caagctgggc tgtgtgcacg aaccccccgt tcagcccgac cgctgcgcct tatccggtaa 6120
   ctatcgtctt gagtccaacc cggtaagaca cgacttatcg ccactggcag cagccactgg 6180
   taacaggatt agcagagcga ggtatgtagg cggtgctaca gagttcttga agtggtggcc 6240
   taactacggc tacactagaa ggacagtatt tggtatctgc gctctgctga agccagttac 6300
   cttcggaaaa agagttggta gctcttgatc cggcaaacaa accaccgctg gtagcggtgg 6360
   tttttttgtt tgcaagcagc agattacgcg cagaaaaaaa ggatctcaag aagatccttt 6420
   gatcttttct acggggtctg acgctcagtg gaacgaaaac tcacgttaag ggattttggt 6480
   catgagatta tcaaaaagga tcttcaccta gatcctttta aattaaaaat gaagttttaa 6540
   atcaatctaa agtatatatg agtaaacttg gtctgacagt taccaatgct taatcagtga 6600
   ggcacctatc tcagcgatct gtctatttcg ttcatccata gttgcctgac tccccgtcgt 6660
   gtagataact acgatacggg agggcttacc atctggcccc agtgctgcaa tgataccgcg 6720
   agacccacgc tcaccggctc cagatttatc agcaataaac cagccagccg gaagggccga 6780
   gcgcagaagt ggtcctgcaa ctttatccgc ctccatccag tctattaatt gttgccggga 6840
   agctagagta agtagttcgc cagttaatag tttgcgcaac gttgttgcca ttgctacagg 6900
   catcgtggtg tcacgctcgt cgtttggtat ggcttcattc agctccggtt cccaacgatc 6960
   aaggcgagtt acatgatccc ccatgttgtg caaaaaagcg gttagctcct tcggtcctcc 7020
   gatcgttgtc agaagtaagt tggccgcagt gttatcactc atggttatgg cagcactgca 7080
   taattctctt actgtcatgc catccgtaag atgcttttct gtgactggtg agtactcaac 7140
   caagtcattc tgagaatagt gtatgcggcg accgagttgc tcttgcccgg cgtcaatacg 7200
   ggataatacc gcgccacata gcagaacttt aaaagtgctc atcattggaa aacgttcttc 7260
   ggggcgaaaa ctctcaagga tcttaccgct gttgagatcc agttcgatgt aacccactcg 7320
   tgcacccaac tgatcttcag catcttttac tttcaccagc gtttctgggt gagcaaaaac 7380
   aggaaggcaa aatgccgcaa aaaagggaat aagggcgaca cggaaatgtt gaatactcat 7440
   actcttcctt tttcaatatt attgaagcat ttatcagggt tattgtctca tgagcggata 7500
   catatttgaa tgtatttaga aaaataaaca aataggggtt ccgcgcacat ttccccgaaa 7560
   agtgccacct gacgtc 7576
<210> 7
   <211> 7561
   <212> DNA
   <213> Artificial Sequence
<220>
   <223> Plasmid
<400> 7
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   ggtaccggat ctcaccatgg agttggggct gagctgggtt ttcctcgttg ctcttttaag 960
   aggtgtccag tgtcaggagc agctggtgga gtctggggga ggcgtggtcc agcctgggag 1020
   gtccctgaga ctctcctgtg cagcgtctgg attcaccttc agtacctatg gcatgcactg 1080
   ggtccgccag gctccaggca aggggctgga gtgggtggca gttacatggc atgatggaag 1140
   taataaatac tatgcagact ccgtgaaggg ccgattcacc atctccagag acaactccaa 1200
   gaacacgctg tatctgcaaa tgaacagcct gagagccgag gacacggctg tgtattactg 1260
   tgcgagagga ggagtgggag caacttacta ctactactac ggtatggacg tctggggcca 1320
   agggaccacg gtcaccgtct cctcagcctc caccaagggc ccatcggtct tccccctggc 1380
   accctctagc aagagcacct ctgggggcac agcggccctg ggctgcctgg tcaaggacta 1440
   cttccccgaa ccggtgacgg tgtcgtggaa ctcaggcgcc ctgaccagcg gcgtgcacac 1500
   cttcccggct gtcctacagt cctcaggact ctactccctc agcagcgtgg tgaccgtgcc 1560
   ctccagcagc ttgggcaccc agacctacat ctgcaacgtg aatcacaagc ccagcaacac 1620
   caaggtggac aagagagttg gtgagaggcc agcacaggga gggagggtgt ctgctggaag 1680
   ccaggctcag cgctcctgcc tggacgcatc ccggctatgc agtcccagtc cagggcagca 1740
   aggcaggccc cgtctgcctc ttcacccgga ggcctctgcc cgccccactc atgctcaggg 1800
   agagggtctt ctggcttttt ccccaggctc tgggcaggca caggctaggt gcccctaacc 1860
   caggccctgc acacaaaggg gcaggtgctg ggctcagacc tgccaagagc catatccggg   1920
   aggaccctgc ccctgaccta agcccacccc aaaggccaaa ctctccactc cctcagctcg   1980
   gacaccttct ctcctcccag attccagtaa ctcccaatct tctctctgca gagcccaaat   2040
   cttgtgacaa aactcacaca tgcccaccgt gcccaggtaa gccagcccag gcctcgccct   2100
   ccagctcaag gcgggacagg tgccctagag tagcctgcat ccagggacag gccccagccg   2160
   ggtgctgaca cgtccacctc catctcttcc tcagcacctg aactcctggg gggaccgtca   2220
   gtcttcctct tccccccaaa acccaaggac accctcatga tctcccggac ccctgaggtc   2280
   acatgcgtgg tggtggacgt gagccacgaa gaccctgagg tcaagttcaa ctggtacgtg   2340
   gacggcgtgg aggtgcataa tgccaagaca aagccgcggg aggagcagta caacagcacg   2400
   taccgtgtgg tcagcgtcct caccgtcctg caccaggact ggctgaatgg caaggagtac   2460
   aagtgcaagg tctccaacaa agccctccca gcccccatcg agaaaaccat ctccaaagcc   2520
   aaaggtggga cccgtggggt gcgagggcca catggacaga ggccggctcg gcccaccctc   2580
   tgccctgaga gtgaccgctg taccaacctc tgtccctaca gggcagcccc gagaaccaca   2640
   ggtgtacacc ctgcccccat cccgggagga gatgaccaag aaccaggtca gcctgacctg   2700
   cctggtcaaa ggcttctatc ccagcgacat cgccgtggag tgggagagca atgggcagcc   2760
   ggagaacaac tacaagacca cgcctcccgt gctggactcc gacggctcct tcttcctcta   2820
   tagcaagctc accgtggaca agagcaggtg gcagcagggg aacgtcttct catgctccgt   2880
   gatgcatgag gctctgcaca accactacac gcagaagagc ctctccctgt ctccgggtaa   2940
   atgactcgag tctagagggc ccgtttaaac ccgctgatca gcctcgactg tgccttctag   3000
   ttgccagcca tctgttgttt gcccctcccc cgtgccttcc ttgaccctgg aaggtgccac   3060
   tcccactgtc ctttcctaat aaaatgagga aattgcatcg cattgtctga gtaggtgtca   3120
   ttctattctg gggggtgggg tggggcagga cagcaagggg gaggattggg aagacaatag   3180
   caggcatgct ggggatgcgg tgggctctat ggcttctgag gcggaaagaa ccagctgggg   3240
   ctctaggggg tatccccacg cgccctgtag cggcgcatta agcgcggcgg gtgtggtggt   3300
   tacgcgcagc gtgaccgcta cacttgccag cgccctagcg cccgctcctt tcgctttctt   3360
   cccttccttt ctcgccacgt tcgccggctt tccccgtcaa gctctaaatc ggggcatccc   3420
   tttagggttc cgatttagtg ctttacggca cctcgacccc aaaaaacttg attagggtga   3480
   tggttcacgt agtgggccat cgccctgata gacggttttt cgccctttga cgttggagtc   3540
   cacgttcttt aatagtggac tcttgttcca aactggaaca acactcaacc ctatctcggt   3600
   ctattctttt gatttataag ggattttggg gatttcggcc tattggttaa aaaatgagct   3660
   gatttaacaa aaatttaacg cgaattaatt ctgtggaatg tgtgtcagtt agggtgtgga   3720
   aagtccccag gctccccagg caggcagaag tatgcaaagc atgcatctca attagtcagc 3780
   aaccaggtgt ggaaagtccc caggctcccc agcaggcaga agtatgcaaa gcatgcatct 3840
   caattagtca gcaaccatag tcccgcccct aactccgccc atcccgcccc taactccgcc 3900
   cagttccgcc cattctccgc cccatggctg actaattttt tttatttatg cagaggccga 3960
   ggccgcctct gcctctgagc tattccagaa gtagtgagga ggcttttttg gaggcctagg 4020
   cttttgcaaa aagctcccgg gagcttgtat atccattttc ggatctgatc agcacgtgat 4080
   gaaaaagcct gaactcaccg cgacgtctgt cgagaagttt ctgatcgaaa agttcgacag 4140
   cgtctccgac ctgatgcagc tctcggaggg cgaagaatct cgtgctttca gcttcgatgt 4200
   aggagggcgt ggatatgtcc tgcgggtaaa tagctgcgcc gatggtttct acaaagatcg 4260
   ttatgtttat cggcactttg catcggccgc gctcccgatt ccggaagtgc ttgacattgg 4320
   ggaattcagc gagagcctga cctattgcat ctcccgccgt gcacagggtg tcacgttgca 4380
   agacctgcct gaaaccgaac tgcccgctgt tctgcagccg gtcgcggagg ccatggatgc 4440
   gatcgctgcg gccgatctta gccagacgag cgggttcggc ccattcggac cgcaaggaat 4500
   cggtcaatac actacatggc gtgatttcat atgcgcgatt gctgatcccc atgtgtatca 4560
   ctggcaaact gtgatggacg acaccgtcag tgcgtccgtc gcgcaggctc tcgatgagct 4620
   gatgctttgg gccgaggact gccccgaagt ccggcacctc gtgcacgcgg atttcggctc 4680
   caacaatgtc ctgacggaca atggccgcat aacagcggtc attgactgga gcgaggcgat 4740
   gttcggggat tcccaatacg aggtcgccaa catcttcttc tggaggccgt ggttggcttg 4800
   tatggagcag cagacgcgct acttcgagcg gaggcatccg gagcttgcag gatcgccgcg 4860
   gctccgggcg tatatgctcc gcattggtct tgaccaactc tatcagagct tggttgacgg 4920
   caatttcgat gatgcagctt gggcgcaggg tcgatgcgac gcaatcgtcc gatccggagc 4980
   cgggactgtc gggcgtacac aaatcgcccg cagaagcgcg gccgtctgga ccgatggctg 5040
   tgtagaagta ctcgccgata gtggaaaccg acgccccagc actcgtccga gggcaaagga 5100
   atagcacgtg ctacgagatt tcgattccac cgccgccttc tatgaaaggt tgggcttcgg 5160
   aatcgttttc cgggacgccg gctggatgat cctccagcgc ggggatctca tgctggagtt 5220
   cttcgcccac cccaacttgt ttattgcagc ttataatggt tacaaataaa gcaatagcat 5280
   cacaaatttc acaaataaag catttttttc actgcattct agttgtggtt tgtccaaact 5340
   catcaatgta tcttatcatg tctgtatacc gtcgacctct agctagagct tggcgtaatc 5400
   atggtcatag ctgtttcctg tgtgaaattg ttatccgctc acaattccac acaacatacg 5460
   agccggaagc ataaagtgta aagcctgggg tgcctaatga gtgagctaac tcacattaat 5520
   tgcgttgcgc tcactgcccg ctttccagtc gggaaacctg tcgtgccagc tgcattaatg   5580
   aatcggccaa cgcgcgggga gaggcggttt gcgtattggg cgctcttccg cttcctcgct   5640
   cactgactcg ctgcgctcgg tcgttcggct gcggcgagcg gtatcagctc actcaaaggc   5700
   ggtaatacgg ttatccacag aatcagggga taacgcagga aagaacatgt gagcaaaagg   5760
   ccagcaaaag gccaggaacc gtaaaaaggc cgcgttgctg gcgtttttcc ataggctccg   5820
   cccccctgac gagcatcaca aaaatcgacg ctcaagtcag aggtggcgaa acccgacagg   5880
   actataaaga taccaggcgt ttccccctgg aagctccctc gtgcgctctc ctgttccgac   5940
   cctgccgctt accggatacc tgtccgcctt tctcccttcg ggaagcgtgg cgctttctca   6000
   atgctcacgc tgtaggtatc tcagttcggt gtaggtcgtt cgctccaagc tgggctgtgt   6060
   gcacgaaccc cccgttcagc ccgaccgctg cgccttatcc ggtaactatc gtcttgagtc   6120
   caacccggta agacacgact tatcgccact ggcagcagcc actggtaaca ggattagcag   6180
   agcgaggtat gtaggcggtg ctacagagtt cttgaagtgg tggcctaact acggctacac   6240
   tagaaggaca gtatttggta tctgcgctct gctgaagcca gttaccttcg gaaaaagagt   6300
   tggtagctct tgatccggca aacaaaccac cgctggtagc ggtggttttt ttgtttgcaa   6360
   gcagcagatt acgcgcagaa aaaaaggatc tcaagaagat cctttgatct tttctacggg   6420
   gtctgacgct cagtggaacg aaaactcacg ttaagggatt ttggtcatga gattatcaaa   6480
   aaggatcttc acctagatcc ttttaaatta aaaatgaagt tttaaatcaa tctaaagtat   6540
   atatgagtaa acttggtctg acagttacca atgcttaatc agtgaggcac ctatctcagc   6600
   gatctgtcta tttcgttcat ccatagttgc ctgactcccc gtcgtgtaga taactacgat   6660
   acgggagggc ttaccatctg gccccagtgc tgcaatgata ccgcgagacc cacgctcacc   6720
   ggctccagat ttatcagcaa taaaccagcc agccggaagg gccgagcgca gaagtggtcc   6780
   tgcaacttta tccgcctcca tccagtctat taattgttgc cgggaagcta gagtaagtag   6840
   ttcgccagtt aatagtttgc gcaacgttgt tgccattgct acaggcatcg tggtgtcacg   6900
   ctcgtcgttt ggtatggctt cattcagctc cggttcccaa cgatcaaggc gagttacatg   6960
   atcccccatg ttgtgcaaaa aagcggttag ctccttcggt cctccgatcg ttgtcagaag   7020
   taagttggcc gcagtgttat cactcatggt tatggcagca ctgcataatt ctcttactgt   7080
   catgccatcc gtaagatgct tttctgtgac tggtgagtac tcaaccaagt cattctgaga   7140
   atagtgtatg cggcgaccga gttgctcttg cccggcgtca atacgggata ataccgcgcc   7200
   acatagcaga actttaaaag tgctcatcat tggaaaacgt tcttcggggc gaaaactctc   7260
   aaggatctta ccgctgttga gatccagttc gatgtaaccc actcgtgcac ccaactgatc   7320
   ttcagcatct tttactttca ccagcgtttc tgggtgagca aaaacaggaa ggcaaaatgc   7380
   cgcaaaaaag ggaataaggg cgacacggaa atgttgaata ctcatactct tcctttttca 7440
   atattattga agcatttatc agggttattg tctcatgagc ggatacatat ttgaatgtat 7500
   ttagaaaaat aaacaaatag gggttccgcg cacatttccc cgaaaagtgc cacctgacgt 7560
   c 7561
<210> 8
   <211> 6082
   <212> DNA
   <213> Artificial Sequence
<220>
   <223> Plasmid
<400> 8
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   aagcttggat ctcaccatga gggtccctgc tcagctcctg ggactcctgc tgctctggct 960
   cccagatacc agatgtgaca tccagatgac ccagtctcca tcctccctgt ctgcatctgt 1020
   aggagacaga gtcaccatca cttgccgggc gagtcagggc attagcaatt atttagcctg 1080
   gtatcagcag aaaacaggga aagttcctaa gttcctgatc tatgaagcat ccactttgca 1140
   atcaggggtc ccatctcggt tcagtggcgg tggatctggg acagatttca ctctcaccat 1200
   cagcagcctg cagcctgaag atgttgcaac ttattactgt caaaattata acagtgcccc 1260
   attcactttc ggccctggga ccaaagtgga tatcaaacga actgtggctg caccctctgt 1320
   cttcatcttc ccgccatctg atgagcagtt gaaatctgga actgctagcg ttgtgtgcct 1380
   gctgaataac ttctatccca gagaggccaa agtacagtgg aaggtggata acgccctcca 1440
   atcgggtaac tcccaggaga gtgtcacaga gcaggacagc aaggacagca cctacagcct 1500
   cagcagcacc ctgacgctga gcaaagcaga ctacgagaaa cacaaagtct acgcctgcga 1560
   agtcacccat cagggcctga gctcgcccgt cacaaagagc ttcaacaggg gagagtgtta 1620
   ggaattcgcg gccgctcgag tctagagggc ccgtttaaac ccgctgatca gcctcgactg 1680
   tgccttctag ttgccagcca tctgttgttt gcccctcccc cgtgccttcc ttgaccctgg 1740
   aaggtgccac tcccactgtc ctttcctaat aaaatgagga aattgcatcg cattgtctga 1800
   gtaggtgtca ttctattctg gggggtgggg tggggcagga cagcaagggg gaggattggg 1860
   aagacaatag caggcatgct ggggatgcgg tgggctctat ggcttctgag gcggaaagaa 1920
   ccagctgggg ctctaggggg tatccccacg cgccctgtag cggcgcatta agcgcggcgg 1980
   gtgtggtggt tacgcgcagc gtgaccgcta cacttgccag cgccctagcg cccgctcctt 2040
   tcgctttctt cccttccttt ctcgccacgt tcgccggctt tccccgtcaa gctctaaatc 2100
   ggggcatccc tttagggttc cgatttagtg ctttacggca cctcgacccc aaaaaacttg 2160
   attagggtga tggttcacgt agtgggccat cgccctgata gacggttttt cgccctttga 2220
   cgttggagtc cacgttcttt aatagtggac tcttgttcca aactggaaca acactcaacc 2280
   ctatctcggt ctattctttt gatttataag ggattttggg gatttcggcc tattggttaa 2340
   aaaatgagct gatttaacaa aaatttaacg cgaattaatt ctgtggaatg tgtgtcagtt 2400
   agggtgtgga aagtccccag gctccccagg caggcagaag tatgcaaagc atgcatctca 2460
   attagtcagc aaccaggtgt ggaaagtccc caggctcccc agcaggcaga agtatgcaaa 2520
   gcatgcatct caattagtca gcaaccatag tcccgcccct aactccgccc atcccgcccc 2580
   taactccgcc cagttccgcc cattctccgc cccatggctg actaattttt tttatttatg 2640
   cagaggccga ggccgcctct gcctctgagc tattccagaa gtagtgagga ggcttttttg 2700
   gaggcctagg cttttgcaaa aagctcccgg gagcttgtat atccattttc ggatctgatc 2760
   aagagacagg atgaggatcg tttcgcatga ttgaacaaga tggattgcac gcaggttctc 2820
   cggccgcttg ggtggagagg ctattcggct atgactgggc acaacagaca atcggctgct 2880
   ctgatgccgc cgtgttccgg ctgtcagcgc aggggcgccc ggttcttttt gtcaagaccg 2940
   acctgtccgg tgccctgaat gaactgcagg acgaggcagc gcggctatcg tggctggcca 3000
   cgacgggcgt tccttgcgca gctgtgctcg acgttgtcac tgaagcggga agggactggc 3060
   tgctattggg cgaagtgccg gggcaggatc tcctgtcatc tcaccttgct cctgccgaga 3120
   aagtatccat catggctgat gcaatgcggc ggctgcatac gcttgatccg gctacctgcc 3180
   cattcgacca ccaagcgaaa catcgcatcg agcgagcacg tactcggatg gaagccggtc 3240
   ttgtcgatca ggatgatctg gacgaagagc atcaggggct cgcgccagcc gaactgttcg 3300
   ccaggctcaa ggcgcgcatg cccgacggcg aggatctcgt cgtgacccat ggcgatgcct 3360
   gcttgccgaa tatcatggtg gaaaatggcc gcttttctgg attcatcgac tgtggccggc 3420
   tgggtgtggc ggaccgctat caggacatag cgttggctac ccgtgatatt gctgaagagc 3480
   ttggcggcga atgggctgac cgcttcctcg tgctttacgg tatcgccgct cccgattcgc 3540
   agcgcatcgc cttctatcgc cttcttgacg agttcttctg agcgggactc tggggttcga 3600
   aatgaccgac caagcgacgc ccaacctgcc atcacgagat ttcgattcca ccgccgcctt 3660
   ctatgaaagg ttgggcttcg gaatcgtttt ccgggacgcc ggctggatga tcctccagcg 3720
   cggggatctc atgctggagt tcttcgccca ccccaacttg tttattgcag cttataatgg 3780
   ttacaaataa agcaatagca tcacaaattt cacaaataaa gcattttttt cactgcattc 3840
   tagttgtggt ttgtccaaac tcatcaatgt atcttatcat gtctgtatac cgtcgacctc 3900
   tagctagagc ttggcgtaat catggtcata gctgtttcct gtgtgaaatt gttatccgct 3960
   cacaattcca cacaacatac gagccggaag cataaagtgt aaagcctggg gtgcctaatg 4020
   agtgagctaa ctcacattaa ttgcgttgcg ctcactgccc gctttccagt cgggaaacct 4080
   gtcgtgccag ctgcattaat gaatcggcca acgcgcgggg agaggcggtt tgcgtattgg 4140
   gcgctcttcc gcttcctcgc tcactgactc gctgcgctcg gtcgttcggc tgcggcgagc 4200
   ggtatcagct cactcaaagg cggtaatacg gttatccaca gaatcagggg ataacgcagg 4260
   aaagaacatg tgagcaaaag gccagcaaaa ggccaggaac cgtaaaaagg ccgcgttgct 4320
   ggcgtttttc cataggctcc gcccccctga cgagcatcac aaaaatcgac gctcaagtca 4380
   gaggtggcga aacccgacag gactataaag ataccaggcg tttccccctg gaagctccct 4440
   cgtgcgctct cctgttccga ccctgccgct taccggatac ctgtccgcct ttctcccttc 4500
   gggaagcgtg gcgctttctc aatgctcacg ctgtaggtat ctcagttcgg tgtaggtcgt 4560
   tcgctccaag ctgggctgtg tgcacgaacc ccccgttcag cccgaccgct gcgccttatc 4620
   cggtaactat cgtcttgagt ccaacccggt aagacacgac ttatcgccac tggcagcagc 4680
   cactggtaac aggattagca gagcgaggta tgtaggcggt gctacagagt tcttgaagtg 4740
   gtggcctaac tacggctaca ctagaaggac agtatttggt atctgcgctc tgctgaagcc 4800
   agttaccttc ggaaaaagag ttggtagctc ttgatccggc aaacaaacca ccgctggtag 4860
   cggtggtttt tttgtttgca agcagcagat tacgcgcaga aaaaaaggat ctcaagaaga 4920
   tcctttgatc ttttctacgg ggtctgacgc tcagtggaac gaaaactcac gttaagggat 4980
   tttggtcatg agattatcaa aaaggatctt cacctagatc cttttaaatt aaaaatgaag 5040
   ttttaaatca atctaaagta tatatgagta aacttggtct gacagttacc aatgcttaat 5100
   cagtgaggca cctatctcag cgatctgtct atttcgttca tccatagttg cctgactccc 5160
   cgtcgtgtag ataactacga tacgggaggg cttaccatct ggccccagtg ctgcaatgat 5220
   accgcgagac ccacgctcac cggctccaga tttatcagca ataaaccagc cagccggaag 5280
   ggccgagcgc agaagtggtc ctgcaacttt atccgcctcc atccagtcta ttaattgttg 5340
   ccgggaagct agagtaagta gttcgccagt taatagtttg cgcaacgttg ttgccattgc 5400
   tacaggcatc gtggtgtcac gctcgtcgtt tggtatggct tcattcagct ccggttccca 5460
   acgatcaagg cgagttacat gatcccccat gttgtgcaaa aaagcggtta gctccttcgg 5520
   tcctccgatc gttgtcagaa gtaagttggc cgcagtgtta tcactcatgg ttatggcagc 5580
   actgcataat tctcttactg tcatgccatc cgtaagatgc ttttctgtga ctggtgagta 5640
   ctcaaccaag tcattctgag aatagtgtat gcggcgaccg agttgctctt gcccggcgtc 5700
   aatacgggat aataccgcgc cacatagcag aactttaaaa gtgctcatca ttggaaaacg 5760
   ttcttcgggg cgaaaactct caaggatctt accgctgttg agatccagtt cgatgtaacc 5820
   cactcgtgca cccaactgat cttcagcatc ttttactttc accagcgttt ctgggtgagc 5880
   aaaaacagga aggcaaaatg ccgcaaaaaa gggaataagg gcgacacgga aatgttgaat 5940
   actcatactc ttcctttttc aatattattg aagcatttat cagggttatt gtctcatgag 6000
   cggatacata tttgaatgta tttagaaaaa taaacaaata ggggttccgc gcacatttcc 6060
   ccgaaaagtg ccacctgacg tc 6082
<210> 9
   <211> 6082
   <212> DNA
   <213> Artificial Sequence
<220>
   <223> Plasmid
<400> 9
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   aagcttggat ctcaccatga gggtccccgc tcagctcctg gggctcctgc tgctctgttt 960
   cccaggtgcc agatgtgaca tccagatgac ccagtctcca tcctcactgt ctgcatctgt 1020
   aggagacaga gtcaccatca cttgtcgggc gagtcagggc attaccaatt atttagcctg 1080
   gtttcagcag aaaccaggga aagcccctaa gtcccttatc tatgctgcat ccagtttgca 1140
   aagtggggtc ccatcaaagt tcagcggcag tggatctggg acagatttca gtctcaccat 1200
   cagcagcctg cagcctgaag attttgcaac ttattactgc caacagtata atagttaccc 1260
   gatcaccttc ggccaaggga cacgactgga gattaaacga actgtggctg caccatctgt 1320
   cttcatcttc ccgccatctg atgagcagtt gaaatctgga actgctagcg ttgtgtgcct 1380
   gctgaataac ttctatccca gagaggccaa agtacagtgg aaggtggata acgccctcca 1440
   atcgggtaac tcccaggaga gtgtcacaga gcaggacagc aaggacagca cctacagcct 1500
   cagcagcacc ctgacgctga gcaaagcaga ctacgagaaa cacaaagtct acgcctgcga 1560
   agtcacccat cagggcctga gctcgcccgt cacaaagagc ttcaacaggg gagagtgtta 1620
   ggaattcgcg gccgctcgag tctagagggc ccgtttaaac ccgctgatca gcctcgactg 1680
   tgccttctag ttgccagcca tctgttgttt gcccctcccc cgtgccttcc ttgaccctgg 1740
   aaggtgccac tcccactgtc ctttcctaat aaaatgagga aattgcatcg cattgtctga 1800
   gtaggtgtca ttctattctg gggggtgggg tggggcagga cagcaagggg gaggattggg 1860
   aagacaatag caggcatgct ggggatgcgg tgggctctat ggcttctgag gcggaaagaa 1920
   ccagctgggg ctctaggggg tatccccacg cgccctgtag cggcgcatta agcgcggcgg 1980
   gtgtggtggt tacgcgcagc gtgaccgcta cacttgccag cgccctagcg cccgctcctt 2040
   tcgctttctt cccttccttt ctcgccacgt tcgccggctt tccccgtcaa gctctaaatc 2100
   ggggcatccc tttagggttc cgatttagtg ctttacggca cctcgacccc aaaaaacttg 2160
   attagggtga tggttcacgt agtgggccat cgccctgata gacggttttt cgccctttga 2220
   cgttggagtc cacgttcttt aatagtggac tcttgttcca aactggaaca acactcaacc 2280
   ctatctcggt ctattctttt gatttataag ggattttggg gatttcggcc tattggttaa 2340
   aaaatgagct gatttaacaa aaatttaacg cgaattaatt ctgtggaatg tgtgtcagtt 2400
   agggtgtgga aagtccccag gctccccagg caggcagaag tatgcaaagc atgcatctca 2460
   attagtcagc aaccaggtgt ggaaagtccc caggctcccc agcaggcaga agtatgcaaa 2520
   gcatgcatct caattagtca gcaaccatag tcccgcccct aactccgccc atcccgcccc 2580
   taactccgcc cagttccgcc cattctccgc cccatggctg actaattttt tttatttatg 2640
   cagaggccga ggccgcctct gcctctgagc tattccagaa gtagtgagga ggcttttttg 2700
   gaggcctagg cttttgcaaa aagctcccgg gagcttgtat atccattttc ggatctgatc 2760
   aagagacagg atgaggatcg tttcgcatga ttgaacaaga tggattgcac gcaggttctc 2820
   cggccgcttg ggtggagagg ctattcggct atgactgggc acaacagaca atcggctgct 2880
   ctgatgccgc cgtgttccgg ctgtcagcgc aggggcgccc ggttcttttt gtcaagaccg 2940
   acctgtccgg tgccctgaat gaactgcagg acgaggcagc gcggctatcg tggctggcca 3000
   cgacgggcgt tccttgcgca gctgtgctcg acgttgtcac tgaagcggga agggactggc 3060
   tgctattggg cgaagtgccg gggcaggatc tcctgtcatc tcaccttgct cctgccgaga 3120
   aagtatccat catggctgat gcaatgcggc ggctgcatac gcttgatccg gctacctgcc 3180
   cattcgacca ccaagcgaaa catcgcatcg agcgagcacg tactcggatg gaagccggtc 3240
   ttgtcgatca ggatgatctg gacgaagagc atcaggggct cgcgccagcc gaactgttcg 3300
   ccaggctcaa ggcgcgcatg cccgacggcg aggatctcgt cgtgacccat ggcgatgcct 3360
   gcttgccgaa tatcatggtg gaaaatggcc gcttttctgg attcatcgac tgtggccggc 3420
   tgggtgtggc ggaccgctat caggacatag cgttggctac ccgtgatatt gctgaagagc 3480
   ttggcggcga atgggctgac cgcttcctcg tgctttacgg tatcgccgct cccgattcgc 3540
   agcgcatcgc cttctatcgc cttcttgacg agttcttctg agcgggactc tggggttcga 3600
   aatgaccgac caagcgacgc ccaacctgcc atcacgagat ttcgattcca ccgccgcctt 3660
   ctatgaaagg ttgggcttcg gaatcgtttt ccgggacgcc ggctggatga tcctccagcg 3720
   cggggatctc atgctggagt tcttcgccca ccccaacttg tttattgcag cttataatgg 3780
   ttacaaataa agcaatagca tcacaaattt cacaaataaa gcattttttt cactgcattc 3840
   tagttgtggt ttgtccaaac tcatcaatgt atcttatcat gtctgtatac cgtcgacctc 3900
   tagctagagc ttggcgtaat catggtcata gctgtttcct gtgtgaaatt gttatccgct 3960
   cacaattcca cacaacatac gagccggaag cataaagtgt aaagcctggg gtgcctaatg 4020
   agtgagctaa ctcacattaa ttgcgttgcg ctcactgccc gctttccagt cgggaaacct 4080
   gtcgtgccag ctgcattaat gaatcggcca acgcgcgggg agaggcggtt tgcgtattgg 4140
   gcgctcttcc gcttcctcgc tcactgactc gctgcgctcg gtcgttcggc tgcggcgagc 4200
   ggtatcagct cactcaaagg cggtaatacg gttatccaca gaatcagggg ataacgcagg 4260
   aaagaacatg tgagcaaaag gccagcaaaa ggccaggaac cgtaaaaagg ccgcgttgct 4320
   ggcgtttttc cataggctcc gcccccctga cgagcatcac aaaaatcgac gctcaagtca 4380
   gaggtggcga aacccgacag gactataaag ataccaggcg tttccccctg gaagctccct 4440
   cgtgcgctct cctgttccga ccctgccgct taccggatac ctgtccgcct ttctcccttc 4500
   gggaagcgtg gcgctttctc aatgctcacg ctgtaggtat ctcagttcgg tgtaggtcgt 4560
   tcgctccaag ctgggctgtg tgcacgaacc ccccgttcag cccgaccgct gcgccttatc 4620
   cggtaactat cgtcttgagt ccaacccggt aagacacgac ttatcgccac tggcagcagc 4680
   cactggtaac aggattagca gagcgaggta tgtaggcggt gctacagagt tcttgaagtg 4740
   gtggcctaac tacggctaca ctagaaggac agtatttggt atctgcgctc tgctgaagcc 4800
   agttaccttc ggaaaaagag ttggtagctc ttgatccggc aaacaaacca ccgctggtag 4860
   cggtggtttt tttgtttgca agcagcagat tacgcgcaga aaaaaaggat ctcaagaaga 4920
   tcctttgatc ttttctacgg ggtctgacgc tcagtggaac gaaaactcac gttaagggat 4980
   tttggtcatg agattatcaa aaaggatctt cacctagatc cttttaaatt aaaaatgaag 5040
   ttttaaatca atctaaagta tatatgagta aacttggtct gacagttacc aatgcttaat 5100
   cagtgaggca cctatctcag cgatctgtct atttcgttca tccatagttg cctgactccc 5160
   cgtcgtgtag ataactacga tacgggaggg cttaccatct ggccccagtg ctgcaatgat 5220
   accgcgagac ccacgctcac cggctccaga tttatcagca ataaaccagc cagccggaag 5280
   ggccgagcgc agaagtggtc ctgcaacttt atccgcctcc atccagtcta ttaattgttg 5340
   ccgggaagct agagtaagta gttcgccagt taatagtttg cgcaacgttg ttgccattgc 5400
   tacaggcatc gtggtgtcac gctcgtcgtt tggtatggct tcattcagct ccggttccca 5460
   acgatcaagg cgagttacat gatcccccat gttgtgcaaa aaagcggtta gctccttcgg 5520
   tcctccgatc gttgtcagaa gtaagttggc cgcagtgtta tcactcatgg ttatggcagc 5580
   actgcataat tctcttactg tcatgccatc cgtaagatgc ttttctgtga ctggtgagta   5640
   ctcaaccaag tcattctgag aatagtgtat gcggcgaccg agttgctctt gcccggcgtc   5700
   aatacgggat aataccgcgc cacatagcag aactttaaaa gtgctcatca ttggaaaacg   5760
   ttcttcgggg cgaaaactct caaggatctt accgctgttg agatccagtt cgatgtaacc   5820
   cactcgtgca cccaactgat cttcagcatc ttttactttc accagcgttt ctgggtgagc   5880
   aaaaacagga aggcaaaatg ccgcaaaaaa gggaataagg gcgacacgga aatgttgaat   5940
   actcatactc ttcctttttc aatattattg aagcatttat cagggttatt gtctcatgag   6000
   cggatacata tttgaatgta tttagaaaaa taaacaaata ggggttccgc gcacatttcc   6060
   ccgaaaagtg ccacctgacg tc                                            6082
<210> 10
   <211> 6082
   <212> DNA
   <213> Artificial Sequence
<220>
   <223> Plasmid
<400> 10
   gacggatcgg gagatctccc gatcccctat ggtcgactct cagtacaatc tgctctgatg     60
   ccgcatagtt aagccagtat ctgctccctg cttgtgtgtt ggaggtcgct gagtagtgcg    120
   cgagcaaaat ttaagctaca acaaggcaag gcttgaccga caattgcatg aagaatctgc    180
   ttagggttag gcgttttgcg ctgcttcgcg atgtacgggc cagatatacg cgttgacatt    240
   gattattgac tagttattaa tagtaatcaa ttacggggtc attagttcat agcccatata    300
   tggagttccg cgttacataa cttacggtaa atggcccgcc tggctgaccg cccaacgacc    360
   cccgcccatt gacgtcaata atgacgtatg ttcccatagt aacgccaata gggactttcc    420
   attgacgtca atgggtggac tatttacggt aaactgccca cttggcagta catcaagtgt    480
   atcatatgcc aagtacgccc cctattgacg tcaatgacgg taaatggccc gcctggcatt    540
   atgcccagta catgacctta tgggactttc ctacttggca gtacatctac gtattagtca    600
   tcgctattac catggtgatg cggttttggc agtacatcaa tgggcgtgga tagcggtttg    660
   actcacgggg atttccaagt ctccacccca ttgacgtcaa tgggagtttg ttttggcacc    720
   aaaatcaacg ggactttcca aaatgtcgta acaactccgc cccattgacg caaatgggcg    780
   gtaggcgtgt acggtgggag gtctatataa gcagagctct ctggctaact agagaaccca    840
   ctgcttactg gcttatcgaa attaatacga ctcactatag ggagacccaa gctggctaga    900
   aagcttggat ctcaccatga gggtccctgc tcagctcctg gggctcctgc tgctctgttt 960
   cccaggtgcc agatgtgaca tccagatgac ccagtctcca tcctcactgt ctgcatctgt 1020
   aggagacaga gtcaccatca cttgtcgggc gagtcagggc attagccatt atttagcctg 1080
   gtttcagcag aaaccaggga aagcccctaa gtccctgatc tatgctgcat ccagtttgca 1140
   aagtggggtc ccatcaaagt tcagcggcag tggatctggg acagatttca ctctcaccat 1200
   cagcagccta cagcctgaag attttgcaac ttattactgc caacagtata atagtttccc 1260
   gctcactttc ggcggaggga ccaaggtgga gatcaaacga actgtggctg caccatctgt 1320
   cttcatcttc ccgccatctg atgagcagtt gaaatctgga actgctagcg ttgtgtgcct 1380
   gctgaataac ttctatccca gagaggccaa agtacagtgg aaggtggata acgccctcca 1440
   atcgggtaac tcccaggaga gtgtcacaga gcaggacagc aaggacagca cctacagcct 1500
   cagcagcacc ctgacgctga gcaaagcaga ctacgagaaa cacaaagtct acgcctgcga 1560
   agtcacccat cagggcctga gctcgcccgt cacaaagagc ttcaacaggg gagagtgtta 1620
   ggaattcgcg gccgctcgag tctagagggc ccgtttaaac ccgctgatca gcctcgactg 1680
   tgccttctag ttgccagcca tctgttgttt gcccctcccc cgtgccttcc ttgaccctgg 1740
   aaggtgccac tcccactgtc ctttcctaat aaaatgagga aattgcatcg cattgtctga 1800
   gtaggtgtca ttctattctg gggggtgggg tggggcagga cagcaagggg gaggattggg 1860
   aagacaatag caggcatgct ggggatgcgg tgggctctat ggcttctgag gcggaaagaa 1920
   ccagctgggg ctctaggggg tatccccacg cgccctgtag cggcgcatta agcgcggcgg 1980
   gtgtggtggt tacgcgcagc gtgaccgcta cacttgccag cgccctagcg cccgctcctt 2040
   tcgctttctt cccttccttt ctcgccacgt tcgccggctt tccccgtcaa gctctaaatc 2100
   ggggcatccc tttagggttc cgatttagtg ctttacggca cctcgacccc aaaaaacttg 2160
   attagggtga tggttcacgt agtgggccat cgccctgata gacggttttt cgccctttga 2220
   cgttggagtc cacgttcttt aatagtggac tcttgttcca aactggaaca acactcaacc 2280
   ctatctcggt ctattctttt gatttataag ggattttggg gatttcggcc tattggttaa 2340
   aaaatgagct gatttaacaa aaatttaacg cgaattaatt ctgtggaatg tgtgtcagtt 2400
   agggtgtgga aagtccccag gctccccagg caggcagaag tatgcaaagc atgcatctca 2460
   attagtcagc aaccaggtgt ggaaagtccc caggctcccc agcaggcaga agtatgcaaa 2520
   gcatgcatct caattagtca gcaaccatag tcccgcccct aactccgccc atcccgcccc 2580
   taactccgcc cagttccgcc cattctccgc cccatggctg actaattttt tttatttatg 2640
   cagaggccga ggccgcctct gcctctgagc tattccagaa gtagtgagga ggcttttttg 2700
   gaggcctagg cttttgcaaa aagctcccgg gagcttgtat atccattttc ggatctgatc 2760
   aagagacagg atgaggatcg tttcgcatga ttgaacaaga tggattgcac gcaggttctc 2820
   cggccgcttg ggtggagagg ctattcggct atgactgggc acaacagaca atcggctgct 2880
   ctgatgccgc cgtgttccgg ctgtcagcgc aggggcgccc ggttcttttt gtcaagaccg 2940
   acctgtccgg tgccctgaat gaactgcagg acgaggcagc gcggctatcg tggctggcca 3000
   cgacgggcgt tccttgcgca gctgtgctcg acgttgtcac tgaagcggga agggactggc 3060
   tgctattggg cgaagtgccg gggcaggatc tcctgtcatc tcaccttgct cctgccgaga 3120
   aagtatccat catggctgat gcaatgcggc ggctgcatac gcttgatccg gctacctgcc 3180
   cattcgacca ccaagcgaaa catcgcatcg agcgagcacg tactcggatg gaagccggtc 3240
   ttgtcgatca ggatgatctg gacgaagagc atcaggggct cgcgccagcc gaactgttcg 3300
   ccaggctcaa ggcgcgcatg cccgacggcg aggatctcgt cgtgacccat ggcgatgcct 3360
   gcttgccgaa tatcatggtg gaaaatggcc gcttttctgg attcatcgac tgtggccggc 3420
   tgggtgtggc ggaccgctat caggacatag cgttggctac ccgtgatatt gctgaagagc 3480
   ttggcggcga atgggctgac cgcttcctcg tgctttacgg tatcgccgct cccgattcgc 3540
   agcgcatcgc cttctatcgc cttcttgacg agttcttctg agcgggactc tggggttcga 3600
   aatgaccgac caagcgacgc ccaacctgcc atcacgagat ttcgattcca ccgccgcctt 3660
   ctatgaaagg ttgggcttcg gaatcgtttt ccgggacgcc ggctggatga tcctccagcg 3720
   cggggatctc atgctggagt tcttcgccca ccccaacttg tttattgcag cttataatgg 3780
   ttacaaataa agcaatagca tcacaaattt cacaaataaa gcattttttt cactgcattc 3840
   tagttgtggt ttgtccaaac tcatcaatgt atcttatcat gtctgtatac cgtcgacctc 3900
   tagctagagc ttggcgtaat catggtcata gctgtttcct gtgtgaaatt gttatccgct 3960
   cacaattcca cacaacatac gagccggaag cataaagtgt aaagcctggg gtgcctaatg 4020
   agtgagctaa ctcacattaa ttgcgttgcg ctcactgccc gctttccagt cgggaaacct 4080
   gtcgtgccag ctgcattaat gaatcggcca acgcgcgggg agaggcggtt tgcgtattgg 4140
   gcgctcttcc gcttcctcgc tcactgactc gctgcgctcg gtcgttcggc tgcggcgagc 4200
   ggtatcagct cactcaaagg cggtaatacg gttatccaca gaatcagggg ataacgcagg 4260
   aaagaacatg tgagcaaaag gccagcaaaa ggccaggaac cgtaaaaagg ccgcgttgct 4320
   ggcgtttttc cataggctcc gcccccctga cgagcatcac aaaaatcgac gctcaagtca 4380
   gaggtggcga aacccgacag gactataaag ataccaggcg tttccccctg gaagctccct 4440
   cgtgcgctct cctgttccga ccctgccgct taccggatac ctgtccgcct ttctcccttc 4500
   gggaagcgtg gcgctttctc aatgctcacg ctgtaggtat ctcagttcgg tgtaggtcgt 4560
   tcgctccaag ctgggctgtg tgcacgaacc ccccgttcag cccgaccgct gcgccttatc 4620
   cggtaactat cgtcttgagt ccaacccggt aagacacgac ttatcgccac tggcagcagc 4680
   cactggtaac aggattagca gagcgaggta tgtaggcggt gctacagagt tcttgaagtg 4740
   gtggcctaac tacggctaca ctagaaggac agtatttggt atctgcgctc tgctgaagcc 4800
   agttaccttc ggaaaaagag ttggtagctc ttgatccggc aaacaaacca ccgctggtag 4860
   cggtggtttt tttgtttgca agcagcagat tacgcgcaga aaaaaaggat ctcaagaaga 4920
   tcctttgatc ttttctacgg ggtctgacgc tcagtggaac gaaaactcac gttaagggat 4980
   tttggtcatg agattatcaa aaaggatctt cacctagatc cttttaaatt aaaaatgaag 5040
   ttttaaatca atctaaagta tatatgagta aacttggtct gacagttacc aatgcttaat 5100
   cagtgaggca cctatctcag cgatctgtct atttcgttca tccatagttg cctgactccc 5160
   cgtcgtgtag ataactacga tacgggaggg cttaccatct ggccccagtg ctgcaatgat 5220
   accgcgagac ccacgctcac cggctccaga tttatcagca ataaaccagc cagccggaag 5280
   ggccgagcgc agaagtggtc ctgcaacttt atccgcctcc atccagtcta ttaattgttg 5340
   ccgggaagct agagtaagta gttcgccagt taatagtttg cgcaacgttg ttgccattgc 5400
   tacaggcatc gtggtgtcac gctcgtcgtt tggtatggct tcattcagct ccggttccca 5460
   acgatcaagg cgagttacat gatcccccat gttgtgcaaa aaagcggtta gctccttcgg 5520
   tcctccgatc gttgtcagaa gtaagttggc cgcagtgtta tcactcatgg ttatggcagc 5580
   actgcataat tctcttactg tcatgccatc cgtaagatgc ttttctgtga ctggtgagta 5640
   ctcaaccaag tcattctgag aatagtgtat gcggcgaccg agttgctctt gcccggcgtc 5700
   aatacgggat aataccgcgc cacatagcag aactttaaaa gtgctcatca ttggaaaacg 5760
   ttcttcgggg cgaaaactct caaggatctt accgctgttg agatccagtt cgatgtaacc 5820
   cactcgtgca cccaactgat cttcagcatc ttttactttc accagcgttt ctgggtgagc 5880
   aaaaacagga aggcaaaatg ccgcaaaaaa gggaataagg gcgacacgga aatgttgaat 5940
   actcatactc ttcctttttc aatattattg aagcatttat cagggttatt gtctcatgag 6000
   cggatacata tttgaatgta tttagaaaaa taaacaaata ggggttccgc gcacatttcc 6060
   ccgaaaagtg ccacctgacg tc 6082
<210> 11
   <211> 6085
   <212> DNA
   <213> Artificial Sequence
<220>
   <223> Plasmid
<400> 11
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   aagcttggat ctcaccatga gggtccccgc tcagcttctc ttccttctgc tactctggct 960
   cccagatacc actggaggaa tagtgatgac gcagtctcca gccaccctgt ctgtgtctcc 1020
   aggggaaaga gccaccctct cctgcaggac cagtcagagt attggctgga acttagcctg 1080
   gtaccaacag aaacctggcc aggctcccag gctcctcatc tatggtgcat cttccaggac 1140
   cactggtatc ccagccaggt tcagtggcag tgggtctggg acagagttca ctctcaccat 1200
   cagcagcctg cagtctgaag attctgcagt ttattactgt cagcattatg ataactggcc 1260
   catgtgcagt tttggccagg ggaccgagct ggagatcaaa cgaactgtgg ctgcaccatc 1320
   tgtcttcatc ttcccgccat ctgatgagca gttgaaatct ggaactgcta gcgttgtgtg 1380
   cctgctgaat aacttctatc ccagagaggc caaagtacag tggaaggtgg ataacgccct 1440
   ccaatcgggt aactcccagg agagtgtcac agagcaggac agcaaggaca gcacctacag 1500
   cctcagcagc accctgacgc tgagcaaagc agactacgag aaacacaaag tctacgcctg 1560
   cgaagtcacc catcagggcc tgagctcgcc cgtcacaaag agcttcaaca ggggagagtg 1620
   ttaggaattc gcggccgctc gagtctagag ggcccgttta aacccgctga tcagcctcga 1680
   ctgtgccttc tagttgccag ccatctgttg tttgcccctc ccccgtgcct tccttgaccc 1740
   tggaaggtgc cactcccact gtcctttcct aataaaatga ggaaattgca tcgcattgtc 1800
   tgagtaggtg tcattctatt ctggggggtg gggtggggca ggacagcaag ggggaggatt 1860
   gggaagacaa tagcaggcat gctggggatg cggtgggctc tatggcttct gaggcggaaa 1920
   gaaccagctg gggctctagg gggtatcccc acgcgccctg tagcggcgca ttaagcgcgg 1980
   cgggtgtggt ggttacgcgc agcgtgaccg ctacacttgc cagcgcccta gcgcccgctc 2040
   ctttcgcttt cttcccttcc tttctcgcca cgttcgccgg ctttccccgt caagctctaa 2100
   atcggggcat ccctttaggg ttccgattta gtgctttacg gcacctcgac cccaaaaaac 2160
   ttgattaggg tgatggttca cgtagtgggc catcgccctg atagacggtt tttcgccctt 2220
   tgacgttgga gtccacgttc tttaatagtg gactcttgtt ccaaactgga acaacactca 2280
   accctatctc ggtctattct tttgatttat aagggatttt ggggatttcg gcctattggt 2340
   taaaaaatga gctgatttaa caaaaattta acgcgaatta attctgtgga atgtgtgtca 2400
   gttagggtgt ggaaagtccc caggctcccc aggcaggcag aagtatgcaa agcatgcatc 2460
   tcaattagtc agcaaccagg tgtggaaagt ccccaggctc cccagcaggc agaagtatgc 2520
   aaagcatgca tctcaattag tcagcaacca tagtcccgcc cctaactccg cccatcccgc 2580
   ccctaactcc gcccagttcc gcccattctc cgccccatgg ctgactaatt ttttttattt 2640
   atgcagaggc cgaggccgcc tctgcctctg agctattcca gaagtagtga ggaggctttt 2700
   ttggaggcct aggcttttgc aaaaagctcc cgggagcttg tatatccatt ttcggatctg 2760
   atcaagagac aggatgagga tcgtttcgca tgattgaaca agatggattg cacgcaggtt 2820
   ctccggccgc ttgggtggag aggctattcg gctatgactg ggcacaacag acaatcggct 2880
   gctctgatgc cgccgtgttc cggctgtcag cgcaggggcg cccggttctt tttgtcaaga 2940
   ccgacctgtc cggtgccctg aatgaactgc aggacgaggc agcgcggcta tcgtggctgg 3000
   ccacgacggg cgttccttgc gcagctgtgc tcgacgttgt cactgaagcg ggaagggact 3060
   ggctgctatt gggcgaagtg ccggggcagg atctcctgtc atctcacctt gctcctgccg 3120
   agaaagtatc catcatggct gatgcaatgc ggcggctgca tacgcttgat ccggctacct 3180
   gcccattcga ccaccaagcg aaacatcgca tcgagcgagc acgtactcgg atggaagccg 3240
   gtcttgtcga tcaggatgat ctggacgaag agcatcaggg gctcgcgcca gccgaactgt 3300
   tcgccaggct caaggcgcgc atgcccgacg gcgaggatct cgtcgtgacc catggcgatg 3360
   cctgcttgcc gaatatcatg gtggaaaatg gccgcttttc tggattcatc gactgtggcc 3420
   ggctgggtgt ggcggaccgc tatcaggaca tagcgttggc tacccgtgat attgctgaag 3480
   agcttggcgg cgaatgggct gaccgcttcc tcgtgcttta cggtatcgcc gctcccgatt 3540
   cgcagcgcat cgccttctat cgccttcttg acgagttctt ctgagcggga ctctggggtt 3600
   cgaaatgacc gaccaagcga cgcccaacct gccatcacga gatttcgatt ccaccgccgc 3660
   cttctatgaa aggttgggct tcggaatcgt tttccgggac gccggctgga tgatcctcca 3720
   gcgcggggat ctcatgctgg agttcttcgc ccaccccaac ttgtttattg cagcttataa 3780
   tggttacaaa taaagcaata gcatcacaaa tttcacaaat aaagcatttt tttcactgca 3840
   ttctagttgt ggtttgtcca aactcatcaa tgtatcttat catgtctgta taccgtcgac 3900
   ctctagctag agcttggcgt aatcatggtc atagctgttt cctgtgtgaa attgttatcc 3960
   gctcacaatt ccacacaaca tacgagccgg aagcataaag tgtaaagcct ggggtgccta 4020
   atgagtgagc taactcacat taattgcgtt gcgctcactg cccgctttcc agtcgggaaa 4080
   cctgtcgtgc cagctgcatt aatgaatcgg ccaacgcgcg gggagaggcg gtttgcgtat 4140
   tgggcgctct tccgcttcct cgctcactga ctcgctgcgc tcggtcgttc ggctgcggcg 4200
   agcggtatca gctcactcaa aggcggtaat acggttatcc acagaatcag gggataacgc 4260
   aggaaagaac atgtgagcaa aaggccagca aaaggccagg aaccgtaaaa aggccgcgtt 4320
   gctggcgttt ttccataggc tccgcccccc tgacgagcat cacaaaaatc gacgctcaag 4380
   tcagaggtgg cgaaacccga caggactata aagataccag gcgtttcccc ctggaagctc 4440
   cctcgtgcgc tctcctgttc cgaccctgcc gcttaccgga tacctgtccg cctttctccc 4500
   ttcgggaagc gtggcgcttt ctcaatgctc acgctgtagg tatctcagtt cggtgtaggt 4560
   cgttcgctcc aagctgggct gtgtgcacga accccccgtt cagcccgacc gctgcgcctt 4620
   atccggtaac tatcgtcttg agtccaaccc ggtaagacac gacttatcgc cactggcagc 4680
   agccactggt aacaggatta gcagagcgag gtatgtaggc ggtgctacag agttcttgaa 4740
   gtggtggcct aactacggct acactagaag gacagtattt ggtatctgcg ctctgctgaa 4800
   gccagttacc ttcggaaaaa gagttggtag ctcttgatcc ggcaaacaaa ccaccgctgg 4860
   tagcggtggt ttttttgttt gcaagcagca gattacgcgc agaaaaaaag gatctcaaga 4920
   agatcctttg atcttttcta cggggtctga cgctcagtgg aacgaaaact cacgttaagg 4980
   gattttggtc atgagattat caaaaaggat cttcacctag atccttttaa attaaaaatg 5040
   aagttttaaa tcaatctaaa gtatatatga gtaaacttgg tctgacagtt accaatgctt 5100
   aatcagtgag gcacctatct cagcgatctg tctatttcgt tcatccatag ttgcctgact 5160
   ccccgtcgtg tagataacta cgatacggga gggcttacca tctggcccca gtgctgcaat 5220
   gataccgcga gacccacgct caccggctcc agatttatca gcaataaacc agccagccgg 5280
   aagggccgag cgcagaagtg gtcctgcaac tttatccgcc tccatccagt ctattaattg 5340
   ttgccgggaa gctagagtaa gtagttcgcc agttaatagt ttgcgcaacg ttgttgccat   5400
   tgctacaggc atcgtggtgt cacgctcgtc gtttggtatg gcttcattca gctccggttc   5460
   ccaacgatca aggcgagtta catgatcccc catgttgtgc aaaaaagcgg ttagctcctt   5520
   cggtcctccg atcgttgtca gaagtaagtt ggccgcagtg ttatcactca tggttatggc   5580
   agcactgcat aattctctta ctgtcatgcc atccgtaaga tgcttttctg tgactggtga   5640
   gtactcaacc aagtcattct gagaatagtg tatgcggcga ccgagttgct cttgcccggc   5700
   gtcaatacgg gataataccg cgccacatag cagaacttta aaagtgctca tcattggaaa   5760
   acgttcttcg gggcgaaaac tctcaaggat cttaccgctg ttgagatcca gttcgatgta   5820
   acccactcgt gcacccaact gatcttcagc atcttttact ttcaccagcg tttctgggtg   5880
   agcaaaaaca ggaaggcaaa atgccgcaaa aaagggaata agggcgacac ggaaatgttg   5940
   aatactcata ctcttccttt ttcaatatta ttgaagcatt tatcagggtt attgtctcat   6000
   gagcggatac atatttgaat gtatttagaa aaataaacaa ataggggttc cgcgcacatt   6060
   tccccgaaaa gtgccacctg acgtc                                         6085
<210> 12
   <211> 6097
   <212> DNA
   <213> Artificial Sequence
<220>
   <223> Plasmid
<400> 12
   gacggatcgg gagatctccc gatcccctat ggtcgactct cagtacaatc tgctctgatg     60
   ccgcatagtt aagccagtat ctgctccctg cttgtgtgtt ggaggtcgct gagtagtgcg    120
   cgagcaaaat ttaagctaca acaaggcaag gcttgaccga caattgcatg aagaatctgc    180
   ttagggttag gcgttttgcg ctgcttcgcg atgtacgggc cagatatacg cgttgacatt    240
   gattattgac tagttattaa tagtaatcaa ttacggggtc attagttcat agcccatata    300
   tggagttccg cgttacataa cttacggtaa atggcccgcc tggctgaccg cccaacgacc    360
   cccgcccatt gacgtcaata atgacgtatg ttcccatagt aacgccaata gggactttcc    420
   attgacgtca atgggtggac tatttacggt aaactgccca cttggcagta catcaagtgt    480
   atcatatgcc aagtacgccc cctattgacg tcaatgacgg taaatggccc gcctggcatt    540
   atgcccagta catgacctta tgggactttc ctacttggca gtacatctac gtattagtca    600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctaga 900
   aagcttggat ctcaccatga gggtccctgc tcagctcctg gggctgctaa tgctctggat 960
   acctggatcc agtgcagata ttgtgatgac ccagactcca ctctctctgt ccgtcacccc 1020
   tggacagccg gcctccatct cctgcaagtc tagtcagagc ctcctgcata gtgatggaaa 1080
   gacctttttg tattggtatc tgcagaagcc aggccagcct ccacagctcc tgatctatga 1140
   ggtttccaac cggttctctg gagtgccaga taggttcagt ggcagcgggt cagggacaga 1200
   tttcacactg aaaatcagcc gggtggaggc tgaggatgtt gggctttatt actgcatgca 1260
   aagtatacag cttccgctca ctttcggcgg agggaccaag gtggagatca aacgaactgt 1320
   ggctgcacca tctgtcttca tcttcccgcc atctgatgag cagttgaaat ctggaactgc 1380
   tagcgttgtg tgcctgctga ataacttcta tcccagagag gccaaagtac agtggaaggt 1440
   ggataacgcc ctccaatcgg gtaactccca ggagagtgtc acagagcagg acagcaagga 1500
   cagcacctac agcctcagca gcaccctgac gctgagcaaa gcagactacg agaaacacaa 1560
   agtctacgcc tgcgaagtca cccatcaggg cctgagctcg cccgtcacaa agagcttcaa 1620
   caggggagag tgttaggaat tcgcggccgc tcgagtctag agggcccgtt taaacccgct 1680
   gatcagcctc gactgtgcct tctagttgcc agccatctgt tgtttgcccc tcccccgtgc 1740
   cttccttgac cctggaaggt gccactccca ctgtcctttc ctaataaaat gaggaaattg 1800
   catcgcattg tctgagtagg tgtcattcta ttctgggggg tggggtgggg caggacagca 1860
   agggggagga ttgggaagac aatagcaggc atgctgggga tgcggtgggc tctatggctt 1920
   ctgaggcgga aagaaccagc tggggctcta gggggtatcc ccacgcgccc tgtagcggcg 1980
   cattaagcgc ggcgggtgtg gtggttacgc gcagcgtgac cgctacactt gccagcgccc 2040
   tagcgcccgc tcctttcgct ttcttccctt cctttctcgc cacgttcgcc ggctttcccc 2100
   gtcaagctct aaatcggggc atccctttag ggttccgatt tagtgcttta cggcacctcg 2160
   accccaaaaa acttgattag ggtgatggtt cacgtagtgg gccatcgccc tgatagacgg 2220
   tttttcgccc tttgacgttg gagtccacgt tctttaatag tggactcttg ttccaaactg 2280
   gaacaacact caaccctatc tcggtctatt cttttgattt ataagggatt tggggatttt 2340
   cggcctattg gttaaaaaat gagctgattt aacaaaaatt taacgcgaat taattctgtg 2400
   gaatgtgtgt cagttagggt gtggaaagtc cccaggctcc ccaggcaggc agaagtatgc 2460
   aaagcatgca tctcaattag tcagcaacca ggtgtggaaa gtccccaggc tccccagcag 2520
   gcagaagtat gcaaagcatg catctcaatt agtcagcaac catagtcccg cccctaactc 2580
   cgcccatccc gcccctaact ccgcccagtt ccgcccattc tccgccccat ggctgactaa 2640
   ttttttttat ttatgcagag gccgaggccg cctctgcctc tgagctattc cagaagtagt 2700
   gaggaggctt ttttggaggc ctaggctttt gcaaaaagct cccgggagct tgtatatcca 2760
   ttttcggatc tgatcaagag acaggatgag gatcgtttcg catgattgaa caagatggat 2820
   tgcacgcagg ttctccggcc gcttgggtgg agaggctatt cggctatgac tgggcacaac 2880
   agacaatcgg ctgctctgat gccgccgtgt tccggctgtc agcgcagggg cgcccggttc 2940
   tttttgtcaa gaccgacctg tccggtgccc tgaatgaact gcaggacgag gcagcgcggc 3000
   tatcgtggct ggccacgacg ggcgttcctt gcgcagctgt gctcgacgtt gtcactgaag 3060
   cgggaaggga ctggctgcta ttgggcgaag tgccggggca ggatctcctg tcatctcacc 3120
   ttgctcctgc cgagaaagta tccatcatgg ctgatgcaat gcggcggctg catacgcttg 3180
   atccggctac ctgcccattc gaccaccaag cgaaacatcg catcgagcga gcacgtactc 3240
   ggatggaagc cggtcttgtc gatcaggatg atctggacga agagcatcag gggctcgcgc 3300
   cagccgaact gttcgccagg ctcaaggcgc gcatgcccga cggcgaggat ctcgtcgtga 3360
   cccatggcga tgcctgcttg ccgaatatca tggtggaaaa tggccgcttt tctggattca 3420
   tcgactgtgg ccggctgggt gtggcggacc gctatcagga catagcgttg gctacccgtg 3480
   atattgctga agagcttggc ggcgaatggg ctgaccgctt cctcgtgctt tacggtatcg 3540
   ccgctcccga ttcgcagcgc atcgccttct atcgccttct tgacgagttc ttctgagcgg 3600
   gactctgggg ttcgaaatga ccgaccaagc gacgcccaac ctgccatcac gagatttcga 3660
   ttccaccgcc gccttctatg aaaggttggg cttcggaatc gttttccggg acgccggctg 3720
   gatgatcctc cagcgcgggg atctcatgct ggagttcttc gcccacccca acttgtttat 3780
   tgcagcttat aatggttaca aataaagcaa tagcatcaca aatttcacaa ataaagcatt 3840
   tttttcactg cattctagtt gtggtttgtc caaactcatc aatgtatctt atcatgtctg 3900
   tataccgtcg acctctagct agagcttggc gtaatcatgg tcatagctgt ttcctgtgtg 3960
   aaattgttat ccgctcacaa ttccacacaa catacgagcc ggaagcataa agtgtaaagc 4020
   ctggggtgcc taatgagtga gctaactcac attaattgcg ttgcgctcac tgcccgcttt 4080
   ccagtcggga aacctgtcgt gccagctgca ttaatgaatc ggccaacgcg cggggagagg 4140
   cggtttgcgt attgggcgct cttccgcttc ctcgctcact gactcgctgc gctcggtcgt 4200
   tcggctgcgg cgagcggtat cagctcactc aaaggcggta atacggttat ccacagaatc 4260
   aggggataac gcaggaaaga acatgtgagc aaaaggccag caaaaggcca ggaaccgtaa 4320
   aaaggccgcg ttgctggcgt ttttccatag gctccgcccc cctgacgagc atcacaaaaa 4380
   tcgacgctca agtcagaggt ggcgaaaccc gacaggacta taaagatacc aggcgtttcc 4440
   ccctggaagc tccctcgtgc gctctcctgt tccgaccctg ccgcttaccg gatacctgtc 4500
   cgcctttctc ccttcgggaa gcgtggcgct ttctcaatgc tcacgctgta ggtatctcag 4560
   ttcggtgtag gtcgttcgct ccaagctggg ctgtgtgcac gaaccccccg ttcagcccga 4620
   ccgctgcgcc ttatccggta actatcgtct tgagtccaac ccggtaagac acgacttatc 4680
   gccactggca gcagccactg gtaacaggat tagcagagcg aggtatgtag gcggtgctac 4740
   agagttcttg aagtggtggc ctaactacgg ctacactaga aggacagtat ttggtatctg 4800
   cgctctgctg aagccagtta ccttcggaaa aagagttggt agctcttgat ccggcaaaca 4860
   aaccaccgct ggtagcggtg gtttttttgt ttgcaagcag cagattacgc gcagaaaaaa 4920
   aggatctcaa gaagatcctt tgatcttttc tacggggtct gacgctcagt ggaacgaaaa 4980
   ctcacgttaa gggattttgg tcatgagatt atcaaaaagg atcttcacct agatcctttt 5040
   aaattaaaaa tgaagtttta aatcaatcta aagtatatat gagtaaactt ggtctgacag 5100
   ttaccaatgc ttaatcagtg aggcacctat ctcagcgatc tgtctatttc gttcatccat 5160
   agttgcctga ctccccgtcg tgtagataac tacgatacgg gagggcttac catctggccc 5220
   cagtgctgca atgataccgc gagacccacg ctcaccggct ccagatttat cagcaataaa 5280
   ccagccagcc ggaagggccg agcgcagaag tggtcctgca actttatccg cctccatcca 5340
   gtctattaat tgttgccggg aagctagagt aagtagttcg ccagttaata gtttgcgcaa 5400
   cgttgttgcc attgctacag gcatcgtggt gtcacgctcg tcgtttggta tggcttcatt 5460
   cagctccggt tcccaacgat caaggcgagt tacatgatcc cccatgttgt gcaaaaaagc 5520
   ggttagctcc ttcggtcctc cgatcgttgt cagaagtaag ttggccgcag tgttatcact 5580
   catggttatg gcagcactgc ataattctct tactgtcatg ccatccgtaa gatgcttttc 5640
   tgtgactggt gagtactcaa ccaagtcatt ctgagaatag tgtatgcggc gaccgagttg 5700
   ctcttgcccg gcgtcaatac gggataatac cgcgccacat agcagaactt taaaagtgct 5760
   catcattgga aaacgttctt cggggcgaaa actctcaagg atcttaccgc tgttgagatc 5820
   cagttcgatg taacccactc gtgcacccaa ctgatcttca gcatctttta ctttcaccag 5880
   cgtttctggg tgagcaaaaa caggaaggca aaatgccgca aaaaagggaa taagggcgac 5940
   acggaaatgt tgaatactca tactcttcct ttttcaatat tattgaagca tttatcaggg 6000
   ttattgtctc atgagcggat acatatttga atgtatttag aaaaataaac aaataggggt 6060
   tccgcgcaca tttccccgaa aagtgccacc tgacgtc 6097
<210> 13
   <211> 6094
   <212> DNA
   <213> Artificial Sequence
<220>
   <223> Plasmid
<400> 13
   gacggatcgg gagatctccc gatcccctat ggtcgactct cagtacaatc tgctctgatg      60
   ccgcatagtt aagccagtat ctgctccctg cttgtgtgtt ggaggtcgct gagtagtgcg     120
   cgagcaaaat ttaagctaca acaaggcaag gcttgaccga caattgcatg aagaatctgc     180
   ttagggttag gcgttttgcg ctgcttcgcg atgtacgggc cagatatacg cgttgacatt     240
   gattattgac tagttattaa tagtaatcaa ttacggggtc attagttcat agcccatata     300
   tggagttccg cgttacataa cttacggtaa atggcccgcc tggctgaccg cccaacgacc     360
   cccgcccatt gacgtcaata atgacgtatg ttcccatagt aacgccaata gggactttcc     420
   attgacgtca atgggtggac tatttacggt aaactgccca cttggcagta catcaagtgt     480
   atcatatgcc aagtacgccc cctattgacg tcaatgacgg taaatggccc gcctggcatt     540
   atgcccagta catgacctta tgggactttc ctacttggca gtacatctac gtattagtca     600
   tcgctattac catggtgatg cggttttggc agtacatcaa tgggcgtgga tagcggtttg     660
   actcacgggg atttccaagt ctccacccca ttgacgtcaa tgggagtttg ttttggcacc     720
   aaaatcaacg ggactttcca aaatgtcgta acaactccgc cccattgacg caaatgggcg     780
   gtaggcgtgt acggtgggag gtctatataa gcagagctct ctggctaact agagaaccca     840
   ctgcttactg gcttatcgaa attaatacga ctcactatag ggagacccaa gctggctaga     900
   aagcttggat ctcaccatgg tgttgcagac ccaggtcttc atttctctgt tactctggat     960
   ctctggtgcc tacggggaca tcgtgatgac ccagtctcca gactccctgg ctgtgtctct    1020
   gggcgagagg gccaccatca actgcaagtc caaccagagt gtcttacaca gctccaacaa    1080
   taagaactat ttagcttggt accagcagaa accaggacag cctcctaaat tgctcattta    1140
   ttgggcattc ctccgggaat ccggggtccc tgaccgcttc agtggcagcg ggtctgggac    1200
   agatttcact ctcaccatca gcagcctgca ggctgaagat gtggcagttt attactgtca    1260
   ccaatattat tctactttat atactttcgg cggagggacc aaggtagaga tcaaacgaac    1320
   ygtggctgca ccatctgtct tcatcttccc gccatctgat gagcagttga aatctggaac    1380
   tgctagcgtt gtgtgcctgc tgaataactt ctatcccaga gaggccaaag tacagtggaa 1440
   ggtggataac gccctccaat cgggtaactc ccaggagagt gtcacagagc aggacagcaa 1500
   ggacagcacc tacagcctca gcagcaccct gacgctgagc aaagcagact acgagaaaca 1560
   caaagtctac gcctgcgaag tcacccatca gggcctgagc tcgcccgtca caaagagctt 1620
   caacagggga gagtgttagg cggccgctcg agtctagagg gcccgtttaa acccgctgat 1680
   cagcctcgac tgtgccttct agttgccagc catctgttgt ttgcccctcc cccgtgcctt 1740
   ccttgaccct ggaaggtgcc actcccactg tcctttccta ataaaatgag gaaattgcat 1800
   cgcattgtct gagtaggtgt cattctattc tggggggtgg ggtggggcag gacagcaagg 1860
   gggaggattg ggaagacaat agcaggcatg ctggggatgc ggtgggctct atggcttctg 1920
   aggcggaaag aaccagctgg ggctctaggg ggtatcccca cgcgccctgt agcggcgcat 1980
   taagcgcggc gggtgtggtg gttacgcgca gcgtgaccgc tacacttgcc agcgccctag 2040
   cgcccgctcc tttcgctttc ttcccttcct ttctcgccac gttcgccggc tttccccgtc 2100
   aagctctaaa tcggggcatc cctttagggt tccgatttag tgctttacgg cacctcgacc 2160
   ccaaaaaact tgattagggt gatggttcac gtagtgggcc atcgccctga tagacggttt 2220
   ttcgcccttt gacgttggag tccacgttct ttaatagtgg actcttgttc caaactggaa 2280
   caacactcaa ccctatctcg gtctattctt ttgatttata agggattttg gggatttcgg 2340
   cctattggtt aaaaaatgag ctgatttaac aaaaatttaa cgcgaattaa ttctgtggaa 2400
   tgtgtgtcag ttagggtgtg gaaagtcccc aggctcccca ggcaggcaga agtatgcaaa 2460
   gcatgcatct caattagtca gcaaccaggt gtggaaagtc cccaggctcc ccagcaggca 2520
   gaagtatgca aagcatgcat ctcaattagt cagcaaccat agtcccgccc ctaactccgc 2580
   ccatcccgcc cctaactccg cccagttccg cccattctcc gccccatggc tgactaattt 2640
   ttttatttta tgcagaggcc gaggccgcct ctgcctctga gctattccag aagtagtgag 2700
   gaggcttttt tggaggccta ggcttttgca aaaagctccc gggagcttgt atatccattt 2760
   tcggatctga tcaagagaca ggatgaggat cgtttcgcat gattgaacaa gatggattgc 2820
   acgcaggttc tccggccgct tgggtggaga ggctattcgg ctatgactgg gcacaacaga 2880
   caatcggctg ctctgatgcc gccgtgttcc ggctgtcagc gcaggggcgc ccggttcttt 2940
   ttgtcaagac cgacctgtcc ggtgccctga atgaactgca ggacgaggca gcgcggctat 3000
   cgtggctggc cacgacgggc gttccttgcg cagctgtgct cgacgttgtc actgaagcgg 3060
   gaagggactg gctgctattg ggcgaagtgc cggggcagga tctcctgtca tctcaccttg 3120
   ctcctgccga gaaagtatcc atcatggctg atgcaatgcg gcggctgcat acgcttgatc 3180
   cggctacctg cccattcgac caccaagcga aacatcgcat cgagcgagca cgtactcgga 3240
   tggaagccgg tcttgtcgat caggatgatc tggacgaaga gcatcagggg ctcgcgccag 3300
   ccgaactgtt cgccaggctc aaggcgcgca tgcccgacgg cgaggatctc gtcgtgaccc 3360
   atggcgatgc ctgcttgccg aatatcatgg tggaaaatgg ccgcttttct ggattcatcg 3420
   actgtggccg gctgggtgtg gcggaccgct atcaggacat agcgttggct acccgtgata 3480
   ttgctgaaga gcttggcggc gaatgggctg accgcttcct cgtgctttac ggtatcgccg 3540
   ctcccgattc gcagcgcatc gccttctatc gccttcttga cgagttcttc tgagcgggac 3600
   tctggggttc gaaatgaccg accaagcgac gcccaacctg ccatcacgag atttcgattc 3660
   caccgccgcc ttctatgaaa ggttgggctt cggaatcgtt ttccgggacg ccggctggat 3720
   gatcctccag cgcggggatc tcatgctgga gttcttcgcc caccccaact tgtttattgc 3780
   agcttataat ggttacaaat aaagcaatag catcacaaat ttcacaaata aagcattttt 3840
   ttcactgcat tctagttgtg gtttgtccaa actcatcaat gtatcttatc atgtctgtat 3900
   accgtcgacc tctagctaga gcttggcgta atcatggtca tagctgtttc ctgtgtgaaa 3960
   ttgttatccg ctcacaattc cacacaacat acgagccgga agcataaagt gtaaagcctg 4020
   gggtgcctaa tgagtgagct aactcacatt aattgcgttg cgctcactgc ccgctttcca 4080
   gtcgggaaac ctgtcgtgcc agctgcatta atgaatcggc caacgcgcgg ggagaggcgg 4140
   tttgcgtatt gggcgctctt ccgcttcctc gctcactgac tcgctgcgct cggtcgttcg 4200
   gctgcggcga gcggtatcag ctcactcaaa ggcggtaata cggttatcca cagaatcagg 4260
   ggataacgca ggaaagaaca tgtgagcaaa aggccagcaa aaggccagga accgtaaaaa 4320
   ggccgcgttg ctggcgtttt tccataggct ccgcccccct gacgagcatc acaaaaatcg 4380
   acgctcaagt cagaggtggc gaaacccgac aggactataa agataccagg cgtttccccc 4440
   tggaagctcc ctcgtgcgct ctcctgttcc gaccctgccg cttaccggat acctgtccgc 4500
   ctttctccct tcgggaagcg tggcgctttc tcaatgctca cgctgtaggt atctcagttc 4560
   ggtgtaggtc gttcgctcca agctgggctg tgtgcacgaa ccccccgttc agcccgaccg 4620
   ctgcgcctta tccggtaact atcgtcttga gtccaacccg gtaagacacg acttatcgcc 4680
   actggcagca gccactggta acaggattag cagagcgagg tatgtaggcg gtgctacaga 4740
   gttcttgaag tggtggccta actacggcta cactagaagg acagtatttg gtatctgcgc 4800
   tctgctgaag ccagttacct tcggaaaaag agttggtagc tcttgatccg gcaaacaaac 4860
   caccgctggt agcggtggtt tttttgtttg caagcagcag attacgcgca gaaaaaaagg 4920
   atctcaagaa gatcctttga tcttttctac ggggtctgac gctcagtgga acgaaaactc 4980
   acgttaaggg attttggtca tgagattatc aaaaaggatc ttcacctaga tccttttaaa 5040
   ttaaaaatga agttttaaat caatctaaag tatatatgag taaacttggt ctgacagtta 5100
   ccaatgctta atcagtgagg cacctatctc agcgatctgt ctatttcgtt catccatagt 5160
   tgcctgactc cccgtcgtgt agataactac gatacgggag ggcttaccat ctggccccag 5220
   tgctgcaatg ataccgcgag acccacgctc accggctcca gatttatcag caataaacca 5280
   gccagccgga agggccgagc gcagaagtgg tcctgcaact ttatccgcct ccatccagtc 5340
   tattaattgt tgccgggaag ctagagtaag tagttcgcca gttaatagtt tgcgcaacgt 5400
   tgttgccatt gctacaggca tcgtggtgtc acgctcgtcg tttggtatgg cttcattcag 5460
   ctccggttcc caacgatcaa ggcgagttac atgatccccc atgttgtgca aaaaagcggt 5520
   tagctccttc ggtcctccga tcgttgtcag aagtaagttg gccgcagtgt tatcactcat 5580
   ggttatggca gcactgcata attctcttac tgtcatgcca tccgtaagat gcttttctgt 5640
   gactggtgag tactcaacca agtcattctg agaatagtgt atgcggcgac cgagttgctc 5700
   ttgcccggcg tcaatacggg ataataccgc gccacatagc agaactttaa aagtgctcat 5760
   cattggaaaa cgttcttcgg ggcgaaaact ctcaaggatc ttaccgctgt tgagatccag 5820
   ttcgatgtaa cccactcgtg cacccaactg atcttcagca tcttttactt tcaccagcgt 5880
   ttctgggtga gcaaaaacag gaaggcaaaa tgccgcaaaa aagggaataa gggcgacacg 5940
   gaaatgttga atactcatac tcttcctttt tcaatattat tgaagcattt atcagggtta 6000
   ttgtctcatg agcggataca tatttgaatg tatttagaaa aataaacaaa taggggttcc 6060
   gcgcacattt ccccgaaaag tgccacctga cgtc 6094
<210> 14
   <211> 481
   <212> DNA
   <213> Artificial Sequence
<220>
   <223> Includes BamHI/BglII cloning junction, signal peptide, V region, portion of C region and 3'XbaI/NheI (heavy) or NheI (light) cloning junction
<400> 14
   ggatctcacc atggagttgg gactgcgctg gggcttcctc gttgctcttt taagaggtgt 60
   ccagtgtcag gtgcaattgg tggagtctgg gggaggcgtg gtccagcctg ggaggtccct 120
   gagactctcc tgtgcagcgt ctggattcgc cttcagtaga tatggcatgc actgggtccg 180
   ccaggctcca ggcaaggggc tggagtgggt ggcagttata tggtatgatg gaagtaataa 240
   atactatgca gactccgtga agggccgatt caccatctcc agagacaatt ccaagaacac 300
   gcagtatctg caaatgaaca gcctgagagc cgaggacacg gctgtgtatt actgtgcgag    360
   aggcggtgac ttcctctact actactatta cggtatggac gtctggggcc aagggaccac    420
   ggtcaccgtc tcctcagcct ccaccaaggg cccatcggtc ttccccctgg caccctctag    480
   c                                                                    481
<210> 15
   <211> 142
   <212> PRT
   <213> Homo sapiens
<400> 15
   Met Glu Leu Gly Leu Arg Trp Gly Phe Leu Val Ala Leu Leu Arg Gly
   1               5                   10                  15
   Val Gln Cys Gln Val Gln Leu Val Glu Ser Gly Gly Gly Val Val Gln
               20                  25                  30
   Pro Gly Arg Ser Leu Arg Leu Ser Cys Ala Ala Ser Gly Phe Ala Phe
           35                  40                  45
   Ser Arg Tyr Gly Met His Trp Val Arg Gln Ala Pro Gly Lys Gly Leu
       50                  55                  60
   Glu Trp Val Ala Val Ile Trp Tyr Asp Gly Ser Asn Lys Tyr Tyr Ala
   65                  70                  75                  80
   Asp Ser Val Lys Gly Arg Phe Thr Ile Ser Arg Asp Asn Ser Lys Asn
                   85                  90                  95
   Thr Gln Tyr Leu Gln Met Asn Ser Leu Arg Ala Glu Asp Thr Ala Val
               100                 105                 110
   Tyr Tyr Cys Ala Arg Gly Gly Asp Phe Leu Tyr Tyr Tyr Tyr Tyr Gly
           115                 120                 125
   Met Asp Val Trp Gly Gln Gly Thr Thr Val Thr Val Ser Ser
       130                 135                 140
<210> 16
   <211> 463
   <212> DNA
   <213> Artificial Sequence
<220>
   <223> Includes BamHI/BglII cloning junction, signal peptide, V region, portion of C region and 3'XbaI/NheI (heavy) or NheI (light) cloning junction
<400> 16
   ggatctcacc atgagggtcc ctgctcagct cctgggactc ctgctgctct ggctcccaga    60
   taccagatgt gacatccaga tgacccagtc tccatcctcc ctgtctgcat ctgtaggaga   120
   cagagtcacc atcacttgcc gggcgagtca gggcattagc aattatttag cctggtatca   180
   gcagaaaaca gggaaagttc ctaagttcct gatctatgaa gcatccactt tgcaatcagg   240
   ggtcccatct cggttcagtg gcggtggatc tgggacagat ttcactctca ccatcagcag   300
   cctgcagcct gaagatgttg caacttatta ctgtcaaaat tataacagtg ccccattcac   360
   tttcggccct gggaccaaag tggatatcaa acgaactgtg gctgcaccct ctgtcttcat   420
   cttcccgcca tctgatgagc agttgaaatc tggaactgct agc                     463
<210> 17
   <211> 127
   <212> PRT
   <213> Homo sapiens
<400> 17
   Met Arg Val Pro Ala Gln Leu Leu Gly Leu Leu Leu Leu Trp Leu Pro
   1               5                   10                  15
   Asp Thr Arg Cys Asp Ile Gln Met Thr Gln Ser Pro Ser Ser Leu Ser
               20                  25                  30
   Ala Ser Val Gly Asp Arg Val Thr Ile Thr Cys Arg Ala Ser Gln Gly
           35                  40                  45
   Ile Ser Asn Tyr Leu Ala Trp Tyr Gln Gln Lys Thr Gly Lys Val Pro
       50                  55                  60
   Lys Phe Leu Ile Tyr Glu Ala Ser Thr Leu Gln Ser Gly Val Pro Ser
   65                  70                  75                  80
   Arg Phe Ser Gly Gly Gly Ser Gly Thr Asp Phe Thr Leu Thr Ile Ser
                   85                  90                  95
   Ser Leu Gln Pro Glu Asp Val Ala Thr Tyr Tyr Cys Gln Asn Tyr Asn
               100                 105                 110
   Ser Ala Pro Phe Thr Phe Gly Pro Gly Thr Lys Val Asp Ile Lys
           115                 120                 125
<210> 18
   <211> 508
   <212> DNA
   <213> Artificial Sequence
<220>
   <223> Includes BamHI/BglII cloning junction, signal peptide, V region, portion of C region and 3'XbaI/NheI (heavy) or NheI (light) cloning junction
<400> 18
   ggatctcacc atggggtcaa ccgccatcct caccatggag ttggggctgc gctgggttct   60
   cctcgttgct cttttaagag gtgtccagtg tcaggtgcag ctggtggagt ctgggggagg  120
   cgtggtccag cctgggaggt ccctgagact ctcctgtgca gcgtctggat tcaccttcag  180
   taactatgtc atgcactggg tccgccaggc tccaggcaag gggctggagt gggtggcaat  240
   tatatggtat gatggaagta ataaatacta tgcagactcc gtgaagggcc gattcaccat  300
   ctccagagac aattccaaga acacgctgta tctgcaaatg aacagcctga gagccgagga  360
   cacggctgtg tattactgtg cgggtggata taactggaac tacgagtacc actactacgg  420
   tatggacgtc tggggccaag ggaccacggt caccgtctcc tcagcctcca ccaagggccc  480
   atcggtcttc cccctggcac cctctagc                                     508
<210> 19
   <211> 143
   <212> PRT
   <213> Homo sapiens
<400> 19
   Met Glu Leu Gly Leu Arg Trp Val Leu Leu Val Ala Leu Leu Arg Gly
   1               5                   10                  15
   Val Gln Cys Gln Val Gln Leu Val Glu Ser Gly Gly Gly Val Val Gln
               20                  25                  30
   Pro Gly Arg Ser Leu Arg Leu Ser Cys Ala Ala Ser Gly Phe Thr Phe
           35                  40                  45
   Ser Asn Tyr Val Met His Trp Val Arg Gln Ala Pro Gly Lys Gly Leu
       50                  55                  60
   Glu Trp Val Ala Ile Ile Trp Tyr Asp Gly Ser Asn Lys Tyr Tyr Ala
   65                  70                  75                  80
   Asp Ser Val Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Gly Gly Tyr Asn Trp Asn Tyr Glu Tyr His Tyr Tyr
           115                 120                 125
   Gly Met Asp Val Trp Gly Gln Gly Thr Thr Val Thr Val Ser Ser
       130                 135                 140
<210> 20
   <211> 463
   <212> DNA
   <213> Artificial Sequence
<220>
   <223> Includes BamHI/BglII cloning junction, signal peptide, V region, portion of C region and 3'XbaI/NheI (heavy) or NheI (light) cloning junction
<400> 20
   ggatctcacc atgagggtcc ccgctcagct cctggggctc ctgctgctct gtttcccagg     60
   tgccagatgt gacatccaga tgacccagtc tccatcctca ctgtctgcat ctgtaggaga    120
   cagagtcacc atcacttgtc gggcgagtca gggcattacc aattatttag cctggtttca    180
   gcagaaacca gggaaagccc ctaagtccct tatctatgct gcatccagtt tgcaaagtgg    240
   ggtcccatca aagttcagcg gcagtggatc tgggacagat ttcagtctca ccatcagcag    300
   cctgcagcct gaagattttg caacttatta ctgccaacag tataatagtt acccgatcac    360
   cttcggccaa gggacacgac tggagattaa acgaactgtg gctgcaccat ctgtcttcat    420
   cttcccgcca tctgatgagc agttgaaatc tggaactgct agc                      463
<210> 21
   <211> 127
   <212> PRT
   <213> Homo sapiens
<400> 21
   Met Arg Val Pro Ala Gln Leu Leu Gly Leu Leu Leu Leu Cys Phe Pro
   1               5                   10                  15
   Gly Ala Arg Cys Asp Ile Gln Met Thr Gln Ser Pro Ser Ser Leu Ser
               20                  25                  30
   Ala Ser Val Gly Asp Arg Val Thr Ile Thr Cys Arg Ala Ser Gln Gly
           35                  40                  45
   Ile Thr Asn Tyr Leu Ala Trp Phe Gln Gln Lys Pro Gly Lys Ala Pro
       50                  55                  60
   Lys Ser Leu Ile Tyr Ala Ala Ser Ser Leu Gln Ser Gly Val Pro Ser
   65                  70                  75                  80
   Lys Phe Ser Gly Ser Gly Ser Gly Thr Asp Phe Ser Leu Thr Ile Ser
                   85                  90                  95
   Ser Leu Gln Pro Glu Asp Phe Ala Thr Tyr Tyr Cys Gln Gln Tyr Asn
               100                 105                 110
   Ser Tyr Pro Ile Thr Phe Gly Gln Gly Thr Arg Leu Glu Ile Lys
           115                 120                 125
<210> 22
   <211> 490
   <212> DNA
   <213> Artificial Sequence
<220>
   <223> Includes BamHI/BglII cloning junction, signal peptide, V region, portion of C region and 3'XbaI/NheI (heavy) or NheI (light) cloning junction
<400> 22
   ggatctcacc atggagttgg gacttagctg ggttttcctc gttgctcttt taagaggtgt     60
   ccagtgtcag gtccagctgg tggagtctgg gggaggcgtg gtccagcctg ggaggtccct    120
   gagactctcc tgtgcagcgt ctggattcac cttcagtagc tatggcatgc actgggtccg    180
   ccaggctcca ggcaaggggc tggactgggt ggcaattatt tggcatgatg gaagtaataa    240
   atactatgca gactccgtga agggccgatt caccatctcc agagacaatt ccaagaagac    300
   gctgtacctg caaatgaaca gtttgagagc cgaggacacg gctgtgtatt actgtgcgag    360
   agcttgggcc tatgactacg gtgactatga atactacttc ggtatggacg tctggggcca    420
   agggaccacg gtcaccgtct cctcagcctc caccaagggc ccatcggtct tccccctggc    480
   accctctagc                                                           490
<210> 23
   <211> 145
   <212> PRT
   <213> Homo sapiens
<400> 23
   Met Glu Leu Gly Leu Ser Trp Val Phe Leu Val Ala Leu Leu Arg Gly
   1               5                   10                  15
   Val Gln Cys Gln Val Gln Leu Val Glu Ser Gly Gly Gly Val Val Gln
               20                  25                  30
   Pro Gly Arg Ser Leu Arg Leu Ser Cys Ala Ala Ser Gly Phe Thr Phe
           35                  40                  45
   Ser Ser Tyr Gly Met His Trp Val Arg Gln Ala Pro Gly Lys Gly Leu
       50                  55                  60
   Asp Trp Val Ala Ile Ile Trp His Asp Gly Ser Asn Lys Tyr Tyr Ala
   65 70 75 80
   Asp Ser Val Lys Gly Arg Phe Thr Ile Ser Arg Asp Asn Ser Lys Lys
   85 90 95
   Thr Leu Tyr Leu Gln Met Asn Ser Leu Arg Ala Glu Asp Thr Ala Val
   100 105 110
   Tyr Tyr Cys Ala Arg Ala Trp Ala Tyr Asp Tyr Gly Asp Tyr Glu Tyr
   115 120 125
   Tyr Phe Gly Met Asp Val Trp Gly Gln Gly Thr Thr Val Thr Val Ser
   130 135 140
   Ser
   145
<210> 24
   <211> 463
   <212> DNA
   <213> Artificial Sequence
<220>
   <223> Includes BamHI/BglII cloning junction, signal peptide, V region, portion of C region and 3'XbaI/NheI (heavy) or NheI (light) cloning junction
<400> 24
   ggatctcacc atgagggtcc ctgctcagct cctggggctc ctgctgctct gtttcccagg    60
   tgccagatgt gacatccaga tgacccagtc tccatcctca ctgtctgcat ctgtaggaga   120
   cagagtcacc atcacttgtc gggcgagtca gggcattagc cattatttag cctggtttca   180
   gcagaaacca gggaaagccc ctaagtccct gatctatgct gcatccagtt tgcaaagtgg   240
   ggtcccatca aagttcagcg gcagtggatc tgggacagat ttcactctca ccatcagcag   300
   cctacagcct gaagattttg caacttatta ctgccaacag tataatagtt tcccgctcac   360
   tttcggcgga gggaccaagg tggagatcaa acgaactgtg gctgcaccat ctgtcttcat   420
   cttcccgcca tctgatgagc agttgaaatc tggaactgct agc                     463
<210> 25
   <211> 127
   <212> PRT
   <213> Homo sapiens
<400> 25
   Met Arg Val Pro Ala Gln Leu Leu Gly Leu Leu Leu Leu Cys Phe Pro
   1               5                   10                  15
   Gly Ala Arg Cys Asp Ile Gln Met Thr Gln Ser Pro Ser Ser Leu Ser
               20                  25                  30
   Ala Ser Val Gly Asp Arg Val Thr Ile Thr Cys Arg Ala Ser Gln Gly
           35                  40                  45
   Ile Ser His Tyr Leu Ala Trp Phe Gln Gln Lys Pro Gly Lys Ala Pro
       50                  55                  60
   Lys Ser Leu Ile Tyr Ala Ala Ser Ser Leu Gln Ser Gly Val Pro Ser
   65                  70                  75                  80
   Lys Phe Ser Gly Ser Gly Ser Gly Thr Asp Phe Thr Leu Thr Ile Ser
                   85                  90                  95
   Ser Leu Gln Pro Glu Asp Phe Ala Thr Tyr Tyr Cys Gln Gln Tyr Asn
               100                 105                 110
   Ser Phe Pro Leu Thr Phe Gly Gly Gly Thr Lys Val Glu Ile Lys
           115                 120                 125
<210> 26
   <211> 469
   <212> DNA
   <213> Artificial Sequence
<220>
   <223> Includes BamHI/BglII cloning junction, signal peptide, V region, portion of C region and 3'XbaI/NheI (heavy) or NheI (light) cloning junction
<400> 26
   ggatcccacc atggggtcaa ccgtcatcct cgccctcctc ctggctgttc tccaaggagt     60
   ctgtgccgag gtgcagctgg tgcagtctgg agcagaggtg aaaaagcccg gggagtctct    120
   gaagatctcc tgtaagggtt ctggatacag ctttaccagt tactggatcg gctgggtgcg    180
   ccagatgccc gggaaaggcc tggagtggat ggggatcatc tatcctggtg actctgatac    240
   cagatacagc ccgtccttcc aaggccaggt caccatctca gccgacaagt ccatcagcac    300
   cgcctacctg cagtggagca gcctgaaggc ctcggacacc gccatgtatt actgtgcgag    360
   acggatggca gcagctggcc cctttgacta ctggggccag ggaaccctgg tcaccgtctc    420
   ctcagcctcc accaagggcc catcggtctt ccccctggca ccctctagc                  469
<210> 27
   <211> 138
   <212> PRT
   <213> Homo sapiens
<400> 27
   Met Gly Ser Thr Val Ile Leu Ala Leu Leu Leu Ala Val Leu Gln Gly
   1 5 10 15
   Val Cys Ala Glu Val Gln Leu Val Gln Ser Gly Ala Glu Val Lys Lys
   20 25 30
   Pro Gly Glu Ser Leu Lys Ile Ser Cys Lys Gly Ser Gly Tyr Ser Phe
   35 40 45
   Thr Ser Tyr Trp Ile Gly Trp Val Arg Gln Met Pro Gly Lys Gly Leu
   50 55 60
   Glu Trp Met Gly Ile Ile Tyr Pro Gly Asp Ser Asp Thr Arg Tyr Ser
   65 70 75 80
   Pro Ser Phe Gln Gly Gln Val Thr Ile Ser Ala Asp Lys Ser Ile Ser
   85 90 95
   Thr Ala Tyr Leu Gln Trp Ser Ser Leu Lys Ala Ser Asp Thr Ala Met
   100 105 110
   Tyr Tyr Cys Ala Arg Arg Met Ala Ala Ala Gly Pro Phe Asp Tyr Trp
   115 120 125
   Gly Gln Gly Thr Leu Val Thr Val Ser Ser
   130 135
<210> 28
   <211> 466
   <212> DNA
   <213> Artificial Sequence
<220>
   <223> Includes BamHI/BglII cloning junction, signal peptide, V region, portion of C region and 3'XbaI/NheI (heavy) or NheI (light) cloning junction
<400> 28
   ggatctcacc atgagggtcc ccgctcagct tctcttcctt ctgctactct ggctcccaga 60
   taccactgga ggaatagtga tgacgcagtc tccagccacc ctgtctgtgt ctccagggga 120
   aagagccacc ctctcctgca ggaccagtca gagtattggc tggaacttag cctggtacca 180
   acagaaacct ggccaggctc ccaggctcct catctatggt gcatcttcca ggaccactgg 240
   tatcccagcc aggttcagtg gcagtgggtc tgggacagag ttcactctca ccatcagcag 300
   cctgcagtct gaagattctg cagtttatta ctgtcagcat tatgataact ggcccatgtg 360
   cagttttggc caggggaccg agctggagat caaacgaact gtggctgcac catctgtctt 420
   catcttcccg ccatctgatg agcagttgaa atctggaact gctagc 466
<210> 29
   <211> 128
   <212> PRT
   <213> Homo sapiens
<400> 29
   Met Arg Val Pro Ala Gln Leu Leu Phe Leu Leu Leu Leu Trp Leu Pro
   1 5 10 15
   Asp Thr Thr Gly Gly Ile Val Met Thr Gln Ser Pro Ala Thr Leu Ser
   20 25 30
   Val Ser Pro Gly Glu Arg Ala Thr Leu Ser Cys Arg Thr Ser Gln Ser
   35 40 45
   Ile Gly Trp Asn Leu Ala Trp Tyr Gln Gln Lys Pro Gly Gln Ala Pro
   50 55 60
   Arg Leu Leu Ile Tyr Gly Ala Ser Ser Arg Thr Thr Gly Ile Pro Ala
   65 70 75 80
   Arg Phe Ser Gly Ser Gly Ser Gly Thr Glu Phe Thr Leu Thr Ile Ser
   85 90 95
   Ser Leu Gln Ser Glu Asp Ser Ala Val Tyr Tyr Cys Gln His Tyr Asp
   100 105 110
   Asn Trp Pro Met Cys Ser Phe Gly Gln Gly Thr Glu Leu Glu Ile Lys
   115 120 125
<210> 30
   <211> 487
   <212> DNA
   <213> Artificial Sequence
<220>
   <223> Includes BamHI/BgIII cloning junction, signal peptide, V region, portion of C region and 3'XbaI/NheI (heavy) or NheI (light) cloning junction
<400> 30
   ggatctcacc atggagtttg ggctgtgctg gattttcctc gttgctcttt taagaggtgt 60
   ccagtgtcag gtgcagctgg tggagtctgg gggaggcgtg gtccagcctg ggaggtccct 120
   gagactctcc tgtgcagcct ctggattcac cttcattagc tatggcatgc actgggtccg 180
   ccaggctcca ggcaaggggc tggagtgggt ggcagttata tcatatgatg gaagtaataa 240
   atactatgca gactccgtga agggccgatt caccatctcc agagacaatt ccaagaacac 300
   gctgtatctg caaatgaaca gcctgagagc tgaggacacg gctgtgtatt actgtgcgag 360
   agtattagtg ggagctttat attattataa ctactacggg atggacgtct ggggccaagg 420
   gaccacggtc accgtctcct cagcctccac caagggccca tcggtcttcc ccctggcacc 480
   ctctagc 487
<210> 31
   <211> 144
   <212> PRT
   <213> Homo sapiens
<400> 31
   Met Glu Phe Gly Leu Cys Trp Ile Phe Leu Val Ala Leu Leu Arg Gly
   1               5                   10                  15
   Val Gln Cys Gln Val Gln Leu Val Glu Ser Gly Gly Gly Val Val Gln
               20                  25                  30
   Pro Gly Arg Ser Leu Arg Leu Ser Cys Ala Ala Ser Gly Phe Thr Phe
           35                  40                  45
   Ile Ser Tyr Gly Met His Trp Val Arg Gln Ala Pro Gly Lys Gly Leu
       50                  55                  60
   Glu Trp Val Ala Val Ile Ser Tyr Asp Gly Ser Asn Lys Tyr Tyr Ala
   65                  70                  75                  80
   Asp Ser Val Lys Gly Arg Phe Thr Ile Ser Arg Asp Asn Ser Lys Asn
                   85                  90                  95
   Thr Leu Tyr Leu Gln Met Asn Ser Leu Arg Ala Glu Asp Thr Ala Val
               100                 105                 110
   Tyr Tyr Cys Ala Arg Val Leu Val Gly Ala Leu Tyr Tyr Tyr Asn Tyr
           115                 120                 125
   Tyr Gly Met Asp Val Trp Gly Gln Gly Thr Thr Val Thr Val Ser Ser
       130                 135                 140
<210> 32
   <211> 478
   <212> DNA
   <213> Artificial Sequence
<220>
   <223> Includes BamHI/BglII cloning junction, signal peptide, V region, portion of C region and 3'XbaI/NheI (heavy) or NheI (light) cloning junction
<400> 32
   ggatctcacc atgagggtcc ctgctcagct cctggggctg ctaatgctct ggatacctgg     60
   atccagtgca gatattgtga tgacccagac tccactctct ctgtccgtca cccctggaca    120
   gccggcctcc atctcctgca agtctagtca gagcctcctg catagtgatg gaaagacctt    180
   tttgtattgg tatctgcaga agccaggcca gcctccacag ctcctgatct atgaggtttc    240
   caaccggttc tctggagtgc cagataggtt cagtggcagc gggtcaggga cagatttcac    300
   actgaaaatc agccgggtgg aggctgagga tgttgggctt tattactgca tgcaaagtat    360
   acagcttccg ctcactttcg gcggagggac caaggtggag atcaaacgaa ctgtggctgc    420
   accatctgtc ttcatcttcc cgccatctga tgagcagttg aaatctggaa ctgctagc      478
<210> 33
   <211> 132
   <212> PRT
   <213> Homo sapiens
<400> 33
   Met Arg Val Pro Ala Gln Leu Leu Gly Leu Leu Met Leu Trp Ile Pro
   1               5                   10                  15
   Gly Ser Ser Ala Asp Ile Val Met Thr Gln Thr Pro Leu Ser Leu Ser
               20                  25                  30
   Val Thr Pro Gly Gln Pro Ala Ser Ile Ser Cys Lys Ser Ser Gln Ser
           35                  40                  45
   Leu Leu His Ser Asp Gly Lys Thr Phe Leu Tyr Trp Tyr Leu Gln Lys
       50                  55                  60
   Pro Gly Gln Pro Pro Gln Leu Leu Ile Tyr Glu Val Ser Asn Arg Phe
   65                  70                  75                  80
   Ser Gly Val Pro Asp Arg Phe Ser Gly Ser Gly Ser Gly Thr Asp Phe
                   85                  90                  95
   Thr Leu Lys Ile Ser Arg Val Glu Ala Glu Asp Val Gly Leu Tyr Tyr
               100                 105                 110
   Cys Met Gln Ser Ile Gln Leu Pro Leu Thr Phe Gly Gly Gly Thr Lys
           115                 120                 125
   Val Glu Ile Lys
       130

## Description

### Field of the Invention

This invention relates generally to antibody-drug conjugates (ADCs). In particular, the invention relates to ADCs which comprise an antibody or antigen-binding fragment thereof which binds to prostate-specific membrane antigen (PSMA) and is conjugated to monomethylauristatin norephedrine (MMAE) or monomethylauristatin phenylalanine (MMAF). The antibody-drug conjugate has a PC-3™ cell to C4-2 or LNCaP™ cell selectivity of at least 250. The invention also relates, in part, to compositions of the ADCs. The methods provided include, for example, methods for treating a PSMA-mediated disease.

### Background of the Invention

Prostate cancer is the most common malignancy and the second leading cause of cancer death in men in the United States (Jemal A, et al., CA Cancer J Clin 2005;55:10-30). Localized prostate cancer typically is treated with surgery or radiation, and recurrent disease can be controlled temporarily with androgen ablation (Klein EA, et al., Urol Clin North Am 2003;30:315-30). However, almost all prostate carcinomas eventually become hormone refractory and then rapidly progress (Denmeade SR, et al., Nat Rev Cancer 2002;2:389-96). Hormone-refractory or androgen-independent prostate cancer has proven to be largely resistant to conventional chemotherapy. With the exception of palliative care, the only approved chemotherapy is docetaxel in combination with prednisone, which offers a modest (2.4 month) survival benefit (Gulley J, et al., Am J Ther. 2004;351:1513-20; Petrylak DP, et al., New Engl J Med 2004;351:1513-20). New molecularly targeted therapies are needed.

### Summary of the Invention

The invention provided herein relates to ADCs that exhibit particularly high selectivity. In one aspect of the invention an antibody-drug conjugate is provided that comprises an antibody or antigen-binding fragment thereof which binds to PSMA and is conjugated to monomethylauristatin norephedrine or monomethylauristatin phenylalanine, wherein the antibody-drug conjugate has a PC-3™ cell to C4-2 or LNCaP™ cell selectivity of at least 250, and 3 or 4 molecules of monomethylauristatin norephedrine or monomethylauristatin phenylalanine are conjugatedton the antibody or antigen-binding fragment thereof. In one embodiment, the selectivity is at least 500, 1000, 2500, 6000 or 13,000. In another embodiment, the selectivity is 1567, 6286 or 13,636.

Examples of antibodies that can be used in the compositions and methods of the invention, in some embodiments, are provided herein. In another embodiment, the antibody or antigen-binding fragment thereof is a monoclonal antibody or antigen-binding fragment thereof that specifically binds PSMA. In yet another embodiment, the antibody or antigen-binding fragment thereof is a monoclonal antibody or antigen-binding fragment thereof that specifically binds an extracellular domain of PSMA. In a further embodiment, the antibody or antigen-binding fragment thereof is a monoclonal antibody or antigen-binding fragment thereof that specifically binds to a conformational epitope of PSMA.

In some embodiments, the antibody or antigen-binding fragment thereof (i) competitively inhibits the specific binding of a second antibody to its target epitope on PSMA, or (ii) binds to an epitope on PSMA defined by an antibody selected from the group consisting of PSMA 3.7, PSMA 3.8, PSMA 3.9, PSMA 3.11, PSMA 5.4, PSMA 7.1, PSMA 7.3, PSMA 10.3, PSMA 1.8.3, PSMA A3.1.3, PSMA A3.3.1, Abgenix 4.248.2, Abgenix 4.360.3, Abgenix 4.7.1, Abgenix 4.4.1, Abgenix 4.177.3, Abgenix 4.16.1, Abgenix 4.22.3, Abgenix 4.28.3, Abgenix 4.40.2, Abgenix 4.48.3, Abgenix 4.49.1, Abgenix 4.209.3, Abgenix 4.219.3, Abgenix 4.288.1, Abgenix 4.333.1, Abgenix 4.54.1, Abgenix 4.153.1, Abgenix 4.232.3, Abgenix 4.292.3, Abgenix 4.304.1, Abgenix 4.78.1 and Abgenix 4.152.1. In other embodiments, the antibody or antigen-binding fragment thereof binds to an epitope on PSMA defined by an antibody selected from the group consisting of antibodies comprising (a) a heavy chain encoded by a nucleic acid molecule comprising a coding region or regions of a nucleotide sequence selected from the group consisting of nucleotide sequences set forth as SEQ ID NOs: 2-7, and (b) a light chain encoded by a nucleic acid molecule comprising a coding region or regions of a nucleotide sequence selected from the group consisting of nucleotide sequences set forth as SEQ ID NOs: 8-13.

In some embodiments, the second antibody is selected from the group consisting of PSMA 3.7, PSMA 3.8, PSMA 3.9, PSMA 3.11, PSMA 5.4, PSMA 7.1, PSMA 7.3, PSMA 10.3, PSMA 1.8.3, PSMA A3.1.3, PSMA A3.3.1, Abgenix 4.248.2, Abgenix 4.360.3, Abgenix 4.7.1, Abgenix 4.4.1, Abgenix 4.177.3, Abgenix 4.16.1, Abgenix 4.22.3, Abgenix 4.28.3, Abgenix 4.40.2, Abgenix 4.48.3, Abgenix 4.49.1, Abgenix 4.209.3, Abgenix 4.219.3, Abgenix 4.288.1, Abgenix 4.333.1, Abgenix 4.54.1, Abgenix 4.153.1, Abgenix 4.232.3, Abgenix 4.292.3, Abgenix 4.304.1, Abgenix 4.78.1, Abgenix 4.152.1 and antibodies comprising (a) a heavy chain encoded by a nucleic acid molecule comprising a coding region or regions of a nucleotide sequence selected from the group consisting of nucleotide sequences set forth as SEQ ID NOs: 2-7, and (b) a light chain encoded by a nucleic acid molecule comprising a coding region or regions of a nucleotide sequence selected from the group consisting of nucleotide sequences set forth as SEQ ID NOs: 8-13.

In other embodiments, the second antibody is selected from the group consisting of AB-PG1-XG1-006, AB-PG1-XG1-026 and antibodies comprising (a) a heavy chain encoded by a nucleic acid molecule comprising a coding region or regions of a nucleotide sequence selected from the group consisting of nucleotide sequences set forth as SEQ ID NOs: 2 and 3, and (b) a light chain encoded by a nucleic acid molecule comprising a coding region or regions of a nucleotide sequence selected from the group consisting of nucleotide sequences set forth as SEQ ID NOs: 8 and 9. In one embodiment, the second antibody comprises (a) a heavy chain encoded by a nucleic acid molecule comprising a coding region or regions of a nucleotide sequence set forth as SEQ ID NO: 2, and (b) a light chain encoded by a nucleic acid molecule comprising a coding region or regions of a nucleotide sequence set forth as SEQ ID NO: 8. In a further embodiment, the second antibody comprises (a) a heavy chain encoded by a nucleic acid molecule comprising a coding region or regions of a nucleotide sequence set forth as SEQ ID NO: 3, and (b) a light chain encoded by a nucleic acid molecule comprising a coding region or regions of a nucleotide sequence set forth as SEQ ID NO: 9.

In some embodiments, the antibody of the antibody-drug conjugate is an antibody encoded by a nucleic acid molecule comprising a nucleotide sequence that is at least 90% identical to a nucleotide sequence encoding an antibody selected from the group consisting of AB-PG1-XG1-006, AB-PG1-XG1-026 and antibodies comprising (a) a heavy chain encoded by a nucleic acid molecule comprising a coding region or regions of a nucleotide sequence selected from the group consisting of nucleotide sequences set forth as SEQ ID NOs: 2 and 3, and (b) a light chain encoded by a nucleic acid molecule comprising a coding region or regions of a nucleotide sequence selected from the group consisting of nucleotide sequences set forth as SEQ ID NOs: 8 and 9. In one embodiment, the antibody is encoded by a nucleic acid molecule comprising a nucleotide sequence that is at least 95% identical. In another embodiment, the antibody is encoded by a nucleic acid molecule comprising a nucleotide sequence that is at least 97% identical. In yet another embodiment, the antibody is encoded by a nucleic acid molecule comprising a nucleotide sequence that is at least 98% identical. In a further embodiment, the antibody is encoded by a nucleic acid molecule comprising a nucleotide sequence that is at least 99% identical.

In other embodiments, the antibody or antigen-binding fragment thereof of the antibody-drug conjugates provided herein is AB-PG1-XG1-006, AB-PG1-XG1-026 or an antigen-binding fragment thereof. In still other embodiments, the antibody or antigen-binding fragment thereof is selected from the group consisting of antibodies comprising (a) a heavy chain encoded by a nucleic acid molecule comprising a coding region or regions of a nucleotide sequence selected from the group consisting of nucleotide sequences set forth as SEQ ID NOs: 2 and 3, and (b) a light chain encoded by a nucleic acid molecule comprising a coding region or regions of a nucleotide sequence selected from the group consisting of nucleotide sequences set forth as SEQ ID NOs: 8 and 9, and antigen-binding fragments thereof. In one embodiment, the antibody or antigen-binding fragment thereof comprises (a) a heavy chain encoded by a nucleic acid molecule comprising a coding region or regions of a nucleotide sequence set forth as SEQ ID NO: 2, and (b) a light chain encoded by a nucleic acid molecule comprising a coding region or regions of a nucleotide sequence set forth as SEQ ID NO: 8, and antigen-binding fragments thereof. In another embodiment, the antibody or antigen-binding fragment thereof comprises (a) a heavy chain encoded by a nucleic acid molecule comprising a coding region or regions of a nucleotide sequence set forth as SEQ ID NO: 3, and (b) a light chain encoded by a nucleic acid molecule comprising a coding region or regions of a nucleotide sequence set forth as SEQ ID NO: 9, and antigen-binding fragments thereof.

In some embodiments, the antibody or antigen-binding fragment thereof is IgG1, IgG2, IgG3, IgG4, IgM, IgA1, IgA2, IgAsec, IgD, IgE or has immunoglobulin constant and/or variable domain of IgG1, IgG2, IgG3, IgG4, IgM, IgA1, IgA2, IgAsec, IgD or IgE.

In further embodiments, the antibody is a monoclonal antibody. In still other embodiments, the antibody is a humanized antibody. In yet other embodiments, the antibody is a human antibody. In still other embodiments, the antibody is a recombinant antibody. In further embodiments, the antibody is a chimeric antibody. In still further embodiments, the antibody is a bispecific or multispecific antibody. In yet other embodiments, the antibody is a single chain antibody.

In other embodiments, the antigen-binding fragment is a Fab fragment, a F(ab')₂ fragment or a Fv fragment. In yet other embodiments, the antigen-binding fragment is a CDR3-containing fragment.

In some embodiments, the monomethylauristatin norephedrine (MMAE) or monomethylauristatin phenylalanine (MMAF) is conjugated to the antibody or antigen-binding fragment thereof with a compound of the formula **(Formula 1)** -Aₙ-Yₘ-Zₘ-Xₙ-Wₙ-, wherein A is a carboxylic acyl unit; Y is an amino acid; Z is an amino acid; X and W are each a self-immolative spacer; n is an integer of 0 or 1; and m is an integer of 0 or 1, 2, 3, 4, 5 or 6. In some embodiments, the conjugate of the present invention is represented by the formula **(Formula 2):** L-{Aₙ-Yₘ-Zₘ-Xₙ-Wₙ-D}ₚ wherein L is an antibody or antigen-binding fragment thereof that binds PSMA, D is MMAE or MMAF and p is an integer of 1, 2, 3, 4, 5, 6, 7 or 8. The rest of the components of the conjugate are as defined immediately above.

In one embodiment, the carboxylic unit "Aₙ" is linked to the antibody or antigen-binding fragment thereof via a sulfur atom derived from the antibody or antigen-binding fragment thereof:

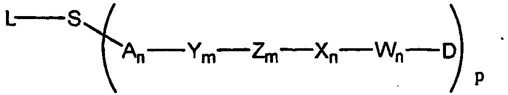

In one embodiment, A is 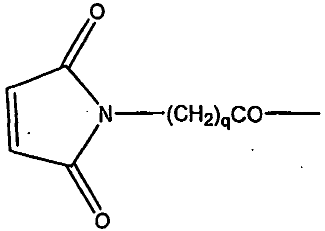 in which q is 1-10. Therefore, in one embodiment, the conjugate of **Formula 2** is: 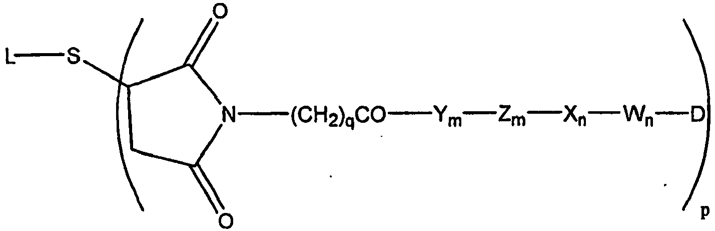 wherein L, Y, Z, X, W, D, n, m, q and p are as previously defined.

In another embodiment, A is 4-(N-succinimidomethyl)cyclohexane-1-carbonyl, m-succinimidobenzoyl, 4-(p-succinimidophenyl) -butyryl, 4-(2-acetamido)benzoyl, 3-thiopropionyl, 4-(1-thioethyl)-benzoyl, 6-(3-thiopropionylamido)-hexanoyl or maleimide caproyl. In a further embodiment, A is maleimide caproyl.

In another embodiment, Y is alanine, valine, leucine, isoleucine, methionine, phenylalanine, tryptophan or proline. In yet another embodiment, Y is valine. In a further embodiment, Z is lysine, lysine protected with acetyl or formyl, arginine, arginine protected with tosyl or nitro groups, histidine, ornithine, ornithine protected with acetyl or formyl, or citrulline. In still a further embodiment, Z is citrulline. In one embodiment Yₘ-Zₘ is valine-citrulline. In another embodiment, Yₘ-Zₘ is a protein sequence which is selectively cleavable by a protease.

In a further embodiment; X is a compound having the formula 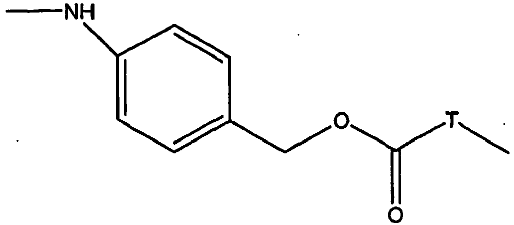 in which T is O, N, or S. In another embodiment, X is a compound having the formula -HN-R¹ -COT in which R¹ is C₁ -C₅ alkyl, T is O, N or S. In a further embodiment, X is a compound having the formula 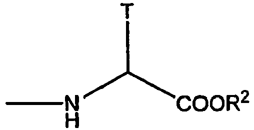 in which T is O, N, or S, R² is H or C₁ -C₅ alkyl. In one embodiment, X is p-aminobenzylcarbamoyloxy. In another embodiment, X is p-aminobenzylalcohol. In a further embodiment, X is p-aminobenzylcarbamate. In yet a further embodiment, X is p-aminobenzyloxycarbonyl. In another embodiment, X is γ-aminobutyric acid; α,α-dimethyl γ-aminobutyric acid or β,β-dimethyl γ-aminobutyric acid.

In some embodiments, W is 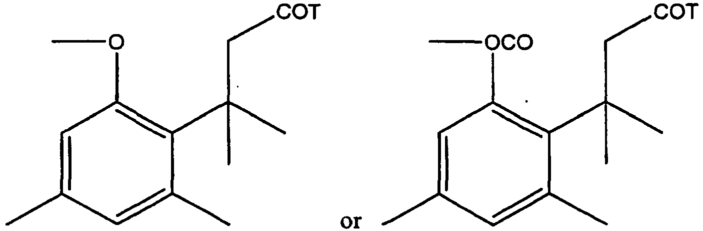 in which T is O, S or N.

In other embodiments, m and n are 0.

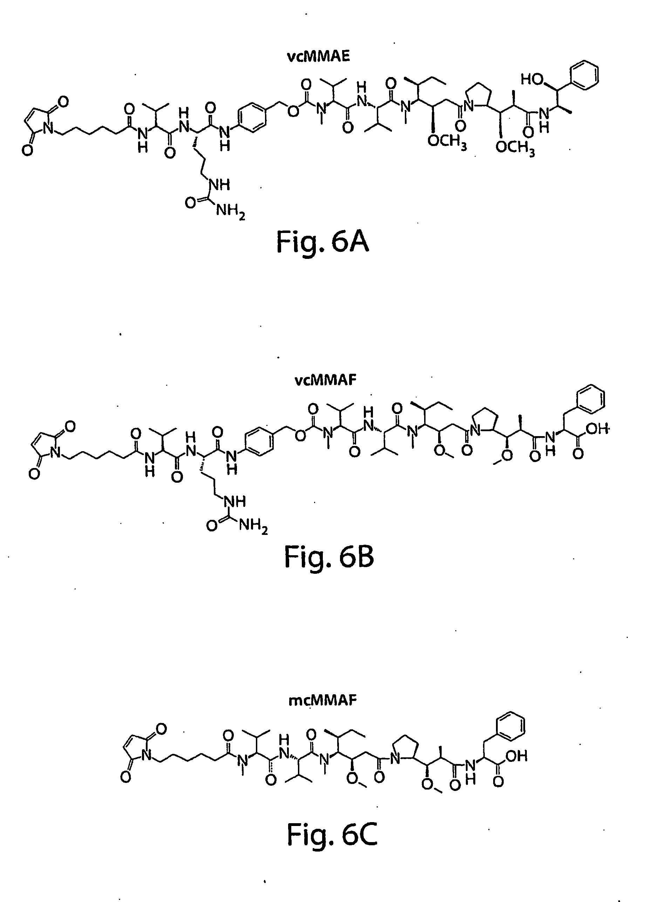

In one embodiment, the antibody-drug conjugate is AB-PG1-XG1-006-maleimide caproyl-valine-citrulline-p-aminobenzyloxycarbonyl-monomethylauristatin norephedrine. In another embodiment, the antibody-drug conjugate is AB-PG1-XG1-006-maleimide caproyl-valine-citrulline-p-aminobenzyloxycarbonyl-monomethylauristatin phenylalanine. In a further embodiment, the antibody-drug conjugate is AB-PG1-XG1-006-maleimide caproyl-monomethylauristatin phenylalanine. In another embodiment, the antibody-drug conjugate is AB-PG1-XG1-026-maleimide caproyl-valine-citrulline-p-aminobenzyloxycarbonyl-monomethylauristatin norephedrine. In yet another embodiment, the antibody-drug conjugate is AB-PG1-XG1-026-maleimide caproyl-valine-citrulline-p-aminobenzyloxycarbonyl-monomethylauristatin phenylalanine. In a further embodiment, the antibody-drug conjugate is AB-PG1-XG1-026-maleimide caproyl-monomethylauristatin phenylalanine. In another embodiment, the antibody-drug conjugate is a PSMA-binding antibody or antigen-binding fragment thereof conjugated to the compound as shown in **Fig. 6A, Fig. 6B or Fig. 6C****.**

In some embodiments, the antibody-drug conjugate binds live cells. In one embodiment, the cell is a tumor cell. In another embodiment, the tumor cell is a prostate tumor cell. In a further embodiment, the tumor cell is a cell of the neovasculature of a non-prostate tumor. In other embodiments, the antibody-drug conjugate does not require cell lysis to bind PSMA. In still other embodiments, the antibody-drug conjugate leads to cell-cycle arrest. In yet further embodiments, the antibody-drug conjugate inhibits the growth of PSMA-expressing cells. In one embodiment, the antibody-drug conjugate mediates specific cell killing of PSMA-expressing cells with an IC₅₀ of less than 1X10⁻¹⁰M. In another embodiment, the IC₅₀ is less than 1X10⁻¹¹M. In yet another embodiment,the IC₅₀ is less than 1X10⁻¹²M. In a further embodiment, the antibody-drug conjugate mediates specific cell killing of PSMA-expressing cells with an IC₅₀ of 11 to 208 X10⁻¹²M. In still a further embodiment, the antibody-drug conjugate mediates specific cell killing of PSMA-expressing cells with an IC₅₀ of 42 to 208 X10⁻¹²M. In yet a further embodiment, the antibody-drug conjugate mediates specific cell killing of PSMA-expressing cells with an IC₅₀ of 60 to 208 X10⁻¹²M. In another embodiment, the antibody-drug conjugate mediates specific cell killing of PSMA-expressing cells with an IC₅₀ of 65 to 208 X10⁻¹²M. In one embodiment, the antibody-drug conjugate mediates specific cell killing of PSMA-expressing cells with an IC₅₀ of 11 X10⁻¹²M. In another embodiment, the antibody-drug conjugate mediates specific cell killing of PSMA-expressing cells with an IC₅₀ of 42 X10⁻¹²M. In still another embodiment, the antibody-drug conjugate mediates specific cell killing of PSMA-expressing cells with an IC₅₀ of 60 X10⁻¹²M. In a further embodiment, the antibody-drug conjugate mediates specific cell killing of PSMA-expressing cells with an IC₅₀ of 83 X10⁻¹²M.

In another embodiment, the antibody-drug conjugate, when administered to mice with a regimen of q4d x 6 at a dose of 6 mg/kg effects a cure rate of at least 20%, 30%, 40% or 50%. In one embodiment, the cure rate is 20%, 30%, 40%, 50%, 60%, 70%, 80% or more. In one embodiment, the mice are those that are a model of androgen-independent human prostate cancer. In another embodiment, the mice are nude mice engrafted with C4-2 cells intramuscularly in the left hind-leg. In a further embodiment, the mice are those as provided in the Examples.

In some embodiments, the antibody-drug conjugate is bound to a label. In other embodiments, the label is a fluorescent label, an enzyme label, a radioactive label, a nuclear magnetic resonance active label, a luminescent label or a chromophore label.

In some embodiments, the antibody-drug conjugate is packaged in lyophilized form. In other embodiments, the antibody-drug conjugate is packaged in an aqueous medium. In further embodiments, the antibody-drug conjugate is in a sterile form.

Also provided herein are compositions comprising one or more antibody-drug conjugates. In some embodiments, the composition comprises two or more different antibody-drug conjugates. In other embodiments, a composition comprising one or more antibody-drug conjugates and one or more unconjugated anti-PSMA antibodies is provided.

In some embodiments, the composition further comprises a pharmaceutically acceptable carrier, excipient or stabilizer. In other embodiments, the composition further comprises an antitumor agent, an immunostimulatory agent, an immunomodulator, a corticosteroid or a combination thereof. In one embodiment, the antitumor agent is a cytotoxic agent, an agent that acts on tumor neovasculature or a combination thereof. In another embodiment, the antitumor agent is docetaxel. In still another embodiment, the immunomodulator is a cytokine, chemokine, adjuvant or a combination thereof. In yet another embodiment, the immunostimulatory agent is interleukin-2, α-interferon, γ-interferon, tumor necrosis factor-α, immunostimulatory oligonucleotides or a combination thereof. In a further embodiment, the corticosteroid is prednisone or hydrocortisone. In still a further embodiment, the composition comprises prednisone and docetaxel.

The antibody-drug conjugates and compositions of the invention can be used in a variety of methods. In one embodiment, the antibody-drug conjugate is for use in a metnod for inhibiting the growth of a PSMA-expressing cell comprising contacting the PSMA-expressing cell with an amount of an antibody-drug conjugate effective to inhibit the growth of the PSMA-expressing cell. In another embodiment, the antibody-drug conjugate is for use in a method for effecting cell-cycle arrest in a PSMA-expressing cell comprising contacting the PSMA-expressing cell with an amount of an antibody-drug conjugate effective to lead to cell-cycle arrest in the PSMA-expressing cell. In still another embodiment, the antibody-drug conjugate is for use in a method for treating a PSMA-mediated disease comprising administering to a subject having a PSMA-mediated disease an amount of an antibody-drug conjugate effective to treat the PSMA-mediated disease. In a further embodiment, the antibody-drug conjugate is for use in a method for inhibiting the growth of a tumor comprising contacting PSMA-expressing cells of the neovasculature of the tumor with an amount of an antibody-drug conjugate effective to inhibit the growth of the tumor.

In one embodiment, the PSMA-mediated disease is cancer. In another embodiment, the cancer is a prostate cancer. In yet another embodiment, the cancer is a non-prostate cancer. In some embodiments, the non-prostate cancer is bladder cancer, pancreatic cancer, lung cancer, kidney cancer, sarcoma, breast cancer, brain cancer, neuroendocrine carcinoma, colon cancer, testicular cancer or melanoma.

In some embodiments, the method further comprises co-administering another therapeutic agent to treat the PSMA-mediated disease. In other embodiments, the method further comprises contacting PSMA-expressing cells with another therapeutic agent. In some embodiments, the other therapeutic agent is to be administered before, during or after the administration of the antibody-drug conjugate. In one embodiment, the other therapeutic agent is an antitumor agent, an immunostimulatory agent, an immunomodulator, a corticosteroid or a combination thereof. In another embodiment, the antitumor agent is a cytotoxic agent, an agent that acts on tumor neovasculature or a combination thereof. In yet another embodiment, the antitumor agent is docetaxel. In still another embodiment, the immunomodulator is a cytokine, chemokines, adjuvant or a combination thereof. In yet another embodiment, the immunostimulatory agent is interleukin-2, α-interferon, γ-interferon, tumor necrosis factor-α, immunostimulatory oligonucleotides or a combination thereof. In a further embodiment, the corticosteroid is prednisone or hydrocortisone. In one embodiment, the therapeutic agent is a vaccine. In another embodiment, the vaccine immunizes the subject against PSMA. In another embodiment, the method further comprises administering still another therapeutic agent. In one embodiment, the still another therapeutic agent is prednisone. In one embodiment, therefore, both docetaxel and prednisone are administered.

The PSMA-expressing cell is, in some embodiments, a prostate tumor cell or a cell of the neovasculature of a non-prostate tumor. In some embodiments, the PSMA-expressing cell is an androgen-dependent cell or an androgen-independent cell.

Each of the limitations of the invention can encompass various embodiments of the invention. It is, therefore, anticipated that each of the limitations of the invention involving any one element or combinations of elements can be included in each aspect of the invention.

### Brief Description of the Figures

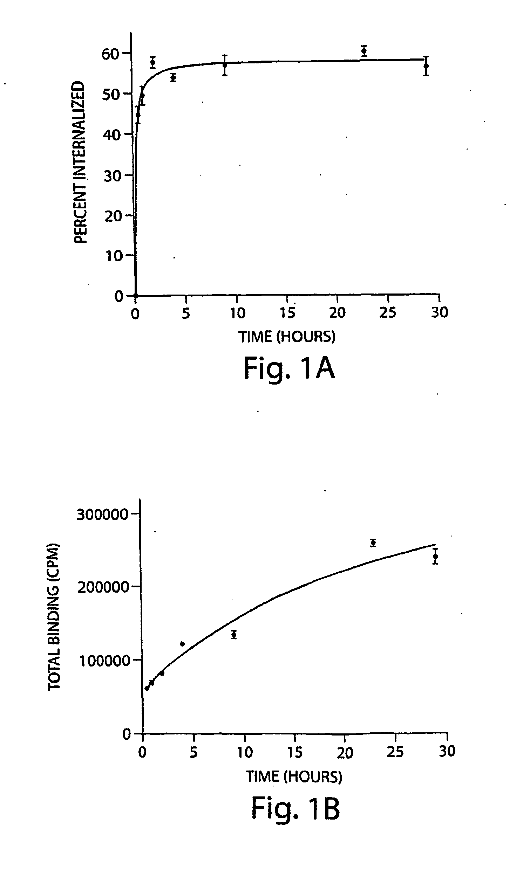

**Fig.1** is a graph that shows the percent internalization and total binding of ¹¹¹Inlabeled PSMA mAb on C4-2 cells. C4-2 cells were incubated with ¹¹¹In-labeled mAb at 37°C, 5% CO₂. At the designated times, cells were washed to remove unbound mAb, and surface bound mAb was stripped using low pH buffer. The radioactivity (counts per minute (CPM)) of the low pH eluate and cell pellet was counted separately using a gamma counter. Percent internalization (**Fig. 1A**) was calculated as the CPM cell pellet/(CPM cell pellet + CPM low pH eluate) x 100. Total binding (**Fig. 1B**) represents the CPM of the cell pellet plus the CPM of the low pH eluate.

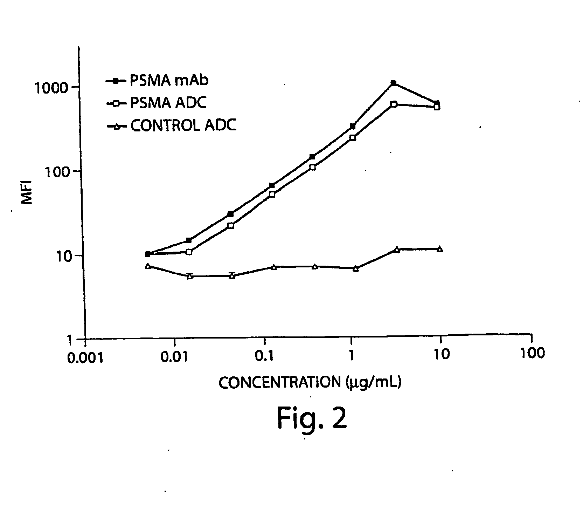

**Fig. 2** is a graph showing the binding of PSMA mAb and ADC to 3T3™-PSMA cells. 3T3™-PSMA cells were incubated with increasing concentrations of the PSMA mAb (filled squares), PSMA ADC (open squares) or isotype-control ADC (open triangles). Cells were incubated on ice for 1h and washed to remove unbound mAb or ADC. The cells were then incubated with goat anti-human IgG-FITC, washed again and examined by flow cytometry. The mean fluorescence intensities (MFIs) are plotted as a function of mAb or ADC concentration.

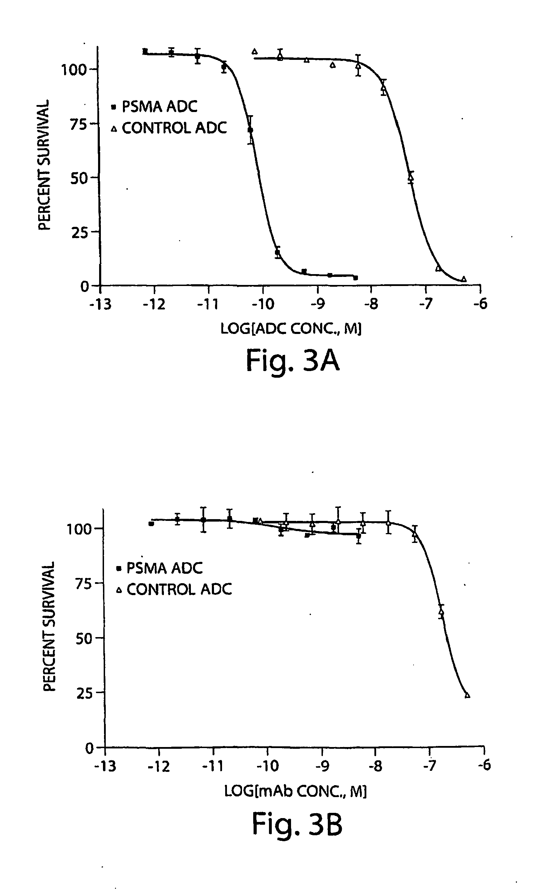

**Fig. 3** is a graph showing the *in vitro* cytotoxicity of the PSMA ADC and control ADC on PSMA-positive and PSMA-negative prostate cancer cell lines. PSMA-positive C4-2 cells (**Fig. 3A**) and PSMA-negative PC-3™ cells (**Fig. 3B**) in 96-well microplates were exposed to ADCs at various concentrations. After 96 hours, cell survival in treated and untreated cultures was assayed using Alamar Blue.

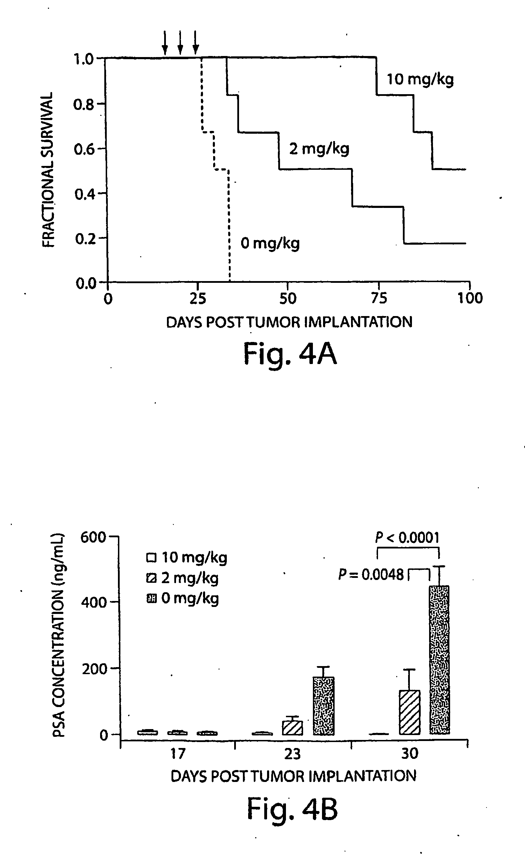

**Fig. 4** is a graph showing the Kaplan-Meier survival and serum PSA levels in a xenograft study. Nude mice were implanted intramuscularly with C4-2 cells, randomly assigned to treatment groups (6 mice per group) according to serum PSA on day 17 and then treated q4d x 3 with PSMA ADC or vehicle. **Fig. 4A** shows the survival of animals treated with 0 (vehicle control, dashed line), 2 mg/kg (thin solid line) and 10 mg/kg PSMA ADC. **Fig. 4B** provides the mean PSA values over 30 days in mice treated with 0 (filled columns), 2 mg/kg (striped columns) and 10 mg/kg (open columns) PSMA ADC. The day 30 data for the control group include day 27 evaluations for two mice which did not survive 30 days.

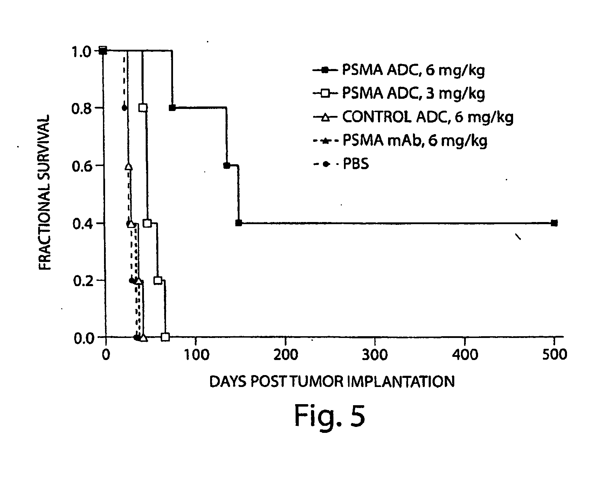

**Fig. 5** shows Kaplan-Meier survival curves of animals treated in another xenograft study. Nude mice were implanted intramuscularly with C4-2 cells, randomly assigned to treatment groups (5 mice per group) according to serum PSA on day 14 and then treated q4d x 6 with PSMA ADC and controls. Mice were treated with 0 (vehicle control, filled circles), 6 mg/kg unmodified PSMA mAb (filled triangles), 6 mg/kg control ADC (open triangles), 3 mg/kg PSMA ADC (open squares) and 6 mg/kg PSMA ADC (filled squares).
**Fig. 6** shows the chemical structures of three different drug-linkers. **Fig 6A** provides the structure of vcMMAE (maleimidocaproyl-valine-citrulline-p-aminobenzyloxycarbonyl-monomethylauristatin E). **Fig 6B** provides the structure of vcMMAF (maleimidocaproyl-valine-citrulline-p-aminobenzyloxycarbonyl-monomethylauristatin F). **Fig 6C** provides the structure of mcMMAF (maleimidocaproyl-monomethylauristatin F).

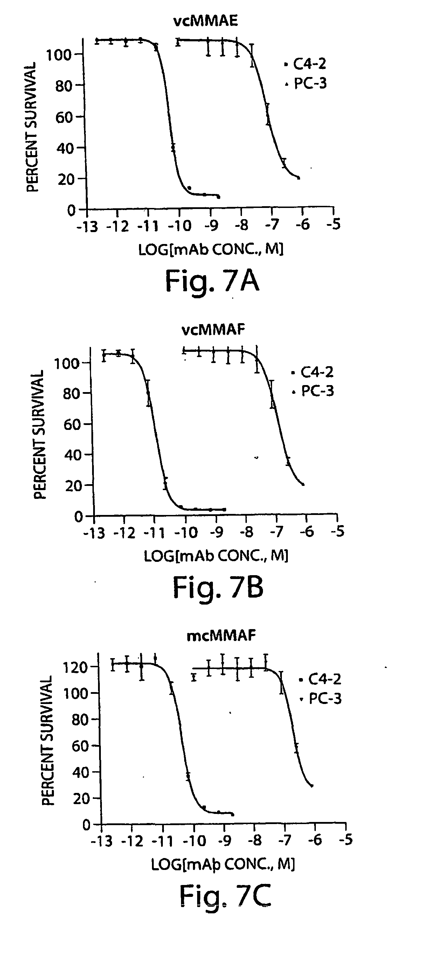

**Fig. 7** demonstrates the *in vitro* cytotoxicity of the PSMA ADCs (vcMMAE (**Fig. 7A**), vcMMAF (**Fig. 7B**), mcMMAF (**Fig. 7C**)) on PSMA-positive (C4-2) and PSMA-negative (PC-3™) prostate cancer cell lines. The cells in 96-well microplates were exposed to ADCs at various concentrations. After 4 days, cell survival in treated and untreated cultures was assayed using Alamar Blue.

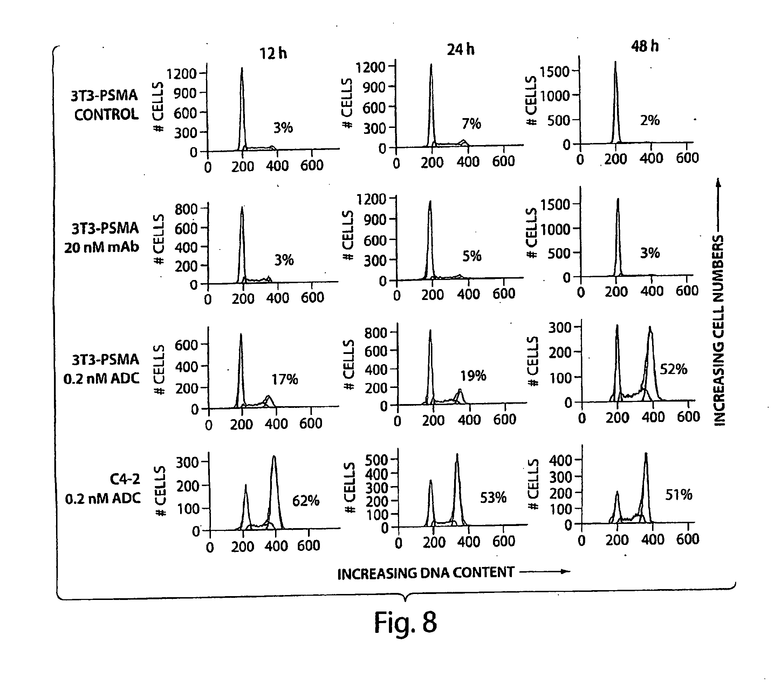

**Fig. 8** illustrates effects of PSMA ADC on cell cycle. In each panel, the left peak corresponds to G₁ phase and the right peak to G₂/M phase. The percent of cells in G₂/M increased markedly upon treatment with the PSMA ADC, consistent with an arrest in cell division that occurs after DNA synthesis. The PSMA ADC did not affect cycling of parental 3T3™ cells.

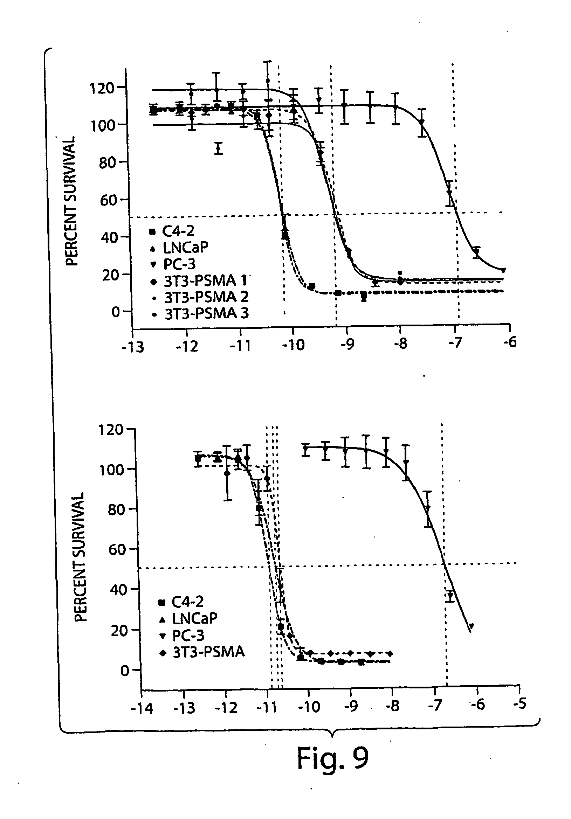

**Fig. 9** shows the results from a comparison of PSMA ADCs vcMMAE v. vcMMAF.

### Detailed Description of the Invention

The present invention relates, in part, to the surprising discovery that ADCs comprising a PSMA-binding antibody or antigen-binding fragment thereof conjugated to MMAE (also referred to herein as monomethylauristatin E and monomethylauristatin norephedrine) or MMAF (also referred to herein as monomethylauristatin F and monomethylauristatin phenylalanine) are particularly useful for killing PSMA-expressing cells. The ADCs have a PC-3™ cell to C4-2 or LNCaP™ cell selectivity of at least 250, and 3 or 4 molecules of monomethylauristatin norephedrine or monomethylauristatin phenylalanine are conjugated ton the antibody or antigen-binding fragment thereof. In some embodiments, the ADCs exhibit certain levels of cell killing (of PSMA-expressing cells), e.g., IC₅₀ values that are at or near picomolar concentrations. In other embodiments, the ADCs effect a cure rate of at least 20%, 30%, 40% or 50% in mice treated with the ADC with a regimen of q4d x 6 at a dose of 6 mg/kg. Compositions of and methods of using these ADCs are, therefore, provided. In some embodiments, the mice are those as provided in the Examples. In one embodiment, the mice are those that are a model of androgen-independent human prostate cancer. In another embodiment, the mice are nude mice engrafted with C4-2 cells intramuscularly in the left hind-leg.

The antibodies or antigen-binding fragments thereof of the ADCs are any antibody or antigen-binding fragment thereof that binds PSMA. In one embodiment the antibody or an antigen-binding fragment thereof specifically binds PSMA (e.g., specifically binds an extracellular domain of PSMA, specifically binds a conformational epitope of PSMA, etc.) and can competitively inhibit the specific binding of a second antibody to its target epitope on PSMA, wherein the second antibody is selected from the group consisting of PSMA 3.7, PSMA 3.8, PSMA 3.9, PSMA 3.11, PSMA 5.4, PSMA 7.1, PSMA 7.3, PSMA 10.3, PSMA 1.8.3, PSMA A3.1.3, PSMA A3.3.1, Abgenix 4.248.2, Abgenix 4.360.3, Abgenix 4.7.1, Abgenix 4.4.1, Abgenix 4.177.3, Abgenix 4.16.1, Abgenix 4.22.3, Abgenix 4.28.3, Abgenix 4.40.2, Abgenix 4.48.3, Abgenix 4.49.1, Abgenix 4.209.3, Abgenix 4.219.3, Abgenix 4.288.1, Abgenix 4.333.1, Abgenix 4.54.1, Abgenix 4.153.1, Abgenix 4.232.3, Abgenix 4.292.3, Abgenix 4.304.1, Abgenix 4.78.1, Abgenix 4.152.1 and antibodies comprising (a) a heavy chain encoded by a nucleic acid molecule comprising a coding region or regions of a nucleotide sequence selected from the group consisting of nucleotide sequences set forth as SEQ ID NOs: 2-7, and (b) a light chain encoded by a nucleic acid molecule comprising a coding region or regions of a nucleotide sequence selected from the group consisting of nucleotide sequences set forth as SEQ ID NOs: 8-13. The second antibody, therefore, include any of the antibodies produced by the hybridomas or encoded by the plasmids shown below in **Table 1**. These hybridomas and plasmids were deposited pursuant to, and in satisfaction of, the requirements of the Budapest Treaty on the International Recognition of the Deposit of Microorganisms for the Purposes of Patent Procedure with the American Type Culture Collection ("ATCC") as an International Depository Authority and given the Patent Deposit Designations shown above and in **Table 1**.

**Table 1**

| Antibody | Hybridoma/Plasmid | Patent Deposit Designation | Date of Deposit |
|---|---|---|---|
| PSMA 3.7 | PSMA 3.7 | PTA-3257 | April 5, 2001 |
| PSMA 3.9 | PSMA 3.9 | PTA-3258 | April 5, 2001 |
| PSMA 3.11 | PSMA 3.11 | PTA-3269 | April 10, 2001 |
| PSMA 5.4 | PSMA 5.4 | PTA-3268 | April 10, 2001 |
| PSMA 7.1 | PSMA 7.1 | PTA-3292 | April 18, 2001 |
| PSMA 7.3 | PSMA 7.3 | PTA-3293 | April 18, 2001 |
| PSMA 10.3 | PSMA 10.3 | PTA-3347 | May 1, 2001 |
| | PSMA 10.3 HC in pcDNA (SEQ ID NO: 7) | PTA-4413 | May 29, 2002 |
| | PSMA 10.3 Kappa in pcDNA (SEQ ID NO: 13) | PTA-4414 | May 29, 2002 |
| PSMA 1.8.3 | PSMA 1.8.3 | PTA-3906 | Dec. 5, 2001 |
| PSMA A3.1.3 | PSMA A3.1.3 | PTA-3904 | Dec. 5, 2001 |
| PSMA A3.3.1 | PSMA A3.3.1 | PTA-3905 | Dec. 5, 2001 |
| Abgenix 4.248.2 | Abgenix 4.248.2 | PTA-4427 | June 4, 2002 |
| Abgenix 4.360.3 | Abgenix 4.360.3 | PTA-4428 | June 4, 2002 |
| Abgenix 4.7.1 | Abgenix 4.7.1 | PTA-4429 | June 4, 2002 |
| Abgenix 4.4.1 | Abgenix 4.4.1 | PTA-4556 | July 18, 2002 |
| Abgenix 4.177.3 | Abgenix 4.177.3 | PTA-4557 | July 18, 2002 |
| Abgenix 4.16.1 | Abgenix 4.16.1 | PTA-4357 | May 16, 2002 |
| Abgenix 4.22.3 | Abgenix 4.22.3 | PTA-4358 | May 16, 2002 |
| Abgenix 4.28.3 | Abgenix 4.28.3 | PTA-4359 | May 16, 2002 |
| Abgenix 4.40.2 | Abgenix 4.40.2 | PTA-4360 | May 16, 2002 |
| Abgenix 4.48.3 | Abgenix 4.48.3 | PTA-4361 | May 16, 2002 |
| Abgenix 4.49.1 | Abgenix 4.49.1 | PTA-4362 | May 16, 2002 |
| Abgenix 4.209.3 | Abgenix 4.209.3 | PTA-4365 | May 16, 2002 |
| Abgenix 4.219.3 | Abgenix 4.219.3 | PTA-4366 | May 16, 2002 |
| Abgenix 4.288.1 | Abgenix 4.288.1 | PTA-4367 | May 16, 2002 |
| Abgenix 4.333.1 | Abgenix 4.333.1 | PTA-4368 | May 16, 2002 |
| Abgenix 4.54.1 | Abgenix 4.54.1 | PTA-4363 | May 16, 2002 |
| Abgenix 4.153.1 | Abgenix 4.153.1 | PTA-4388 | May 23, 2002 |
| Abgenix 4,232.3 | Abgenix 4.232.3 | PTA-4389 | May 23, 2002 |
| Abgenix 4.292.3 | Abgenix 4.292.3 | PTA-4390 | May 23, 2002 |
| Abgenix 4.304.1 | Abgenix 4.304.1 | PTA-4391 | May 23, 2002 |
| AB-PG1-XG1-006 | AB-PG1-XG1-006 Heavy Chain (SEQ ID NO: 2) | PTA-4403 | May 29, 2002 |
| | AB-PG1-XG1-006 Light Chain (SEQ ID NO: 8) | PTA-4404 | |
| AB-PG1-XG1-026 | AB-PG1-XG1-026 Heavy Chain (SEQ ID NO: 3) | PTA-4405 | May 29, 2002 |
| | AB-PG1-XG1-026 Light Chain (SEQ ID NO: 9) | PTA-4406 | |
| AB-PG1-XG1-051 | AB-PG1-XG1-051 Heavy Chain (SEQ ID NO: 4) | PTA-4407 | May 29, 2002 |
| | AB-PG1-XG1-051 Light Chain (SEQ ID NO: 10) | PTA-4408 | |
| AB-PG1-XG1-069 | AB-PG1-XG1-069 Heavy Chain (SEQ ID NO: 5) | PTA-4409 | May 29, 2002 |
| | AB-PG1-XG1-069 Light Chain (SEQ ID NO: 11) | PTA-4410 | |
| AB-PG1-XG1-077 | AB-PG1-XG1-077 Heavy Chain (SEQ ID NO: 6) | PTA-4411 | May 29, 2002 |
| | AB-PG1-XG1-077 Light Chain (SEQ ID NO: 12) | PTA-4412 | |

To determine competitive inhibition, a variety of assays known to one of ordinary skill in the art can be employed. For example, cross-competition assays can be used to determine if an antibody or antigen-binding fragment thereof competitively inhibits binding to PSMA by another antibody or antigen-binding fragment thereof. These include cell-based methods employing flow cytometry or solid phase binding analysis. Other assays that evaluate the ability of antibodies or antigen-binding fragments thereof to cross-compete for PSMA molecules that are not expressed on the surface of cells, in solid phase or in solution phase, also can be used.

In some embodiments, the antibodies or antigen-binding fragments thereof competitively inhibit the specific binding of a second antibody to its target epitope on PSMA by at least about 10%, 20%, 30%, 40%, 50%, 60%, 70%, 80%, 90%, 95%, or 99%. Inhibition can be assessed at various molar ratios or mass ratios; for example competitive binding experiments can be conducted with a 2-fold, 3-fold, 4-fold, 5-fold, 7-fold, 10-fold or more molar excess of a first antibody or antigen-binding fragment thereof over a second antibody or antigen-binding fragment thereof.

In another embodiment the antibody or an antigen-binding fragment thereof specifically binds to an epitope on PSMA defined by an antibody selected from the group consisting of PSMA 3.7, PSMA 3.8, PSMA 3.9, PSMA 3.11, PSMA 5.4, PSMA 7.1, PSMA 7.3, PSMA 10.3, PSMA 1.8.3, PSMA A3.1.3, PSMA A3.3.1, 4.248.2, 4.360.3, 4.7.1, 4.4.1, 4.177.3, 4.16.1, 4.22.3, 4.28.3, 4.40.2, 4.48.3, 4.49.1, 4.209.3, 4.219.3, 4.288.1, 4.333.1, 4.54.1, 4.153.1, 4.232.3, 4.292.3, 4.304.1, 4.78.1, and 4.152.1. PSMA 3.7, PSMA 3.8, PSMA 3.9, PSMA 3.11, PSMA 5.4, PSMA 7.1, PSMA 7.3, PSMA 10.3, PSMA 1.8.3, PSMA A3.1.3, PSMA A3.3.1, Abgenix 4.248.2, Abgenix 4.360.3, Abgenix 4.7.1, Abgenix 4.4.1, Abgenix 4.177.3, Abgenix 4.16.1, Abgenix 4.22.3, Abgenix 4.28.3, Abgenix 4.40.2, Abgenix 4.48.3, Abgenix 4.49.1, Abgenix 4.209.3, Abgenix 4.219.3, Abgenix 4.288.1, Abgenix 4.333.1, Abgenix 4.54.1, Abgenix 4.153.1, Abgenix 4.232.3, Abgenix 4.292.3, Abgenix 4.304.1, Abgenix 4.78.1, Abgenix 4.152.1 and antibodies comprising (a) a heavy chain encoded by a nucleic acid molecule comprising a coding region or regions of a nucleotide sequence selected from the group consisting of nucleotide sequences set forth as SEQ ID NOs: 2-7, and (b) a light chain encoded by a nucleic acid molecule comprising a coding region or regions of a nucleotide sequence selected from the group consisting of nucleotide sequences set forth as SEQ ID NOs: 8-13. The antibodies or antigen-binding fragments of the ADCs, therefore, include those that specifically bind to an epitope on PSMA defmed by the antibodies produced by the hybridomas or encoded by the plasmids provided above in **Table 1.**

To determine the epitope, one can use standard epitope mapping methods known in the art. For example, fragments (peptides) of PSMA antigen (e.g., synthetic peptides) that bind the antibody can be used to determine whether a candidate antibody or antigen-binding fragment thereof binds the same epitope. For linear epitopes, overlapping peptides of a defined length (e.g., 8 or more amino acids) are synthesized. The peptides can be offset by 1 amino acid, such that a series of peptides covering every 8 amino acid fragment of the PSMA protein sequence are prepared. Fewer peptides can be prepared by using larger offsets, e.g., 2 or 3 amino acids. In addition, longer peptides (e.g., 9-, 10- or 11-mers) can be synthesized. Binding of peptides to antibodies or antigen-binding fragments can be determined using standard methodologies including surface plasmon resonance (BIACORE) and ELISA assays. For examination of conformational epitopes, larger PSMA fragments can be used. Other methods that use mass spectrometry to define conformational epitopes have been described and can be used (see, e.g., Baerga-Ortiz et al., Protein Science 11:1300-1308, 2002 and references cited therein). Still other methods for epitope determination are provided in standard laboratory reference works, such as Unit 6.8 ("Phage Display Selection and Analysis of B-cell Epitopes") and Unit 9.8 ("Identification of Antigenic Determinants Using Synthetic Peptide Combinatorial Libraries") of Current Protocols in Immunology, Coligan et al., eds., John Wiley & Sons. Epitopes can be confirmed by introducing point mutations or deletions into a known epitope, and then testing binding with one or more antibodies or antigen-binding fragments to determine which mutations reduce binding of the antibodies or antigen-binding fragments.

In particular embodiments, the antibodies of the ADCs, or from which the antigen-binding fragments of the ADCs are derived, are those produced by hybridomas referred to herein as PSMA 3.7, PSMA 3.8, PSMA 3.9, PSMA 3.11, PSMA 5.4, PSMA 7.1, PSMA 7.3, PSMA 10.3, PSMA 1.8.3, PSMA A3.1.3, PSMA A3.3.1, Abgenix 4.248.2, Abgenix 4.360.3, Abgenix 4.7.1, Abgenix 4.4.1, Abgenix 4.177.3, Abgenix 4.16.1, Abgenix 4.22.3, Abgenix 4.28.3, Abgenix 4.40.2, Abgenix 4.48.3, Abgenix 4.49.1, Abgenix 4.209.3, Abgenix 4.219.3, Abgenix 4.288.1, Abgenix 4.333.1, Abgenix 4.54.1, Abgenix 4.153.1, Abgenix 4.232.3, Abgenix 4.292.3, Abgenix 4.304.1, Abgenix 4.78.1, and Abgenix 4.152.1, respectively. In other embodiments, the antibodies are those encoded by the plasmids shown in **Table 1**. In still other particular embodiments, the antibodies are those that comprise a heavy chain encoded by a nucleic acid molecule comprising the heavy chain coding region or regions of a nucleotide sequence selected from the group consisting of nucleotide sequences set forth as SEQ ID NOs: 2-7, and a light chain encoded by a nucleic acid molecule comprising the light chain coding region or regions of a nucleotide sequence selected from the group consisting of nucleotide sequences set forth as SEQ ID NOs: 8-13.

As used herein, the names of the deposited hybridomas or plasmids may be used interchangeably with the names of the antibodies. It would be clear to one of ordinary skill in the art when the name is intended to refer to the antibody or when it refers to the plasmids or hybridomas that encode or produce the antibodies, respectively. Additionally, the antibody names may be an abbreviated form of the name shown in **Table 1**. For instance, antibody AB-PG1-XG1-006 may be referred to as AB-PG1-XG1-006, PG1-XG1-006, X1-006, 006, etc. In another example, the antibody name PSMA 4.232.3 may be referred to as PSMA 4.232.1, 4.232.3, 4.232.1, 4.232, etc. It is intended that all of the variations in the name of the antibody refer to the same antibody and not a different one.

The antibodies of the ADCs, or from which the antigen-binding fragments of the ADCs are derived, include those encoded by particular sets of heavy and light chain sequences. In one embodiment, the antibody (AB-PG1-XG1-006) is encoded by a nucleic acid molecule which comprises a coding region or regions of the nucleic acid sequences set forth as SEQ ID NOs: 2 and 8. In another embodiment, the antibody (AB-PG1-XG1-026) is encoded by a nucleic acid molecule which comprises a coding region or regions of the nucleic acid sequences set forth as SEQ ID NOs: 3 and 9. In still another embodiment, the antibody (AB-PG1-XG1-051) is encoded by a nucleic acid molecule which comprises a coding region or regions of the nucleic acid sequences set forth as SEQ ID NOs: 4 and 10. In yet another embodiment, the antibody (AB-PG1-XG1-069) is encoded by a nucleic acid molecule which comprises a coding region or regions of the nucleic acid sequences set forth as SEQ ID NOs: 5 and 11. In another embodiment, the antibody (AB-PG1-XG1-077) is encoded by a nucleic acid molecule which comprises a coding region or regions of the nucleic acid sequences set forth as SEQ ID NOs: 6 and 12. In yet another embodiment, the antibody (PSMA 10.3) is encoded by a nucleic acid molecule which comprises a coding region or regions of the nucleic acid sequences set forth as SEQ ID NOs: 7 and 13. In other embodiments, the antibodies of the ADCs, or from which the antigen-binding fragments of the ADCs are derived, include a heavy chain variable region encoded by a nucleic acid molecule comprising a coding region or regions of a nucleotide sequence selected from the group consisting of nucleotide sequences set forth as SEQ ID NOs: 14, 18, 22, 26 and 30, and a light chain variable region encoded by a nucleic acid molecule comprising a coding region or regions of a nucleotide sequence selected from the group consisting of nucleotide sequences set forth as SEQ ID NOs: 16, 20, 24, 28 and 32. In one embodiment, the antibody (AB-PG1-XG1-006) includes an immunoglobulin variable sequence encoded by nucleic acid molecules which comprise a coding region or regions of the nucleic acid sequences set forth as SEQ ID NOs: 14 and 16. Likewise, the antibody can be one that includes an immunoglobulin variable sequence which comprises the amino acid sequences set forth as SEQ ID NOs: 15 and 17. In another embodiment, the antibody (AB-PG1-XG1-026) includes an immunoglobulin variable sequence encoded by nucleic acid molecules comprising a coding region or regions of nucleotide sequences set forth as SEQ ID NOs: 18 and 20 or includes an immunoglobulin variable sequence which comprises the amino acid sequences set forth as SEQ ID NOs 19 and 21. In still another embodiment, the antibody (AB-PG1-XG1-051) includes an immunoglobulin variable sequence encoded by the nucleic acid molecules comprising a coding region or regions of nucleotide sequences set forth as SEQ ID NOs: 22 and 24 or includes an immunoglobulin variable sequence which comprises the amino acid sequences set forth as SEQ ID NOs: 23 and 25. In yet another embodiment, the antibody (AB-PG1-XG1-069) includes an immunoglobulin variable sequence encoded by the nucleic acid molecules comprising a coding region or regions of nucleotide sequences set forth as SEQ ID NOs: 26 and 28 or includes an immunoglobulin variable sequence which comprises the amino acid sequences set forth as SEQ ID NOs: 27 and 29. In another embodiment, the antibody (AB-PG1-XG1-077) includes an immunoglobulin variable sequence encoded by the nucleic acid molecules comprising a coding region or regions of nucleotide sequences set forth as SEQ ID NOs: 30 and 32 or includes an immunoglobulin variable sequence which comprises the amino acid sequences set forth as SEQ ID NOs: 31 and 33. In other embodiments, the antibody includes a heavy chain variable region comprising an amino acid sequence selected from the group consisting of amino acid sequences set forth as: SEQ ID NOs: 15, 19, 23, 27 and 31, and a light chain variable region comprising an amino acid sequence selected from the group consisting of amino acid sequences set forth as: SEQ ID NOs: 17, 21, 25, 29 and 33.

As used herein, a "coding region" refers to a region of a nucleotide sequence that encodes a polypeptide sequence. Its use herein is consistent with the recognized meaning known in the art.

In certain embodiments, the antibodies of the ADCs, or from which the antigen-binding fragments of the ADCs are derived, are those that are encoded by nucleic acid molecules that are highly homologous to the foregoing nucleic acids. The homologous nucleic acid molecule can, in some embodiments, comprise a nucleotide sequence that is at least about 90% identical to the nucleotide sequence provided herein. In other embodiments, the nucleotide sequence is at least about 95% identical, at least about 97% identical, at least about 98% identical, or at least about 99% identical to a nucleotide sequence provided herein. The homology can be calculated using various, publicly available software tools well know to one of ordinary skill in the art. Exemplary tools include the BLAST system available from the website of the National Center for Biotechnology Information (NCBI) at the National Institutes of Health.

One method of identifying highly homologous nucleotide sequences is via nucleic acid hybridization. Thus, the invention also includes antibodies having the PSMA-binding properties and other functional properties described herein, which are encoded by nucleic acid molecules that hybridize under high stringency conditions to the foregoing nucleic acid molecules. Identification of related sequences can also be achieved using polymerase chain reaction (PCR) and other amplification techniques suitable for cloning related nucleic acid sequences. PCR primers can be selected to amplify portions of a nucleic acid sequence of interest, such as a CDR.

The term "high stringency conditions", as used herein, refers to parameters with which the art is familiar. Nucleic acid hybridization parameters may be found in references that compile such methods, e.g. Molecular Cloning: A Laboratory Manual, J. Sambrook, et al., eds., Second Edition, Cold Spring Harbor Laboratory Press, Cold Spring Harbor, New York, 1989, or Current Protocols in Molecular Biology, F.M. Ausubel, et al., eds., John Wiley & Sons, Inc., New York. One example of high-stringency conditions is hybridization at 65°C in hybridization buffer (3.5X SSC, 0.02% Ficoll, 0.02% polyvinyl pyrrolidone, 0.02% Bovine Serum Albumin, 2.5mM NaH₂PO₄(pH7), 0.5% SDS, 2mM EDTA). SSC is 0.15M sodium chloride/0.015M sodium citrate, pH7; SDS is sodium dodecyl sulphate; and EDTA is ethylenediaminetetracetic acid. After hybridization, a membrane upon which the nucleic acid is transferred is washed, for example, in 2X SSC at room temperature and then at 0.1 - 0.5X SSC/0.1X SDS at temperatures up to 68°C.

As used herein, the term "antibody" refers to a glycoprotein comprising at least two heavy (H) chains and two light (L) chains inter-connected by disulfide bonds. Each heavy chain is comprised of a heavy chain variable region (abbreviated herein as HCVR or V_{H}) and a heavy chain constant region. The heavy chain constant region is comprised of three domains, C_{H}1 , C_{H}2 and C_{H}3. Each light chain is comprised of a light chain variable region (abbreviated herein as LCVR or V_{L}) and a light chain constant region. The light chain constant region is comprised of one domain, CL. The V_{H} and V_{L} regions can be further subdivided into regions of hypervariability, termed complementarity determining regions (CDR), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

The term "antigen-binding fragment" of an antibody as used herein, refers to one or more portions of an antibody that retain the ability to specifically bind to an antigen (i.e., PSMA). It has been shown that the antigen-binding function of an antibody can be performed by fragments of a full-length antibody. Examples of binding fragments encompassed within the term "antigen-binding fragment" of an antibody include (i) a Fab fragment, a monovalent fragment consisting of the V_{L}, V_{H}, C_{L} and C_{H}1 domains; (ii) a F(ab')₂ fragment, a bivalent fragment comprising two Fab fragments linked by a disulfide bridge at the hinge region; (iii) a Fd fragment consisting of the V_{H} and CH1 domains; (iv) a Fv fragment consisting of the V_{L} and V_{H} domains of a single arm of an antibody, (v) a dAb fragment (Ward et al., (1989) Nature 341:544-546) which consists of a V_{H} domain; and (vi) an isolated complementarity determining region (CDR). The CDRs, and in particular the CDR3 regions, and more particularly the heavy chain CDR3 contribute to antibody specificity. Because these CDR regions and in particular the CDR3 region confer antigen specificity on the antibody these regions may be incorporated into other antibodies or antigen-binding fragments to confer the identical antigen specificity onto that antibody or peptide. Furthermore, although the two domains of the Fv fragment, V and V_{H}, are coded for by separate genes, they can be joined, using recombinant methods, by a synthetic linker that enables them to be made as a single protein chain in which the V_{L} and V_{H} regions pair to form monovalent molecules (known as single chain Fv (scFv); see e.g., Bird et al. (1988) Science 242:423-426; and Huston et al. (1988) Proc. Natl. Acad. Sci. USA 85:5879-5883). Such single chain antibodies are also intended to be encompassed within the term "antigen-binding fragment" of an antibody. These antibody fragments are obtained using conventional procedures, such as proteolytic fragmentation procedures, as described in J. Goding, Monoclonal Antibodies: Principles and Practice, pp 98-118 (N.Y. Academic Press 1983), as well as by other techniques known to those with skill in the art. The fragments are screened for utility in the same manner as are intact antibodies.

The antibodies, or antigen-binding fragments thereof, of the ADCs are, in some embodiments, isolated. "Isolated", as used herein, is intended to refer to an antibody (or antigen-binding fragment thereof), which is substantially free of other antibodies (or antigen-binding fragments) having different antigenic specificities (e.g., an isolated antibody that specifically binds to PSMA is substantially free of antibodies that specifically bind antigens other than PSMA). An isolated antibody that specifically binds to an epitope, isoform or variant of PSMA may, however, have cross-reactivity to other related antigens, e.g., from other species (e.g., PSMA species homologs). Moreover, an isolated antibody (or antigen-binding fragment thereof) may be substantially free of other cellular material and/or chemicals. As used herein, "specific binding" refers to antibody binding to a predetermined antigen, in this case PSMA. Typically, the antibody binds with an affinity that is at least twofold greater than its affinity for binding to a non-specific antigen (e.g., BSA, casein), which is an antigen other than PSMA, an isoform or variant of PSMA, or a closely-related antigen.

The antibodies encompass various antibody isotypes, such as IgG1, IgG2, IgG3, IgG4, IgM, IgA1, IgA2, IgAsec, IgD, IgE. As used herein, "isotype" refers to the antibody class (e.g., IgM or IgG1) that is encoded by heavy chain constant region genes. The antibodies can be full length or can include only an antigen-binding fragment such as the antibody constant and/or variable domain of IgG1, IgG2, IgG3, IgG4, IgM, IgA1, IgA2, IgAsec, IgD or IgE or could consist of a Fab fragment, a F(ab')₂ fragment and a Fv fragment.

The antibodies of the ADCs, or from which the antigen-binding fragments of the ADCs are derived, are, in some embodiments monoclonal. The antibodies can be produced by a variety of techniques well know in the art. Monoclonal antibody production may be effected by techniques which are well known in the art. The term "monoclonal antibody", as used herein, refers to a preparation of antibody molecules of single molecular composition. A monoclonal antibody displays a single binding specificity and affinity for a particular epitope. The process of monoclonal antibody production involves obtaining immune somatic cells with the potential for producing antibody, in particular B lymphocytes, which have been previously immunized with the antigen of interest either *in vivo* or *in vitro* and that are suitable for fusion with a B-cell myeloma line.

Mammalian lymphocytes typically are immunized by *in vivo* immunization of the animal (e.g., a mouse) with the desired protein or polypeptide. Such immunizations are repeated as necessary at intervals of up to several weeks to obtain a sufficient titer of antibodies. Once immunized, animals can be used as a source of antibody-producing lymphocytes. Following the last antigen boost, the animals are sacrificed and spleen cells removed. Mouse lymphocytes give a higher percentage of stable fusions with the mouse myeloma lines described herein. For example, of the BALB/c mouse. However, other mouse strains, rabbit, hamster, sheep and frog may also be used as hosts for preparing antibody-producing cells. See; Goding (in Monoclonal Antibodies: Principles and Practice, 2d ed., pp. 60-61, Orlando, Fla., Academic Press, 1986). In particular, mouse strains that have human immunoglobulin genes inserted in the genome (and which cannot produce mouse immunoglobulins) can be used. Examples include the HuMAb mouse strains produced by Medarex/GenPharm International, and the XenoMouse strains produced by Abgenix. Such mice produce fully human immunoglobulin molecules in response to immunization. In some embodiments, therefore, the ADCs comprise a fully human monoclonal antibody or an antigen-binding fragment thereof that binds PSMA.

Those antibody-producing cells that are in the dividing plasmablast stage fuse preferentially. Somatic cells may be obtained from the lymph nodes, spleens and peripheral blood of antigen-primed animals, and the lymphatic cells of choice depend to a large extent on their empirical usefulness in the particular fusion system. The antibody-secreting lymphocytes are then fused with (mouse) B cell myeloma cells or transformed cells, which are capable of replicating indefinitely in cell culture, thereby producing an immortal, immunoglobulin-secreting cell line. The resulting fused cells, or hybridomas, are cultured, and the resulting colonies screened for the production of the desired monoclonal antibodies. Colonies producing such antibodies are cloned, and grown either *in vivo* or *in vitro* to produce large quantities of antibody. A description of the theoretical basis and practical methodology of fusing such cells is set forth in Kohler and Milstein, Nature 256:495 (1975).

Alternatively, human somatic cells capable of producing antibody, specifically B lymphocytes, are suitable for fusion with myeloma cell lines. While B lymphocytes from biopsied spleens, tonsils or lymph nodes of an individual may be used, the more easily accessible peripheral blood B lymphocytes can also be used. The lymphocytes may be derived from patients with diagnosed prostate carcinomas or another PSMA-expressing cancer. In addition, human B cells may be directly immortalized by the Epstein-Barr virus (Cole et al., 1995, Monoclonal Antibodies and Cancer Therapy, Alan R. Liss, Inc., pp. 77-96). Although somatic cell hybridization procedures can be used, in principle, other techniques for producing monoclonal antibodies can be employed such as viral or oncogenic transformation of B lymphocytes.

Myeloma cell lines suited for use in hybridoma-producing fusion procedures can be non-antibody-producing, have high fusion efficiency, and enzyme deficiencies that render them incapable of growing in certain selective media which support the growth of the desired hybridomas. Examples of such myeloma cell lines that may be used for the production of fused cell lines include P3-X63/Ag8, X63-Ag8.653, NS1/1.Ag 4.1, Sp2/0-Ag14, FO, NSO/U, MPC-11, MPC11-X45-GTG 1.7, S194/5XX0 Bul, all derived from mice; R210.RCY3, Y3-Ag 1.2.3, IR983F and 4B210 derived from rats and U-266, GM1500-GRG2, LICR-LON-HMy2, UC729-6, all derived from humans (Goding, in Monoclonal Antibodies: Principles and Practice, 2d ed., pp. 65-66, Orlando, Fla., Academic Press, 1986; Campbell, in Monoclonal Antibody Technology, Laboratory Techniques in Biochemistry and Molecular Biology Vol. 13, Burden and Von Knippenberg, eds. pp. 75-83, Amsterdam, Elseview, 1984).

Fusion with mammalian myeloma cells or other fusion partners capable of replicating indefinitely in cell culture is effected by standard and well-known techniques, for example, by using polyethylene glycol ("PEG") or other fusing agents (See Milstein and Kohler, Eur: J. Immunol. 6:511 (1976)).

In other embodiments, the antibodies of the ADCs, or from which the antigen-binding fragments of the ADCs are derived, are recombinant antibodies. The term "recombinant antibody", as used herein, is intended to include antibodies that are prepared, expressed, created or isolated by recombinant means, such as antibodies isolated from an animal (e.g., a mouse) that is transgenic for another species' immunoglobulin genes, antibodies expressed using a recombinant expression vector transfected into a host cell, antibodies isolated from a recombinant, combinatorial antibody library, or antibodies prepared, expressed, created or isolated by any other means that involves splicing of immunoglobulin gene sequences to other DNA sequences.

In yet other embodiments, the antibodies are chimeric or humanized antibodies. As used herein, the term "chimeric antibody" refers to an antibody, that combines the murine variable or hypervariable regions with the human constant region or constant and variable framework regions. As used herein, the term "humanized antibody" refers to an antibody that retains only the antigen-binding CDRs from the parent antibody in association with human framework regions (see, Waldmann, 1991, Science 252:1657). Such chimeric or humanized antibodies retaining binding specificity of the murine antibody are expected to have reduced immunogenicity when administered *in vivo* for applications according to the invention.

According to an alternative embodiment, the monoclonal antibodies of the present invention can be modified to be in the form of a bispecific antibody, or a multispecific antibody. The term "bispecific antibody" is intended to include any agent, e.g., a protein, peptide, or protein or peptide complex, which has two different binding specificities which bind to, or interact with (a) a cell surface antigen and (b) an Fc receptor on the surface of an effector cell. The term "multispecific antibody" is intended to include any agent, e.g., a protein, peptide, or protein or peptide complex, which has more than two different binding specificities which bind to, or interact with (a) a cell surface antigen, (b) an Fc receptor on the surface of an effector cell, and (c) at least one other component. Accordingly, the antibodies include, but are not limited to, bispecific, trispecific, tetraspecific, and other multispecific antibodies which are directed to PSMA and to Fc receptors on effector cells. The term "bispecific antibodies" further includes diabodies. Diabodies are bivalent, bispecific antibodies in which the V_{H} and V_{L} domains are expressed on a single polypeptide chain, but using a linker that is too short to allow for pairing between the two domains on the same chain, thereby forcing the domains to pair with complementary domains of another chain and creating two antigen-binding sites (see e.g., Holliger, P., et al. (1993) Proc. Natl. Acad Sci. USA 90:6444-6448; Poijak, R.J., et al. (1994) Structure 2:1121-1123).

A bispecific antibody can be formed of an antigen-binding region specific for PSMA and an antigen-binding region specific for an effector cell which has tumoricidal or tumor inhibitory activity. The two antigen-binding regions of the bispecific antibody are either chemically linked or can be expressed by a cell genetically engineered to produce the bispecific antibody. (See generally, Fanger et al., 1995 Drug News & Perspec. 8(3):133-137). Suitable effector cells having tumoricidal activity include but are not limited to cytotoxic T-cells (primarily CD8⁺ cells), natural killer cells, etc. An effective amount of a bispecific antibody according to the invention can be administered to a subject with cancer and the bispecific antibody kills and/or inhibits proliferation of the cancer cells after localization at sites of primary or metastatic tumors bearing PSMA.

In certain embodiments, the antibodies of the ADCs, or from which the antigen-binding fragments of the ADCs are derived, are human antibodies. The term "human antibody", as used herein, is intended to include antibodies having variable and constant regions derived from human germline immunoglobulin sequences. The human antibodies of the invention can include amino acid residues not encoded by human germline immunoglobulin sequences (e.g., mutations introduced by random or site-specific mutagenesis *in vitro* or by somatic mutation *in vivo*). However, the term "human antibody", as used herein, is not intended to include antibodies in which CDR sequences derived from the germline of another mammalian species, such as a mouse have been grafted onto human framework sequences (referred to herein as "humanized antibodies"). Human antibodies directed against PSMA can be generated using transgenic mice carrying parts of the human immune system rather than the mouse system. Some examples of which were described above.

Fully human monoclonal antibodies also can be prepared by immunizing mice transgenic for large portions of human immunoglobulin heavy and light chain loci. See, e.g., U.S. patents 5,591,669, 5,598,369, 5,545,806, 5,545,807, 6,150,584, and references cited therein. These animals have been genetically modified such that there is a functional deletion in the production of endogenous (e.g., murine) antibodies. The animals are further modified to contain all or a portion of the human germ-line immunoglobulin gene locus such that immunization of these animals results in the production of fully human antibodies to the antigen of interest. Following immunization of these mice (e.g., XenoMouse (Abgenix), HuMAb mice (Medarex/GenPharm)), monoclonal antibodies are prepared according to standard hybridoma technology. These monoclonal antibodies have human immunoglobulin amino acid sequences and therefore will not provoke human anti-mouse antibody (HAMA) responses when administered to humans. In general, but not intended to be limiting, the mice are 6-16 weeks of age upon the first immunization. For example, a purified or enriched preparation of PSMA antigen (e.g., recombinant PSMA or PSMA-expressing cells) is used to immunize the mice intraperitoneally (IP), although other routes of immunization known to one of ordinary skill in the art are also possible. PSMA antigen is injected in combination with an adjuvant, such as complete Freund's adjuvant, and, in some embodiments, the initial injection is followed by booster immunizations with antigen in an adjuvant, such as incomplete Freund's adjuvant. The immune response is monitored over the course of the immunization protocol with plasma samples obtained by, for example, retroorbital bleeds. The plasma is screened by ELISA, and mice with sufficient titers of anti-PSMA human immunoglobulin are used for fusions. Mice are boosted intravenously with antigen 3 days before sacrifice and removal of the spleen.

The antibody or antigen-binding fragment thereof of the ADCs can, in some embodiments, be selected for the ability to bind live PSMA-expressing cells. In order to demonstrate binding to live PSMA-expressing cells, flow cytometry can be used. For example, PSMA-expressing cells lines (grown under standard growth conditions) or prostate cancer cells that express PSMA are mixed with various concentrations of monoclonal antibodies in PBS containing 0.1% Tween 80 and 20% mouse serum, and incubated at 37°C for 1 hour. After washing, the cells are reacted with fluorescein-labeled anti-human IgG secondary antibody (if human anti-PSMA antibodies were used) under the same conditions as the primary antibody staining. The samples can be analyzed by a fluorescence activated cell sorter (FACS) instrument using light and side scatter properties to gate on single cells. An alternative assay using fluorescence microscopy can be used (in addition to or instead of) the flow cytometry assay. Cells can be stained and examined by fluorescence microscopy. This method allows visualization of individual cells, but may have diminished sensitivity depending on the density of the antigen. It follows, that the ADCs, in some embodiments, bind live cells. The ADCs, in some embodiments, therefore, do not require cell lysis to bind PSMA.

The antibodies can, in some embodiments, promote cytolysis of PSMA-expressing cells. Cytolysis can be complement-mediated or can be mediated by effector cells. In one embodiment, the cytolysis is carried out in a living organism, such as a mammal, and the live cell is a tumor cell. Examples of tumors which can be targeted with the antibodies or antigen-binding fragments thereof include, any tumor that expresses PSMA (this includes tumors with neovascualture expressing PSMA), such as, prostate, bladder, pancreas, lung, colon, kidney, melanomas and sarcomas. In one embodiment, the tumor cell is a prostate cancer cell.

The testing of cytolytic activity *in vitro* by chromium release assay can provide an initial screening prior to testing *in vivo* models. This testing can be carried out using standard chromium release assays. Briefly, polymorphonuclear cells (PMN), or other effector cells, from healthy donors can be purified by Ficoll Hypaque density centrifugation, followed by lysis of contaminating erythrocytes. Washed PMNs can be suspended in RPMI supplemented with 10% heat-inactivated fetal calf serum and mixed with ⁵¹Cr labeled cells expressing PSMA, at various ratios of effector cells to tumor cells (effector cells:tumor cells). Purified anti-PSMA IgGs can then be added at various concentrations. Irrelevant IgG can be used as a negative control. Assays can be carried out for 0-120 minutes at 37°C. Samples can be assayed for cytolysis by measuring ⁵¹Cr release into the culture supernatant. Anti-PSMA monoclonal antibodies and/or ADCs can also be tested in combinations with each other to determine whether cytolysis is enhanced with multiple monoclonal antibodies and/or ADCs. Antibodies that bind to PSMA and/or ADCs also can be tested in an *in vivo* model (e.g., in mice) to determine their efficacy in mediating cytolysis and killing of cells expressing PSMA, e.g., tumor cells.

The antibodies of the ADCs, or from which the antigen-binding fragments of the ADCs are derived, can be selected, for example, based on the following criteria, which are not intended to be exclusive:
1) binding to live cells expressing PSMA;
2) high affinity of binding to PSMA;
3) binding to a unique epitope on PSMA (i.e., an epitope not recognized by a previously produced antibody);
4) opsonization of cells expressing PSMA;
5) mediation of growth inhibition, phagocytosis and/or killing of cells expressing PSMA in the presence of effector cells;
6) modulation (inhibition or enhancement) of NAALADase, folate hydrolase, dipeptidyl peptidase IV and/or γ-glutamyl hydrolase activities;
7) growth inhibition, cell cycle arrest and/or cytotoxicity in the absence of effector cells;
8) internalization of PSMA;
9) binding to a conformational epitope on PSMA;
10) minimal cross-reactivity with cells or tissues that do not express PSMA; and
11) preferential binding to dimeric forms of PSMA rather than monomeric forms of PSMA.
The antibodies can meet one or more, and possibly all, of these criteria.

In one embodiment, the antibody or antigen-binding fragment thereof binds to a conformational epitope, such as a conformational epitope within the extracellular domain of PSMA. To determine if an anti-PSMA antibody or antigen-binding fragment thereof binds to conformational epitopes, each antibody can be tested in assays using native protein (e.g., non-denaturing immunoprecipitation, flow cytometric analysis of cell surface binding) and denatured protein (e.g., Western blot, immunoprecipitation of denatured proteins). A comparison of the results will indicate whether the antibody or antigen-binding fragment thereof binds a conformational epitope. Antibodies or antigen-binding fragments thereof that bind to native protein but not denatured protein are, in some embodiments, those that bind conformational epitopes. It follows, that the ADCs, in some embodiments, bind comformational epitopes of PSMA.

In another embodiment, the antibody or antigen-binding fragment thereof binds to a dimer-specific epitope on PSMA. Generally, antibodies or antigen-binding fragments thereof which bind to a dimer-specific epitope preferentially bind the PSMA dimer rather than the PSMA monomer. To determine if an antibody or antigen-binding fragment thereof binds preferentially (i.e., selectively and/or specifically) to a PSMA dimer, the antibody or antigen-binding fragment thereof can be tested in assays (e.g., immunoprecipitation followed by Western blotting) using native dimeric PSMA protein and dissociated monomeric PSMA protein. A comparison of the results will indicate whether the antibody or antigen-binding fragment thereof binds preferentially to the dimer. In some embodiments, the antibodies or antigen-binding fragments thereof bind to the PSMA dimer but not to the monomeric PSMA protein. It follows, that the ADCs, in some embodiments, bind to a dimer-specific epitope on PSMA.

The invention, therefore, also includes ADCs that selectively bind PSMA multimers. As used herein, particularly with respect to the binding of PSMA multimers by the ADCs, "selectively binds" means that an antibody preferentially binds to a PSMA protein multimer (e.g., with greater avidity, greater binding affinity) rather than to a PSMA protein monomer. In some embodiments, the ADCs of the invention bind to a PSMA protein multimer with an avidity and/or binding affinity that is 1.1-fold, 1.2-fold, 1.3-fold, 1.4-folk, 1.5-fold, 1.6-fold, 1.7-fold, 1.8-fold, 1.9-fold, 2-fold, 3-fold, 4-fold, 5-fold, 7-fold, 10-fold, 20-fold, 30-fold, 40-fold, 50-fold, 70-fold, 100-fold, 200-fold, 300-fold, 500-fold, 1000-fold or more than that exhibited by the ADC for a PSMA protein monomer. The ADC can, in some embodiments, selectively bind a PSMA protein multimer, and not a PSMA protein monomer, i.e., exclusively binds to a PSMA protein multimer. In some embodiments, the ADC selectively binds a PSMA protein dimer.

A PSMA protein multimer, as used herein, is a protein complex of at least two PSMA proteins or fragments thereof. The PSMA protein multimers can be composed of various combinations of full-length PSMA proteins (e.g., SEQ ID NO: 1), recombinant soluble PSMA (rsPSMA, e.g., amino acids 44-750 of SEQ ID NO: 1) and fragments of the foregoing that form multimers (i.e., that retain the protein domain required for forming dimers and/or higher order multimers of PSMA). In some embodiments, at least one of the PSMA proteins forming the multimer is a recombinant, soluble PSMA (rsPSMA) polypeptide. The PSMA protein multimers can be dimers, such as those formed from recombinant soluble PSMA protein. In one embodiment, the dimer is a rsPSMA homodimer. The PSMA protein multimers referred to herein are believed to assume a native conformation and can have such a conformation. The PSMA proteins in certain embodiments are noncovalently bound together to form the PSMA protein multimer. For example, it has been discovered that PSMA protein noncovalently associates to form dimers under non-denaturing conditions. The PSMA protein multimers can retain the activities of PSMA. The PSMA activity may be an enzymatic activity, such as folate hydrolase activity, NAALADase activity, dipeptidyl peptidase IV activity or γ-glutamyl hydrolase activity. Methods for testing the PSMA activity of multimers are well known in the art (reviewed by O'Keefe et al. in: Prostate Cancer: Biology, Genetics, and the New Therapeutics, L.W.K. Chung, W.B. Isaacs and J.W. Simons (eds.) Humana Press, Totowa, NJ, 2000, pp. 307-326).

The antibody or antigen-binding fragment thereof of the ADCs can bind to and is internalized with PSMA expressed on cells. The mechanism by which the antibody or antigen-binding fragment thereof is internalized with PSMA is not critical to the practice of the present invention. For example, the antibody or antigen-binding fragment thereof can induce internalization of PSMA. Alternatively, internalization of the antibody or antigen-binding fragment thereof can be the result of routine internalization of PSMA. It follows that the ADC can be internalized with PSMA expressed on cells.

The antibodies or antigen-binding fragments thereof, and therefore the ADCs of the invention, can specifically bind cell-surface PSMA and/or rsPSMA with sub-nanomolar affinity. The binding affinities can be about 1 X 10⁻⁹M or less, about 1 X 10⁻¹⁰M or less, or about 1 X 10⁻¹M or less. In a particular embodiment the binding affinity is less than about 5 X 10⁻¹⁰M.

The antibodies or antigen-binding fragments thereof can, in some embodiments, modulate at least one enzymatic activity of PSMA. The activity can be selected from the group consisting of N-acetylated α-linked acidic dipeptidase (NAALADase), folate hydrolase, dipeptidyl dipeptidase IV, γ-glutamyl hydrolase activity and combinations thereof *in vitro* or *in vivo*. The modulation may be enhancement or inhibition of at least one enzymatic activity of PSMA.

Tissue levels of NAALADase can be determined by detergent solubilizing homogenizing tissues, pelleting the insoluble material by centrifugation and measuring the NAALADase activity in the remaining supernatant. Likewise, the NAALADase activity in bodily fluids can also be measured by first pelleting the cellular material by centrifugation and performing a typical enzyme assay for NAALADase activity on the supernatant. NAALADase enzyme assays have been described by Frieden, 1959, J. Biol, Chem., 234:2891. In this assay, the reaction product of the NAALADase enzyme is glutamic acid. This is derived from the enzyme catalyzed cleavage of N-acetylaspartylglutamate to yield N-acetylaspartic acid and glutamic acid. Glutamic acid, in a NAD(P)⁺ requiring step, yields 2-oxoglutarate plus NAD(P)H in a reaction catalyzed by glutamate dehydrogenase. Progress of the reaction can easily and conveniently be measured by the change in absorbance at 340 nm due to the conversion of NAD(P)⁺ to NAD(P)H.

Folate hydrolase activity of PSMA can be measured by performing enzyme assays as described by Heston and others (e.g., Clin. Cancer Res. 2(9):1445-51, 1996; Urology 49(3A Suppl):104-12,1997). Folate hydrolases such as PSMA remove the gamma-linked glutamates from polyglutamated folates. Folate hydrolase activity can be measured using substrates such as methotrexate tri-gamma glutamate (MTXGlu3), methotrexate di-gamma glutamate (MTXGlu2) or pteroylpentaglutamate (PteGlu5), for example using capillary electrophoresis (see Clin. Cancer Res. 2(9):1445-51, 1996). Timed incubations of PSMA with polyglutamated substrates is followed by separation and detection of hydrolysis products.

An ADC of the invention comprises an antibody or antigen-binding fragment thereof conjugated to MMAE or MMAF. The antibody or antigen-binding fragment thereof can be, in some embodiments, conjugated to MMAE or MMAF with a compound of the following formula (**Formula 1**): -Aₙ-Yₘ-Zₘ-Xₙ-Wₙ-, wherein A is a carboxylic acyl unit; Y is an amino acid; Z is an amino acid; X and W are each a self-immolative spacer; n is an integer of 0 or 1; and m is an integer of 0 or 1, 2, 3, 4, 5 or 6. A conjugate of the present invention, in some embodiments, is represented by the formula (**Formula 2**): L-{Aₙ-Yₘ-Zₘ-Xₙ-Wₙ-D}ₚ wherein L is an antibody or antigen-binding fragment thereof that binds PSMA, D is MMAE or MMAF and p is an integer of 1, 2, 3, 4, 5, 6, 7 or 8. The other components are as described above. In one embodiment, the carboxylic unit "Aₙ" is linked to the antibody or antigen-binding fragment via a sulfur atom derived from the antibody or antigen-binding fragment:

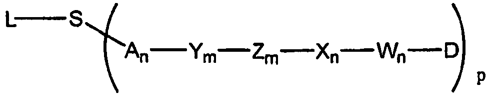

In one embodiment, A is 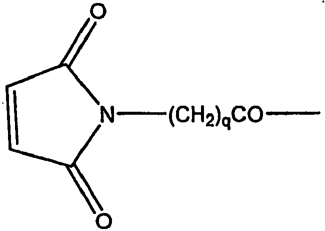 in which q is 1-10. Therefore, in one embodiment, the conjugate is: 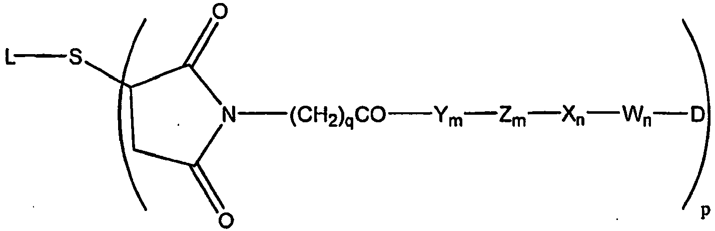 wherein L, Y, Z, X, W, D, n, m, q and p are as previously defined.

In another embodiment, A is 4-(N-succinimidomethyl)cyclohexane-1-carbonyl, m-succinimidobenzoyl, 4-(p-succinimidophenyl) -butyryl, 4-(2-acetamido)benzoyl, 3-thiopropionyl, 4-(1-thioethyl)-benzoyl, 6-(3-thiopropionylamido)-hexanoyl or maleimide caproyl. In a further embodiment, A is maleimide caproyl. Representative examples of various carboxylic acyl units and methods for their synthesis and attachment are described in US Pat. No. 6,214,345, the entire contents of which are herein incorporated by reference.

In another embodiment, Y is alanine, valine, leucine, isoleucine, methionine, phenylalanine, tryptophan or proline. In yet another embodiment, Y is valine. In a further embodiment, Z is lysine, lysine protected with acetyl or formyl, arginine, arginine protected with tosyl or nitro groups, histidine, ornithine, ornithine protected with acetyl or formyl, or citrulline. In still a further embodiment, Z is citrulline. In one embodiment Yₘ-Zₘ is valine-citrulline. In another embodiment, Yₘ-Zₘ is a protein sequence which is selectively cleavable by a protease.

In a further embodiment, X is a compound having the formula 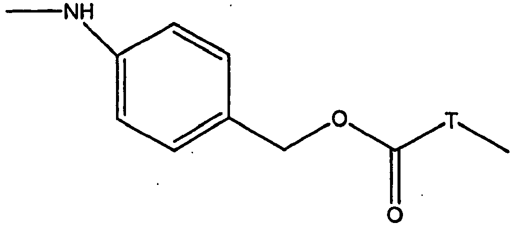 in which T is O, N, or S. In another embodiment, X is a compound having the formula -HN-R¹ -COT in which R¹ is C₁ -C₅ alkyl, T is O, N or S. In a further embodiment, X is a compound having the formula 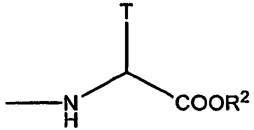 in which T is O, N, or S, R² is H or C₁ -C₅ alkyl. In one embodiment, X is p-aminobenzylcarbamoyloxy. In another embodiment, X is p-aminobenzylalcohol. In a further embodiment, X is p-aminobenzylcarbamate. In yet a further embodiment, X is p-aminobenzyloxycarbonyl. In another embodiment, X is γ-aminobutyric acid; α,α-dimethyl γ-aminobutyric acid or β,β-dimethyl γ-aminobutyric acid.

In some embodiments, W is 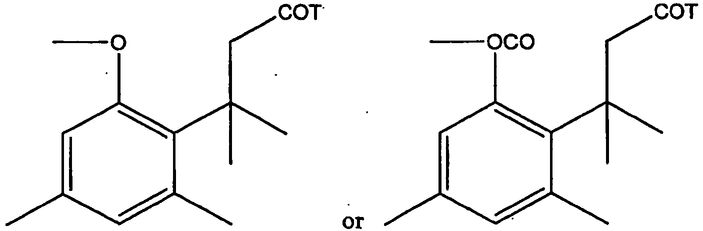 in which T is O, S or N.

In one embodiment, the compound of **Formula 1** is maleimidocaproyl. Maleimidocaproyl has been used for conjugation of two specific auristatins to an anti-CD30 mAb (AC10) (Doronina, Svetlana et al. "Novel Linkers for Monoclonal Antibody-Mediated Delivery of Anticancer Agents", AACR, Anaheim, CA, Abstract No. 1421, April 16-20, 2005). Maleimidocaproyl reacts with thiol groups to form a thioether.

MMAE or MMAF can be conjugated to an antibody or antigen-binding fragment thereof using methods known to those of ordinary skill in the art (e.g., See, Niemeyer, CM, Bioconjugation Protocols, Strategies and Methods, Humana Press, 2004) or as described herein. 3 or 4 MMAE or MMAF molecule are conjugated to the antibody or antigen-binding fragment thereof.

The ADCs of the invention have been found to have particularly high levels of selectivity when killing of non-PSMA-expressing cells is compared to killing of PSMA-expressing cells. Therefore, the ADCs have a PC-3™ cell to C4-2 cell or LNCaP™ cell selectivity of at least 250. In embodiments, the selectivity is at least 300, 350, 400, 450, 500, 600, 700, 800, 900, 1000,1100, 1200, 1300, 1400, 1500, 1600, 1700, 1800, 1900, 2000, 2250, 2500, 2750, 3000, 3500, 4000, 4500, 5000, 5500, 6000, 6500, 7000, 7500, 8000, 8500, 9000, 9500, 10000, 11000, 12000, 13000, 14000, 15000, 17500, 20000 or more. In some embodiments, the selectivity is between 250-500, 500-750, 750-1000, 1000-2000, 2000-5000, 5000-10000, 10000-15000 or 15000-20000. "Selectivity", as defined herein, refers to the ratio of IC₅₀ values of an ADC on PC-3™ cells (non-PSMA-expressing cells) to C4-2 cells or LNCaP™ cells (PSMA-expressing cells).

It has also been found that the ADCs of the invention mediate, in some embodiments, PSMA-expressing specific cell killing at very low concentrations, such as at or near picomolar concentrations. The ADCs, in some embodiments, exhibit IC₅₀s at concentrations of less than about 1 X 10⁻¹⁰M, less than about 1 X 10⁻¹¹M, or less than about 1 X 10⁻¹²M. In a particular embodiment, an IC₅₀ is achieved at a concentration of less than about 1.5 X 10⁻¹¹M. In another embodiment, the ADCs provided exhibit IC₅₀s of between 10-210, 40-210, 60-210 or 65-210 pM. In yet another embodiment, the ADCs provided exhibit IC₅₀s of about 10, 40, 60 or 80 pM. In still another embodiment, the ADCs provided exhibit IC₅₀s of about 11, 42, 60 or 83 pM.

It has also been found that the ADCs, in some embodiments, effect a cure rate in mice of at least 20%, 30%, 40%, 50%, 60%, 70%, 80%, 90% or 95%. In other embodiments, the cure rate in mice is about 20%, 30%, 40%, 50%, 60%, 70%, 80%, 90% or 95%. In still other embodiments, the cure rate is 20-40%, 40-60% or 60-80%. As used herein, "cure rate" refers to the number of mice still alive after about 500 days from the start of a study period, with no evidence of a tumor and no measurable PSA levels, divided by the number of mice at the beginning of the study period. To assess the cure rate, mice are administered 6 mg/kg ADC with a regimen of q4d x 6. In some embodiments, the number of mice at the beginning of the study is at least 5, 6, 7, 8, 9, 10, 12, 15, 17, 20, 25, 30 or more mice. Further details regarding an example of such a study are provided herein below in the **Examples**. In one embodiment, the mice are those that are a model of androgen-independent human prostate cancer. In another embodiment, the mice are nude mice engrafted with C4-2 cells intramuscularly in the left hind-leg. Techniques for determining the presence of a tumor and for measuring PSA levels are well known to those of ordinary skill in the art.

Binding of the ADCs of the invention to live PSMA-expressing cells can inhibit the growth of PSMA-expressing cells, result in cell-cycle arrest (e.g., G₂/M arrest), promote apoptosis of PSMA-expressing cells, etc. As used herein, "result in cell-cycle arrest" refers to an increase in the number of cells in the G₂/M phase due to the administration of an ADC. In some embodiments, the ADCs can effect apoptosis. In other embodiments, the ADCs result in both cell cycle arrest and subsequent apoptosis. The ADCs of the invention, therefore, can be used in various *in vitro* and *in vivo* methods for effecting these possible endpoints. In particular, the ADCs of the invention can be used in methods for treating PSMA-mediated disease.

As used herein, a "PSMA-mediated disease" is any disease in which PSMA is causative or a symptom of the disease. PSMA-mediated diseases also include diseases or disorders in which there is aberrant (e.g., overexpression) of PSMA. PSMA is a 100 kD Type II membrane glycoprotein expressed in prostate tissues (Horoszewicz et al., 1987, Anticancer Res. 7:927-935; U.S. Pat. No. 5,162,504). PSMA was characterized as a type II transmembrane protein having sequence identity with the transferrin receptor (Israeli et al., 1994, Cancer Res. 54:1807-1811) and with NAALADase activity (Carter et al., 1996, Proc. Natl. Acad. Sci: U.S.A. 93:749-753). More importantly, PSMA is expressed in increased amounts in prostate cancer, and elevated levels of PSMA are also detectable in the sera of these patients (Horoszewicz et al., 1987; Rochon et al., 1994, Prostate 25:219-223; Murphy et al., 1995, Prostate 26:164-168; and Murphy et al., 1995, Anticancer Res. 15:1473-1479). Therefore, a PSMA-mediated disorder is, for example, prostate cancer. PSMA expression increases with disease progression, becoming highest in metastatic, hormone-refractory disease for which there is no present therapy. In addition, provocative data indicates that PSMA is also abundantly expressed on the neovasculature of a variety of other important tumors, including bladder, pancreas, sarcoma, melanoma, lung, and kidney tumor cells, but not on normal vasculature. PSMA-mediated diseases, therefore, include cancers in which PSMA is expressed on the cells of the tumor or of the tumor neovasculature.

Compositions are, therefore, provided that can be used to treat any PSMA-mediated disorder. For example, ADCs can be used to inhibit the neovascularization of a tumor. In another example, PSMA ADCs can be used to kill tumor cells. In some embodiments, two or more different ADCs are used in combination. In another embodiment, one or more unconjugated anti-PSMA antibodies or antigen-binding fragments thereof can be combined with one or more ADCs in a single therapy to achieve a desired therapeutic effect. As an illustration, an unconjugated anti-PSMA antibody that mediates highly effective killing of target cells in the presence of effector cells and/or that inhibits the growth of cells expressing PSMA can be used with one or more ADCs. In yet another embodiment, the ADCs can be combined with one or more additional therapeutic agents. Such therapeutic agents include antitumor agents, such as docetaxel; corticosteroids, such as prednisone or hydrocortisone; immunostimulatory agents; immunomodulators; or some combination thereof.

Antitumor agents include cytotoxic agents, chemotherapeutic agents and agents that act on tumor neovasculature. Cytotoxic agents include cytotoxic radionuclides, chemical toxins and protein toxins. The cytotoxic radionuclide or radiotherapeutic isotope can be an alpha-emitting isotope such as ²²⁵Ac, ²¹¹At, ²¹²Bi, ²¹³Bi, ²¹²Pb, ²²⁴Ra or ²²³Ra. Alternatively, the cytotoxic radionuclide can be a beta-emitting isotope such as ¹⁸⁶Rh, ¹⁸⁸Rh, ¹⁷⁷Lu, ⁹⁰Y, ¹³¹I, ⁶⁷Cu, ⁶⁴Cu, ¹⁵³Sm or ¹⁶⁶Ho. Further, the cytotoxic radionuclide can emit Auger and low energy electrons and include the isotopes ¹²⁵I, ¹²³I or ⁷⁷Br.

Suitable chemical toxins or chemotherapeutic agents include members of the enediyne family of molecules, such as calicheamicin and esperamicin. Chemical toxins can also be taken from the group consisting of methotrexate, doxorubicin, melphalan, chlorambucil, ARA-C, vindesine, mitomycin C, cis-platinum, etoposide, bleomycin and 5-fluorouracil. Other antineoplastic agents include dolastatins (U.S. Patent Nos. 6,034,065 and 6,239,104) and derivatives thereof. Dolastatins and derivatives thereof include dolastatin 10 (dolavaline-valine-dolaisoleuine-dolaproine-dolaphenine) and the derivatives auristatin PHE (dolavaline-valine-dolaisoleuine-dolaproine-phenylalanine-methyl ester) (Pettit, G.R. et al., Anticancer Drug Des. 13(4):243-277, 1998; Woylce, T. et al., Antimicrob. Agents Chemother. 45(12):3580-3584, 2001), and aurastatin E and the like. Toxins also include poisonous lectins, plant toxins such as ricin, abrin, modeccin, botulina and diphtheria toxins. Other chemotherapeutic agents are known to those skilled in the art.

Agents that act on the tumor vasculature include tubulin-binding agents such as combrestatin A4 (Griggs et al., Lancet Oncol. 2:82, 200 1), angiostatin and endostatin (reviewed in Rosen, Oncologist 5:20, 2000) and interferon inducible protein 10 (U.S. Patent No. 5,994,292). A number of other antiangiogenic agents are also contemplated and include: 2ME2, Angiostatin, Angiozyme, Anti-VEGF RhuMAb, Apra (CT-2584), Avicine, Benefin, BMS275291, Carboxyamidotriazole, CC4047, CC5013, CC7085, CDC801, CGP-41251 (PKC 412), CM101, Combretastatin A-4 Prodrug, EMD 121974, Endostatin, Flavopiridol, Genistein (GCP), Green Tea Extract, IM-862, ImmTher, Interferon alpha, Interleukin-12, Iressa (ZD1839), Marimastat, Metastat (Col-3), Neovastat, Octreotide, Paclitaxel, Penicillamine, Photofrin, Photopoint, PI-88, Prinomastat (AG-3340), PTK787 (ZK22584), RO317453, Solimastat, Squalamine, SU 101, SU 5416, SU-6668, Suradista (FCE 26644), Suramin (Metaret), Tetrathiomolybdate, Thalidomide, TNP-470 and Vitaxin. Additional antiangiogenic agents are described by Kerbel, J. Clin. Oncol. 19(18s):45s-51s, 2001.

The ADCs can be administered with one or more immunostimulatory agents to induce or enhance an immune response, such as IL-2 and immunostimulatory oligonucleotides (e.g., those containing CpG motifs). Immunostimulatory agents can, in some embodiments, stimulate specific arms of the immune system, such as natural killer (NK) cells that mediate antibody-dependent cell cytotoxicity (ADCC). Immunostimulatory agents include interleukin-2, α-interferon, γ-interferon, tumor necrosis factor alpha (TNFα), immunostimulatory oligonucleotides or a combination thereof. Immunomodulators include cytokines, chemokines, adjuvants or a combination thereof. Chemokines useful in increasing immune responses include but are not limited to SLC, ELC, MIP3α, MIP3β, IP-10, MIG, and combinations thereof.

The other therapeutic agent can also be a vaccine. In some embodiments, the vaccine immunizes a subject against PSMA. Such vaccines, in some embodiments, include antigens, such as PSMA dimers, with, optionally, one or more adjuvants to induce or enhance an immune response. An adjuvant is a substance which potentiates the immune response. Adjuvants of many kinds are well known in the art. Specific examples of adjuvants include monophosphoryl lipid A (MPL, SmithKline Beecham); saponins including QS21 (SmithKline Beecham); immunostimulatory oligonucleotides (e.g., CpG oligonucleotides described by Kreig et al., Nature 374:546-9, 1995);incomplete Freund's adjuvant; complete Freund's adjuvant; montanide; vitamin E and various water-in-oil emulsions prepared from biodegradable oils such as squalene and/or tocopherol, Quil A, Ribi Detox, CRL-1005, L-121, and combinations thereof. Formulations, such as those described in U.S. Application Serial No. 10/976352, are also contemplated for use as vaccines in the methods provided herein.

The vaccines can, in some embodiments, include one or more of the isolated PSMA protein multimers described herein, such as the PSMA protein dimer. In some embodiments, a PSMA protein multimer composition contains at least about 10% PSMA protein multimer (of the total amount of PSMA protein in the composition). In other embodiments, the PSMA protein multimer composition contains at least about 20%, 30%, 40%, 50%, 60%, 70%, 75%, 80%, 85%, 90%, 95%, 99% or 99.5% PSMA protein multimer. In one embodiment, the PSMA protein multimer composition contains substantially pure PSMA protein multimer, with substantially no PSMA protein monomer. It is understood that the list of specific percentages includes by inference all of the unnamed percentages between the recited percentages.

Cytokines can also be used in vaccination protocols as a result of their lymphocyte regulatory properties. Many cytokines useful for such purposes will be known to one of ordinary skill in the art, including interleukin-2 (IL-2); IL-4; IL-5; IL-12, which has been shown to enhance the protective effects of vaccines (*see, e.g.,* Science 268: 1432-1434, 1995); GM-CSF; IL-15; IL-18; combinations thereof, and the like. Thus cytokines can be administered in conjunction with antigen, chemokines and/or adjuvants to increase an immune response.

The other therapeutic agents can be present in the compositions of the invention in unconjugated form or in conjugated form, such as conjugated to an anti-PSMA antibody or antigen-binding fragment thereof. Coupling of one or more toxin molecules to the anti-PSMA antibody or antigen-binding fragment thereof can include many chemical mechanisms, for instance covalent binding, affinity binding, intercalation, coordinate binding and complexation.

The covalent binding can be achieved either by direct condensation of existing side chains or by the incorporation of external bridging molecules. Many bivalent or polyvalent agents are useful in coupling protein molecules to other proteins, peptides or amine functions, etc. For example, the literature is replete with coupling agents such as carbodiimides, diisocyanates, glutaraldehyde, diazobenzenes, and hexamethylene diamines. This list is not intended to be exhaustive of the various coupling agents known in the art but, rather, is exemplary of the more common coupling agents.

In some embodiments, it is contemplated that one may wish to first derivatize the antibody, and then attach the therapeutic agent to the derivatized product. Suitable cross-linking agents for use in this manner include, for example, SPDP (N-succinimidyl-3-(2-pyridyldithio)propionate), and SMPT, 4-succinimidyl-oxycarbonyl-methyl-(2-pyridyldithio)toluene.

In addition, protein toxins can be fused to the anti-PSMA antibody or antigen-binding fragment thereof by genetic methods to form a hybrid immunotoxin fusion protein. The fusion proteins can include additional peptide sequences, such as peptide spacers which operatively attach, for example, the anti-PSMA antibody and toxin, as long as such additional sequences do not appreciably affect the targeting or toxin activities of the fusion protein. The proteins can be attached by a peptide linker or spacer, such as a glycine-serine spacer peptide, or a peptide hinge, as is well known in the art. Thus, for example, the C-terminus of an anti-PSMA antibody or antigen-binding fragment thereof can be fused to the N-terminus of the protein toxin molecule to form an immunotoxin that retains the binding properties of the anti-PSMA antibody. Other fusion arrangements will be known to one of ordinary skill in the art. To express the fusion immunotoxin, the nucleic acid encoding the fusion protein is inserted into an expression vector in accordance with standard methods, for stable expression of the fusion protein, such as in mammalian cells, such as CHO cells. The fusion protein can be isolated and purified from the cells or culture supernatant using standard methodology, such as a PSMA affinity column.

Radionuclides typically are coupled to an antibody or antigen-binding fragment thereof by chelation. For example, in the case of metallic radionuclides, a bifunctional chelator is commonly used to link the isotope to the antibody or other protein of interest. Typically, the chelator is first attached to the antibody, and the chelator-antibody conjugate is contacted with the metallic radioisotope. A number of bifunctional chelators have been developed for this purpose, including the diethylenetriamine pentaacetic acid (DTPA) series of amino acids described in U.S. patents 5,124,471, 5,286,850 and 5,434,287. As another example, hydroxamic acid-based bifunctional chelating agents are described in U.S. patent 5,756,825. Another example is the chelating agent termed *p*-SCN-Bz-HEHA (1,4,7,10,13,16-hexaazacyclo-octadecane- N,N',N",N"',N"",N""'-hexaacetic acid) (Deal et al., J. Med. Chem. 42:2988, 1999), which is an effective chelator of radiometals such as ²²⁵Ac. Yet another example is DOTA (1,4,7,10-tetraazacyclododecane N,N',N",N"'-tetraacetic acid), which is a bifunctional chelating agent (see McDevitt et al., Science 294:1537-1540, 2001) that can be used in a two-step method for labeling followed by conjugation.

Other therapeutic agents also include replication-selective viruses. Replication-competent virus such as the p53 pathway targeting adenovirus mutant dl1520, ONYX-015, kills tumor cells selectively (Biederer, C. et al., J. Mol. Med. 80(3):163-175, 2002). The virus can, in some embodiments, be conjugated to PSMA antibodies or antigen-binding fragments thereof.

The compositions provided of the present invention can be used in conjunction with other therapeutic treatment modalities. Such other treatments include surgery, radiation, cryosurgery, thermotherapy, hormone treatment, chemotherapy, vaccines and other immunotherapies.

The ADCs of the invention, such as through their antibody or antigen-binding fragment thereof, can be linked to a label. Labels include, for example, fluorescent labels, enzyme labels, radioactive labels, nuclear magnetic resonance active labels, luminescent labels or chromophore labels.

The compositions provided can include a physiologically or pharmaceutically acceptable carrier, excipient or stabilizer mixed with the ADC. In some embodiments, when a composition comprises two or more different ADCs, each of the antibodies or antigen-binding fragments thereof of the ADCs binds to a distinct conformational epitope of PSMA.

As used herein, "target cell" shall mean any undesirable cell in a subject (e.g., a human or animal) that can be targeted by an ADC of the invention. In some embodiments, the target cell is a cell expressing or overexpressing PSMA. Cells expressing PSMA or PSMA-expressing cells, typically include tumor cells, such as prostate, bladder, pancreas, lung, kidney, colon tumor cells, as well as melanoma and sarcoma cells.

Pharmaceutical compositions of the invention can be administered in combination therapy, i.e., combined with other agents. For example, the combination therapy can include a composition of the present invention with at least one anti-tumor agent, immunomodulator, immunostimulatory agent or other conventional therapy. The other agent can be conjugated to or formed as a recombinant fusion molecule with a PSMA antibody or antigen-binding fragment thereof for directed targeting of the agent to PSMA-expressing cells. In another embodiment the other therapeutic agent can be unconjugated. Additional therapeutic agents can be administered or contacted with the PSMA-expressing cells through co-administration. "Co-administering," as used herein, refers to administering two or more therapeutic agents simultaneously as an admixture in a single composition, or sequentially, and close enough in time so that the compounds may exert an additive or even synergistic effect. In still other embodiments, an additional therapeutic agent can be administered before, during or after the administration of one or more ADCs or compositions thereof.

As used herein, "pharmaceutically acceptable carrier" or "physiologically acceptable carrier" includes any and all salts, solvents, dispersion media, coatings, antibacterial and antifungal agents, isotonic and absorption delaying agents, and the like that are physiologically compatible. In some embodiments, the carrier is suitable for intravenous, intramuscular, subcutaneous, parenteral, spinal or epidermal administration (e.g., by injection or infusion). Depending on the route of administration, the active compound, can be coated in a material to protect the compound from the action of acids and other natural conditions that may inactivate the compound.

When administered, the pharmaceutical preparations of the invention are applied in pharmaceutically-acceptable amounts and in pharmaceutically-acceptable compositions. The term "pharmaceutically acceptable" means a non-toxic material that does not interfere with the effectiveness of the biological activity of the active ingredients. Such preparations may routinely contain salts, buffering agents, preservatives, compatible carriers, and optionally other therapeutic agents, such as supplementary immune potentiating agents including adjuvants, chemokines and cytokines. When used in medicine, the salts should be pharmaceutically acceptable, but non-pharmaceutically acceptable salts may conveniently be used to prepare pharmaceutically-acceptable salts thereof and are not excluded from the scope of the invention.

A salt retains the desired biological activity of the parent compound and does not impart any undesired toxicological effects (see e.g., Berge, S.M., et al. (1977) J. Pharm. Sci. 66: 1-19). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous and the like, as well as from nontoxic organic acids such as aliphatic mono- and dicarboxylic acids, phenyl substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chioroprocaine, choline, diethanolamine, ethylenediamine, procaine and the like.

An ADC can be combined, if desired, with a pharmaceutically-acceptable carrier. The term "pharmaceutically-acceptable carrier" as used herein means one or more compatible solid or liquid fillers, diluents or encapsulating substances which are suitable for administration into a human. The term "carrier" denotes an organic or inorganic ingredient, natural or synthetic, with which the active ingredient is combined to facilitate the application. The components of the pharmaceutical compositions also are capable of being co-mingled in a manner such that there is no interaction which would substantially impair the desired pharmaceutical efficacy.

The pharmaceutical compositions may contain suitable buffering agents, including: acetic acid in a salt; citric acid in a salt; boric acid in a salt; and phosphoric acid in a salt.

The pharmaceutical compositions also may contain, optionally, suitable preservatives, such as: benzalkonium chloride; chlorobutanol; parabens and thimerosal.

The pharmaceutical compositions may conveniently be presented in unit dosage form and may be prepared by any of the methods well-known in the art of pharmacy. All methods include the step of bringing the active agent into association with a carrier which constitutes one or more accessory ingredients. In general, the compositions are prepared by uniformly and intimately bringing the active compound into association with a liquid carrier, a finely divided solid carrier, or both, and then, if necessary, shaping the product.

Compositions suitable for parenteral administration conveniently comprise a sterile aqueous or non-aqueous preparation of the compounds, which is, in some embodiments, isotonic with the blood of the recipient. This preparation may be formulated according to known methods using suitable dispersing or wetting agents and suspending agents. The sterile injectable preparation also may be a sterile injectable solution or suspension in a non-toxic parenterally-acceptable diluent or solvent, for example, as a solution in 1,3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono-or di-glycerides. In addition, fatty acids such as oleic acid may be used in the preparation of injectables. Carrier formulations suitable for oral, subcutaneous, intravenous, intramuscular, etc. administration can be found in Remington's Pharmaceutical Sciences, Mack Publishing Co., Easton, PA.

The active compounds can be prepared with carriers that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, e.g., Sustained and Controlled Release Drug Delivery Systems, J.R. Robinson, ed., Marcel Dekker, Inc., New York, 1978.

The therapeutics of the invention can be administered by any conventional route, including injection or by gradual infusion over time. The administration may, for example, be oral, intravenous, intraperitoneal, intramuscular, intracavity, intratumor, or transdermal. When compounds containing antibodies are used therapeutically, routes of administration include intravenous and by pulmonary aerosol. Techniques for preparing aerosol delivery systems containing antibodies are well known to those of skill in the art. Generally, such systems should utilize components which will not significantly impair the biological properties of the antibodies, such as the paratope binding capacity (see, for example, Sciarra and Cutie, "Aerosols," in Remington's Pharmaceutical Sciences, 18th edition,1990, pp. 1694-1712). Those of skill in the art can readily determine the various parameters and conditions for producing antibody aerosols without resorting to undue experimentation.

The compositions of the invention are administered in effective amounts. An "effective amount" is that amount of any of the ADCs provided herein that alone, or together with further doses and/or other therapeutic agents, produces the desired response, e.g., treats a PSMA-mediated disease in a subject. This can involve only slowing the progression of the disease temporarily, although in some embodiments, it involves halting the progression of the disease permanently. This can be monitored by routine methods. The desired response to treatment of the disease or condition also can be delaying the onset or even preventing the onset of the disease or condition. An amount that is effective can be the amount of an ADC alone which produces the desired therapeutic endpoint. An amount that is effective is also the amount of an ADC in combination with another agent that produces the desired result.

Such amounts will depend, of course, on the particular PSMA-mediated disease being treated, the severity of the condition, the individual patient parameters including age, physical condition, size and weight, the duration of the treatment, the nature of concurrent therapy (if any), the specific route of administration and like factors within the knowledge and expertise of the health practitioner. These factors are well known to those of ordinary skill in the art and can be addressed with no more than routine experimentation. It is generally preferred that a maximum dose of the individual components or combinations thereof be used, that is, the highest safe dose according to sound medical judgment. It will be understood by those of ordinary skill in the art, however, that a patient may insist upon a lower dose or tolerable dose for medical reasons, psychological reasons or for virtually any other reasons.

The pharmaceutical compositions used in the foregoing methods preferably are sterile and contain an effective amount of an ADC, alone or in combination with another agent, for producing the desired response in a unit of weight or volume suitable for administration to a patient. The response can, for example, be measured by determining the physiological effects of the ADC composition, such as regression of a tumor or decrease of disease symptoms. Other assays will be known to one of ordinary skill in the art and can be employed for measuring the level of the response.

The doses of ADCs administered to a subject can be chosen in accordance with different parameters, in particular in accordance with the mode of administration used and the state of the subject. Other factors include the desired period of treatment. In the event that a response in a subject is insufficient at the initial doses applied, higher doses (or effectively higher doses by a different, more localized delivery route) may be employed to the extent that patient tolerance permits.

In general, doses can range from about 10 µg/kg to about 100,000 µg/kg. In some embodiments, the doses can range from about 0.1 mg/kg to about 20 mg/kg. In still other embodiments, the doses range from about 0.1 mg/kg to 5 mg/kg, 0.1 mg/kg to 10 mg/kg or 0.1 mg/kg to 15 mg/kg. In yet other embodiments, the doses range from about 1 mg/kg to 5 mg/kg, 5 mg/kg to 10 mg/kg, 10 mg/kg to 15 mg/kg or 15 mg/kg to 20 mg/kg. In further embodiments, the dose is about 0.1 mg/kg, 0.5 mg/kg, 1 mg/kg, 2 mg/kg, 3 mg/kg, 5 mg/kg, 7 mg/kg, 10 mg/kg, 12 mg/kg, 15 mg/kg, 17 mg/kg, 20 mg/kg, 25 mg/kg or 30 mg/kg. In another embodiment, the dose is about 1 mg/kg, 3 mg/kg, 5 mg/kg or 6 mg/kg. Based upon the composition, the dose can be delivered continuously, such as by continuous pump, or at periodic intervals. In some embodiments, when the ADC is administered intravenously, the dose is between 0.1 and 20 mg/kg or any value in between. Desired time intervals of multiple doses of a particular composition can be determined without undue experimentation by one skilled in the art. Other protocols for the administration of the compositions provided will be known to one of ordinary skill in the art, in which the dose amount, schedule of administration, sites of administration, mode of administration and the like vary from the foregoing. In some embodiments, subjects are administered the ADC with a dose regimen of q4d x 3 or q4d x 6. In one embodiment, the dose is administered intravenously. In another embodiment, the dose regimen is a single intravenous dose.

Administration of ADC compositions to mammals other than humans, e.g. for testing purposes or veterinary therapeutic purposes, is carried out under substantially the same conditions as described above.

The compositions of the present invention have *in vitro* and *in vivo* diagnostic and therapeutic utilities. For example, these molecules can be administered to cells in culture, e.g. *in vitro* or *ex vivo*, or in a subject, e.g., *in vivo,* to treat, prevent or diagnose a variety of PSMA-mediated diseases. As used herein, the term "subject" is intended to include humans and non-human animals. Subjects include a human patient having a disorder characterized by expression, typically aberrant expression (e.g., overexpression) of PSMA, such disorders are included in the definition of "PSMA-mediated disease".

The compositions provided herein can be utilized in *in vivo* therapy of cancer. The ADCs can be used to inhibit proliferation of the malignant cells or tissues following administration and localization of the conjugates. The compositions provided can include anti-PSMA antibodies, in some embodiments, that may mediate tumor destruction by complement fixation or antibody-dependent cellular cytotoxicity. Alternatively, the compositions can contain an additional therapeutic agent to result in synergistic therapeutic effects (Baslya and Mendelsohn, 1994 Breast Cancer Res. and Treatment 29:127-138).

The compositions of the invention can also be administered together with, in some embodiments, complement and/or unconjugated anti-PSMA antibodies. Accordingly, within the scope of the invention are compositions comprising ADC and serum or complement. These compositions are advantageous in that the complement is located in close proximity to the human antibodies or antigen-binding fragments thereof. Alternatively, the ADCs, antibodies or antigen-binding fragments thereof and/or complement or serum can be administered separately.

Use of the ADCs of the present invention has a number of benefits. Since the ADCs preferentially target PSMA e.g., on prostate cancer cells, other tissue can be spared. As a result, treatment with such biological agents is safer, particularly for elderly patients. Treatment with ADCs according to the present invention is expected to be particularly effective, in some embodiments, because it can direct high levels of ADCs to the bone marrow and lymph nodes where cancer metastases, such as prostate cancer metastases, can predominate. Treatment with ADCs in accordance with the present invention can be effectively monitored with clinical parameters such as serum prostate specific antigen and/or pathological features of a patient's cancer, including stage, Gleason score, extracapsular, seminal, vesicle or perineural invasion, positive margins, involved lymph nodes, etc. Alternatively, these parameters can be used to indicate when such treatment should be employed.

Also within the scope of the invention are kits comprising the compositions, e.g., one or more ADCs, of the invention and instructions for use. The kits can further contain at least one additional reagent, such as complement, a chemotherapeutic agent, a corticosteroid, or one or more antibodies that bind PSMA. Other kits can also include PSMA multimers. In another embodiment, a kit can comprise a carrier being compartmentalized to receive in close confinement therein one or more container means or series of container means such as test tubes, vials, flasks, bottles, syringes, or the like. A first of said container means or series of container means may contain one or more anti-PSMA antibodies or antigen-binding fragments thereof. A second container means or series of container means can, in some embodiments, contain MMAE or MMAF or the compound of **Formula 1** conjugated to MMAE or MMAF. In some embodiments, a third container means or series of container means contain a compound of **Formula 1**. Kits for use in *in vivo* tumor localization and therapy method containing the ADCs can be prepared. The components of the kits can be packaged either in aqueous medium or in lyophilized form. The components of the ADC conjugates can be supplied either in fully conjugated form, in the form of intermediates or as separate moieties to be conjugated by the user of the kit.

As used herein with respect to polypeptides, proteins or fragments thereof, "isolated" means separated from its native environment and present in sufficient quantity to permit its identification or use. Isolated, when referring to a protein or polypeptide, means, for example: (i) selectively produced by expression cloning or (ii) purified as by chromatography or electrophoresis. Isolated proteins or polypeptides may be, but need not be, substantially pure. The term "substantially pure" means that the proteins or polypeptides are essentially free of other substances with which they may be found in nature or *in vivo* systems to an extent practical and appropriate for their intended use. Substantially pure polypeptides may be produced by techniques well known in the art. Because an isolated protein may be admixed with a pharmaceutically acceptable carrier in a pharmaceutical preparation, the protein may comprise only a small percentage by weight of the preparation. The protein is nonetheless isolated in that it has been separated from the substances with which it may be associated in living systems, i.e. isolated from other proteins.

The compositions provided herein can be in lyophilized form or provided in an aqueous medium.

The present invention is further illustrated by the following **Examples**.

### Examples

### Example 1: Potent Antitumor Activity of an Auristatin-Coniugated, Fully Human

### Monoclonal Antibody to Prostate-Specific Membrane Antigen

### Materials and Methods

### Cell Lines and Antibodies

LNCaP™ (CRL-1740), PC-3™ (CRL-1435), and 3T3™ (CRL-2752) were obtained from American Type Culture Collection (Rockville, MD). C4-2 cell line, a sub-cell line from LNCaP™, was obtained from The Cleveland Clinic Foundation (Cleveland, OH). A 3T3™-PSMA cell line was obtained from Memorial Sloan-Kettering Cancer Center (New York, NY). LNCaP™, C4-2 and PC-3™ were cultured in RPMI 1640 (Life Technologies, Gaithersburg, MD), and 3T3™ and 3T3™-PSMA were cultured in DMEM (Life Technologies). Culture media were supplemented with 10% fetal bovine serum (Hyclone, Logan, UT), L-glutamine, penicillin and streptomycin (Life Technologies). C4-2, LNCaP™ and 3T3™-PSMA cells were determined to express PSMA at levels of approximately 2 x 10⁵, 6 x 10⁵ and >1 x 10⁶ copies/cell, respectively, according to published methods (Ma D, et al., Leukemia 2002;16:60-6.). C4-2 is an androgen-independent subclone of androgen-dependent LNCaP™ cells. PC-3™ is a de-differentiated prostate cancer cell line that does not express PSMA. PSMA mAbs (AB-PG1-XG1-006 (PTA-4403 and PTA-4404) and Abgenix 4.40.2 (PTA-4360)) were produced as described previously in U.S. Pat. Appl. No. 10/395,894 and Schulke N et al., PNAS USA, 2003; 100:12590-5, each of which is herein incorporated by reference in its entirety. Abgenix 4.40.2 was used as a control. A fully human PSMA mAb (IgG1,κ) was raised in mice transgenic for the human immunoglobulin gene locus (XenoMice™, Abgenix, Inc., Fremont, CA) following immunization with recombinant soluble PSMA and LNCaP cells as previously described (Schulke N et al., PNAS USA, 2003; 100:12590-5).

### PSMA Internalization

mAbs were modified with bifunctional chelates of cyclohexyl-diethylenetriamine pentaacetic acid (CHX-DTPA) obtained from the National Cancer Institute (Bethesda, MD), and labeled with ¹¹¹In (PerkinElmer, Boston, MA) as previously described (Ma D, et al., Leukemia 2002;16:60-6; Nikula TK, et al., J Nucl.Med 1999;40:166-76). ¹¹¹In-labeled mAb was determined to be >90% immunoreactive by incubating the radioconjugate with an excess of 3T3™-PSMA cells and measuring the bound fraction according to published methods (Ma D, et al., Leukemia 2002;16:60-6; Nikula TK, et al., J Nucl.Med 1999;40:166-76). For internalization analysis, ¹¹¹In-labeled mAb was incubated with 2 x 10⁵ C4-2 cells at 37°C in 5% CO₂. At sequential time points, unbound mAb was removed by washing in PBS and cell-surface mAb was eluted using low pH buffer (pH 2.4, glycine/NaCl). The low pH eluate was counted separately from the cell pellet, and percent internalization was calculated as previously described (McDevitt MR, et al., Cancer Res 2000;60:6095-100).

### Preparation of Antibody-Drug Conjugates

The synthesis and design of the linkers and the conjugation of the linker to the cytotoxic drug were carried out as described in U.S. Pat. No. 6,884,889 and U.S. Pat. No. 6,214,345, each of which is herein incorporated by reference in its entirety. The conjugation of mAbs with maleimidocaproyl (mc)-valine (Val)-citrulline (Cit)-monomethyl auristatin E (MMAE) was performed as described (Doronina SO, et al., Nat. Biotechnology. 2003;21:778-84). PSMA mAb and isotype-control human IgG1 (Calbiochem, San Diego, CA) in PBS containing 50 mM borate, pH 8.0, were treated with dithiothreitol (DTT) (10 mM final) at 37°C for 30 min. The final reaction concentrations were 7.5 mL - 8.0 ml, 1 mL 0.5 M sodium borate pH 8 and 0.5 M NaCl, 1 mL 100 mM DTT, and 0.5 mL or 0 ml, respectively, of PBS. This solution was incubated at 40°C for 1hr, and the antibody purified on a gel filtration column. The column was equilibrated with 10 mM DTPA in PBS at 10 mL/min, loaded with 10.0 mL of the antibody reduction mixture, and eluted at 8 mL/min in PBS/DTPA buffer. The concentration of antibody-cysteine thiols produced was determined by titrating with 5,5'-dithio-bis-(2-nitrobenzoic acid) (DTNB) (Pierce Chemical Co., Rockford, IL). An equivalent chemical can be obtained from Sigma (St. Louis, MO).

The fully reduced mAb Abgenix 4.40.2 (22.6 mL of 7.8 µM mAb, 75.6 µM cysteine thiol) was partially reoxidized with 35.43 µL of 10 mM DTNB, and the fully reduced mAb AB-PG1-XG1-006 (25.1 mL of 11.2 µM mAb, 95.8 µM cysteine thiol) was partially reoxidized with 56.27 µL of 10 mM DTNB. The color of the solution immediately turned yellow.

The drug mc-Val-Cit-paraaminobenzyl carbamate-MMAE (vcMMAE) was then conjugated to the partially reoxidized mAbs as follows: the mAbs were first cooled to 0°C. vcMMAE (5 molar equivalents per antibody: 89.7 and 140.6 µL, respectively, of a 10 mM stock solution of vcMMAE) was dissolved in 5 mL acetonitrile, then added to the antibody solution while carefully vortexing. The reaction mixtures were incubated on ice. No additional color change was observed. The reaction mixtures were quenched with 20 molar equivalents of cysteine/drug. The conjugate was purified using a gel-filtration column at 4°C and eluted with PBS at 8.0 mL/min. The ADCs were determined to have ≥ 98% monomeric mAb containing 3.0-3.5 drugs per mAb using published methods (Doronina SO, et al., Nat Biotechnol. 2003;21:778-84).

Alternatively, the conjugation of mAbs with maleimidocaproyl (mc)-valine (Val)-citrulline (Cit)-monomethyl auristatin E (MMAE) was performed as described (Doronina SO, et al., Nat. Biotechnology. 2003;21:778-84). PSMA mAb and isotype-control human IgG1 (Calbiochem, San Diego, CA) in PBS containing 50 mM borate, pH 8.0, were treated with dithiothreitol (DTT) (10 mM final) at 37°C for 30 min. The mAbs were exchanged into PBS containing 1 mM DTPA (Aldrich, Milwaukee, WI) by passage through a Sephadex G-25 column (Amersham Biosciences, Piscataway, NJ). The mAb solutions were chilled to 4°C and combined with the maleimido drug derivative in cold CH₃CN. After 1 hour, the reactions were quenched with excess cysteine, and the conjugates were concentrated and exchanged into PBS buffer. The ADCs were determined to have ≥ 98% monomeric mAb containing 3.0-3.5 drugs per mAb using published methods (Doronina SO, et al., Nat Biotechnol. 2003;21:778-84).

### Reactivity of ADCs with Cell-Surface, PSMA

Binding of PSMA mAb and ADC to 3T3™-PSMA and parental 3T3™ cells was analyzed using a FACSCalibur flow cytometer (BD Bioscience, San Diego, CA). Briefly, 2 x 10⁵ 3T3™-PSMA (or 3T3™) cells were incubated with different concentrations of mAb or ADC on ice for 1h. After washing, the presence of bound antibody was detected using goat anti-human IgG-FITC (Caltag Laboratories, Burlingame, CA). Isotype-control antibody and ADC were examined in parallel.

### In Vitro Cytotoxicity Assay

PSMA-positive cells (C4-2, LNCaP™ or 3T3™-PSMA) and PSMA-negative cells (PC-3™ or 3T3™) were added to 96-well microplates (Falcon, BD Biosciences, San Jose, CA) at 2.5 x 10³ cells/well and incubated overnight at 37°C and 5% CO₂. Cells were then incubated with serially diluted ADCs for 4 days. The cell culture medium was replaced with fresh medium containing 10% Alamar Blue (Biosource International, Camarillo, CA), and cells were incubated for 4h. Plates were then read on a fluorescence plate reader using an excitation wavelength of 530 nm and an emission wavelength of 590 nm. Cell survival was compared in treated and untreated cultures, and the concentration of ADC required for 50% cell kill (IC₅₀ value) was determined.

### Xenograft Model of Androgen-Independent Prostate Cancer

All animal studies were carried out in accordance with Animal Care and Use Committee guidelines. Athymic male nude mice (National Cancer Institute, Frederick, MD) 6-8 weeks in age were implanted with an intramuscular injection of 5 x 10⁶ C4-2 cells mixed with 50% Matrigel (Beckon Dickinson Labware, Bedford, MA) into the left hind-leg as described (McDevitt MR, et al., Cancer Res 2000;60:6095-100). Approximately 1 day prior to initiation of treatment, animals were randomized according to serum levels of prostate-specific antigen (PSA) as measured by ELISA (Medicorp, Montreal, Quebec, Canada). ADC, mAbs and vehicle control were administered via tail vein injection. In the first series of experiments, mice were treated in groups of 6 with 2 or 10 mg/kg PSMA ADC or with vehicle control. Treatment was initiated 17 days post-implantation and consisted of 3 injections at 4-day intervals (q4d x 3). The second series of experiments examined dose levels of 0, 3 or 6 mg/kg. Treatment was initiated 14 days post-implantation and consisted of 6 injections at 4-day intervals (q4d x 6). Animals were monitored for their physical appearance, body weight, PSA level and tumor size. Survival rates were recorded throughout the studies.

### Statistical Analyses

Treatment effects were examined for significance via t-tests (for PSA levels) or log-rank tests (for animal survival) using two-tailed, paired analyses. Data were considered significant when *P* < 0.05.

### Results

### Internalization of PSMA mAb into Human Prostate Cancer Cells

Internalization was examined using ¹¹¹In-labeled PSMA mAb and C4-2 cells. Total binding and percent internalization over time are illustrated in **Fig.1****.** Over half of the bound mAb was internalized within 2h (**Fig. 1A**). Total binding increased over time, presumably due to PSMA recycling (**Fig. 1B**). Thus, the PSMA mAb is readily internalized into PSMA-expressing cells.

### Reactivity of the PSMA ADC with PSMA-expressing Cells

Flow cytometry was used to compare the binding of PSMA mAb and ADC. The unmodified mAb and ADC demonstrated comparable levels of binding to 3T3™-PSMA over a broad range of dilutions (**Fig. 2**). Neither the maximal amount of binding nor the concentration required for half-maximal binding was appreciably affected by conjugation. No significant binding was observed for the isotype-control ADC or antibody on 3T3™-PSMA cells or for PSMA mAb or ADC on parental 3T3™ cells.

### In vitro Potency and Selectivity of the PSMA ADC

PSMA and control ADCs were tested for cytotoxicity *in vitro* against human prostate cancer cells lines and 3T3™-PSMA cells. **Fig. 3** illustrates dose-response curves for PSMA-positive C4-2 cells and PSMA-negative PC-3™ cells in a representative experiment, and IC₅₀ values for the various cell lines are listed in **Table 2.** The PSMA ADC potently eliminated all PSMA-positive cell lines examined at IC₅₀ values of 65-210 pM, whereas these concentrations had no effect on PSMA-negative cells. In contrast, nearly 1000-fold higher concentrations were required for the control ADC, whose activity was independent of PSMA expression (**Fig. 3** and **Table 2**).

**Table 2: Summary of in vitro cytotoxicity (IC₅₀ values in pM)**

| | **C4-2** | **LNCaP™** | **3T3™-PSMA** |
|---|---|---|---|
| **PSMA ADC** | **65 ± 19 (n = 3)** | **83 ± 21 (n = 2)** | **208 ± 37 (n = 3)** |
| **Control ADC** | **54,954 (n = 1)** | **72,444 (n = 1)** | **154,880 (n = 1)** |
| **Selectivity*** | **848** | **877** | **744** |

| | | | |
|---|---|---|---|
| *Selectivity equals the ratio of IC₅₀ values observed for the PSMA ADC and control ADC. | | | |

### Efficacy of the PSMA ADC in a Xenograft Model of Androgen-Independent Prostate Cancer

*In vivo* efficacy of the PSMA ADC was evaluated in a mouse model of androgen-independent human prostate cancer. Nude mice were engrafted with C4-2 cells intramuscularly in the left hind-leg. Approximately 14-17 days later, serum PSA levels were measured and used to randomly assign animals to treatment groups. Animals were treated intravenously with the PSMA ADC, and animals were monitored for tumor burden, PSA levels and other parameters for as long as 500 days.

In the first experiment, animals were treated q4d x 3 with 0, 2 or 10 mg/kg PSMA ADC. Left untreated, tumors grew rapidly and animals had a median survival of 32 days. In contrast, the groups treated with 2 mg/kg and 10 mg/kg PSMA ADC had median survivals of 58 days (*P* = 0.0035) and 94.5 days (*P* = 0.0012), respectively (**Table 3,** **Fig. 4A**). The PSMA ADC treatment significantly improved median survival up to 4.5-fold in a dose-dependent fashion. There was no evidence of treatment-related toxicity.

Serum PSA levels were measured over time by ELISA. **Fig. 4B** depicts the mean PSA concentration in each group at study days 17, 23 and 30. Treatment at 10 mg/kg reduced PSA levels >10-fold from 8.8 ± 11.7 ng/mL at day 17 to 0.7 ± 0.9 ng/mL at day 30, whereas PSA levels in the control group increased >60-fold over the same time period. An intermediate response was observed at 2 mg/kg PSMA ADC. The differences in PSA levels at day 30 were significant for both the 2 mg/kg (*P* = 0.0048) and 10 mg/kg (*P* = 0.0006) dose groups. Three of six animals in the 10 mg/kg group had undetectable PSA through day 52 of the study.

To extend these findings, a second PSMA ADC study was conducted that also included unmodified mAb and isotype-control ADC. After randomization at day 14 with a mean PSA level of 2.0 ± 1.1 ng/mL in each group (n = 5), animals were treated with a regimen of q4d x 6. Kaplan-Meier survival curves for each group are depicted in **Fig. 5****.** Animals treated with vehicle control, 6 mg/kg unmodified PSMA mAb and 6 mg/kg control ADC had similar median survival times of 29, 31 and 31 days, respectively; and these differences were not significant. However, median survival was extended to 49 days and 148 days for animals treated with 3 mg/kg and 6 mg/kg PSMA ADC, respectively (**Table 3**). Treatment of the PSMA ADC group with 6 mg/kg improved post-randomization survival 7.9-fold relative to the control ADC group (*P* = 0.0018). At day 500, 2 of 5 animals had no evidence of tumor, no measurable PSA and were considered to be cured by treatment. As in the first study, treatment had a significant impact on PSA levels on day 29 (*P* = 0.0068 for 6 mg/kg PSMA and vehicle groups). Moreover, in the 6 mg/kg PSMA ADC group, serum PSA decreased to undetectable levels post-treatment and remained undetectable through day 63 in 4 of 5 animals. There was no overt toxicity associated with ADC therapy. Physical appearance and activity were unaffected by treatment, and body weights of treated and vehicle-control animals were not significantly different at any time point.

**Table 3: Summary of median survival times of C4-2 tumor-bearing animals treated with PSMA ADC**

| | **Test article** | **Dose (mg/kg)** | **Median survival (days)** | ***P* value*** |
|---|---|---|---|---|
| **Study #1** | **Vehicle** | **NA** | **32** | **NA** |
| | **PSMA ADC** | **2** | **58** | **0.0035** |
| | **PSMA ADC** | **10** | **95** | **0.0010** |
| **Study #2** | **Vehicle** | **NA** | **29** | **NA** |
| | **PSMA mAb** | **6** | **31** | **0.1869** |
| | **Control ADC** | **6** | **31** | **0.2970** |
| | **PSMA ADC** | **3** | **49** | **0.0018** |
| | **PSMA ADC** | **6** | **148** | **0.0018** |

| | | | | |
|---|---|---|---|---|
| *Compared to the vehicle control group in a two-sided log-rank analysis. NA= not applicable. | | | | |

### Example 2: Evaluation of PSMA mAb Conjugated to Three Different Drug-linkers

The PSMA mAb when conjugated to vcMMAE and two other drug-linkers, vcMMAF and mcMMAF, was evaluated. The full chemical structures of three different drug-linkers are illustrated in **Fig. 6****.**

### Preparation of Three Drug-linker Conjugates of PSMA mAb

The three drug-linkers were directly conjugated to PSMA mAb via a thioether bond to prepare approximately four drugs per antibody conjugates. Partial reduction of the mAb interchain disulfides proceeded with a slight excess of tris(2-carboxyethyl)phosphine (TCEP) at pH 7.2 and 37°C and subsequent conjugation of the free thiols with drug-linkers was quantitative. Briefly, the PSMA mAb (10 mg, 67.5 nmol in PBS) was incubated at 37°C with 1 mM DTPA and 169 nmol of TCEP for 90 min. At three time points during the incubation (30, 60 and 90 minutes), aliquots of 50 µg mAb were removed and reacted with an excess of vcMMAE. Analysis of the resulting ADCs by hydrophobic interaction chromatography allowed the progress of the reduction to be followed. The results indicated that the mAb was rapidly reduced under the above conditions, being essentially complete after 1 hour. Furthermore, the extent of reduction resulted in an average drug loading of 5 drugs/mAb.

To prepare a 4-loaded ADC with drug-linkers from the above partially reduced mAb, 0.5 equivalents of DTNB were added to re-oxidize the mAb population back to the desired level. Then, 3 mg of this material (20.3 nmol) was reacted with 101 nmol of vcMMAE, vcMMAF or mcMMAF in a 15% dimethyl sulfoxide (DMSO) reaction solution. This reaction proceeded for 1 hour at 0°C and was then quenched with a 20-fold excess of N-acetyl cysteine. The ADCs were separated from unreacted drug and other small molecule impurities by size exclusion chromatography (SEC) on a PD-10 column (Amersham Biosciences/GE Healthcare, Piscataway, NJ) and concentrated with a centrifugal concentration device (30 kD MWCO) (Amicon Bioseparations, Millipore Corporation, Bedford, MA).

A summary of the characterization of three drug-linker conjugates is provided in **Tables 4-6** for vcMMAE, vcMMAF and mcMMAF, respectively. For each of the three drug-linkers, ADC contains approximately 4 drugs per mAb, as determined by H/L-chain loading distribution and species distribution, and <2% free drug as determined using reversed phase (RP) HPLC. For all conjugates, no aggregates were detected by SEC-HPLC. In addition, the overall mAb yields were 70-80%.

**Table 4**

| Conjugate Certificate of Testing | | |
|---|---|---|
| **699028A** | | |
| PSMA mAb vcMMAE | | |
| Partial Reduction | | |
| Assay | Method | Result |
| **mAb Concentration** | UV | 3.3 |
| mg/mL | | |
| **Drug/mAb** | H/L-Chain Loading Distribution (PLRP) Species Distribution (HIC) | 4.3 |
| mol/mol | | |
| **Unconjugated Drug** | RP-HPLC | <0.5 |
| % of total drug | | |
| **Size Homogeneity** | SEC-HPLC | Not detected |
| % Aggregate | | |
| **Molar Ratio Distribution** | HIC-HPLC | 3.5% 0 drugs/Ab |
| % of total | | 19.4% 2 drugs/Ab |
| | | 39.6% 4 drugs/Ab |
| | | 21.0% 6 drugs/Ab |
| | | 11.7% 8 drugs/Ab |
| **Denatured Antibody** | PLRP-HPLC | 31.3% L0 |
| | | 68.7% L1 |
| | | 10.7% H0 |
| | | 40.4% H1 |
| | | 25.1% H2 |
| | | 23.7%H3 |

**Table 5**

| Conjugate Certificate of Testing | | |
|---|---|---|
| **699028B** | | |
| PSMA mAb vcMMAF | | |
| Partial Reduction | | |
| Assay | Method | Result |
| **mAb Concentration** | UV | 3.1 |
| mg/mL | | |
| **Drug/mAb** | H/L-Chain Loading Distribution (PLRP) Species Distribution (HIC) | 4.4 |
| mol/mol | | |
| **Unconjugated Drug** | RP-HPLC | <0.5 |
| % of total drug | | |
| **Size Homogeneity** | SEC-HPLC | Not detected |
| % Aggregate | | |
| **Molar Ratio Distribution** | HIC-HPLC | 3.3% 0 drugs/Ab |
| % of total | | 18.5% 2 drugs/Ab |
| | | 39.0% 4 drugs/Ab |
| | | 22.2% 8 drugs/Ab |
| | | 13.5% 8 drugs/Ab |
| **Denatured Antibody** | PLRP-HPLC | 29.0% L0 |
| | | 71.0% L1 |
| | | 9.9% H0 |
| | | 40.2% H1 |
| | | 24.9% H2 |
| | | 25.0%H3 |

**Table 6**

| Conjugate Certificate of Testing | | |
|---|---|---|
| **699028C** | | |
| PSMA mAb mcMMAF | | |
| Partial Reduction | | |
| Assay | Method | Result |
| **mAb Concentration** | UV | 3.7 |
| mg/mL | | |
| **Drug/mAb** | H/L-Chain Loading Distribution (PLRP) Species Distribution (HIC) | 4.4 |
| mol/mol | | |
| **Unconjugated Drug** | RP-HPLC | 1.8 |
| % of total drug | | |
| **Size Homogeneity** | SEC-HPLC | Not detected |
| % Aggregate | | |
| **Molar Ratio Distribution** | HIC-HPLC | 3.8% 0 drugs/Ab |
| % of total | | 22.4% 2 drugs/Ab |
| | | 36.3% 4 drugs/Ab |
| | | 23.1% 6 drugs/Ab |
| | | 14.4% 8 drugs/Ab |
| **Denatured Antibody** | PLRP-HPLC | 32.7% L0 |
| | | 67.3% L1 |
| | | 14.7% H0 |
| | | 39.6% H1 |
| | | 23.8% H2 |
| | | 21.9%H3 |

### Potency and Selectivity of PSMA mAb Conjugates on Human Prostate Cancer Cells

*In vitro* cytotoxicity studies were conducted with PSMA-positive and PSMA-negative cell lines. Briefly, PSMA-positive cells (C4-2, LNCaP™ or 3T3™-PSMA) and PSMA-negative cells (PC-3™ or 3T3™) were added to 96-well microplates at 2.5 x 10³ cells/well and incubated overnight at 37°C and 5% CO₂. Cells were then incubated with serially diluted ADCs for 4 days and assayed for percent cell kill compared to untreated controls using 10% Alamar Blue. The concentration of ADCs required for 50% cell kill (IC₅₀ value) was determined.

**Fig. 7** illustrates dose-response curves of vcMMAE (**Fig. 7A**), vcMMAF (**Fig. 7B**) and mcMMAF (**Fig. 7C**) conjugates for PSMA-positive C4-2 cells and PSMA-negative PC-3™ cells in a representative experiment. A summary of the potency (IC₅₀) and selectivity on C4-2 and PC-3™ cell lines is listed in **Table 7**. The IC₅₀s on PSMA-expressing C4-2 cells were at picomolar concentrations of 11, 42, and 60 for vcMMAF, mcMMAF and vcMMAE conjugates, respectively. In contrast, the **IC₅₀s** on PC-3™ PSMA-negative cells were greater than 90 nM ranging from 94 to 264 nM. Based on the potency of each conjugate on PC-3™ and C4-2, the selectivity was calculated to be 13,636; 6,286 and 1,567 for vcMMAF, mcMMAF and vcMMAE conjugates, respectively. The vcMMAF conjugate was the most potent on the C4-2 PSMA positive cell line, and the mcMMAF was the least toxic over the PC-3™ control cell line. Compared to the vcMMAE conjugate, there was a 4-fold and 9-fold improvement in selectivity for mcMMAF and vcMMAF conjugates, respectively.

**Table 7: Summary of in vitro potency (IC₅₀ values in pM) and selectivity**

| **Drug-linker** | **Potency (pM)** | | **Selectivity (PC-3/C4-2)** | **Improvement over vcMMAE** |
|---|---|---|---|---|
| | **C4-2 (n=3)** | **PC-3 (n=2)** | | |
| vcMMAF | 11 | 150,000 | 13636 | 9-fold |
| mcMMAF | 42 | 264,000 | 6286 | 4-fold |
| vcMMAE | 60 | 94,000 | 1567 | - |

### Mechanism of Cell Killing by the PSMA mAb Drug Conjugate

Cell-cycle analysis was performed to determine the mechanism of cytotoxicity mediated by MMAE-conjugated mAb. 3T3™-PSMA or C4-2 cells were cultured in the presence of 0.2 nM PSMA ADC or 20 nM unmodified PSMA mAb. Untreated cells served as a control culture. At 12h, 24h and 48h, cells were stained with propidium iodide (PI) to detect total DNA and analyzed by flow cytometry. As indicated in **Fig. 8**, cells treated with PSMA ADC were arrested in G₂ phase. By 48h post-treatment, the percent of cells with a duplicate set of chromosomes was >50% for the PSMA ADC cultures and 2% for untreated cultures. Cell-cycle arrest required the presence of the toxin, in this case MMAE, as only 3% of cells treated with unmodified mAb were in G₂/M phase at 48h. The data demonstrate that treatment of prostate cancer cells with MMAF ADCs lead to G₂/M arrest and then apoptosis of target cells.

## Claims

1. An antibody-drug conjugate comprising:
an antibody or antigen-binding fragment thereof, which binds to prostate-specific membrane antigen (PSMA), conjugated to monomethylauristatin norephedrine or monomethylauristatin phenylalanine, wherein the antibody-drug conjugate has a PC3 cell to C4-2 or LNCaP cell selectivity of at least 250, and 3 or 4 molecules of monomethylauristatin norephedrine or monomethylauristatin phenylalanine are conjugated to the antibody or antigen-binding fragment thereof.

2. The antibody-drug conjugate of claim 1, wherein the antibody or antigen-binding fragment thereof is a monoclonal antibody or antigen-binding fragment thereof that specifically binds:
(a) PSMA;
(b) an extracellular domain of PSMA; or
(c) to a conformational epitope of PSMA.

3. The antibody-drug conjugate of claim 1, wherein the antibody is encoded by a nucleic acid molecule comprising a nucleotide sequence that is at least 90, 95, 97, 98 or 99% identical to a nucleotide sequence encoding an antibody selected from the group consisting of: AB-PGI-XG1-006, AB-PGI-XG1-026 and antibodies comprising:
(a) a heavy chain encoded by a nucleic acid molecule comprising the coding region or regions of a nucleotide sequence selected from the group consisting of nucleotide sequences set forth as SEQ ID NOs: 2 and 3, and
(b) a light chain encoded by a nucleic acid molecule comprising the coding region or regions of a nucleotide sequence selected from the group consisting of nucleotide sequences set forth as SEQ ID NOs: 8 and 9, or
(c) a heavy chain variable region encoded by a nucleic acid molecule comprising the coding region or regions of a nucleotide sequence selected from the group consisting of nucleotide sequences set forth as SEQ ID NOs: 14 and 18, and
(d) a light chain variable region encoded by a nucleic acid molecule comprising the coding region or regions of a nucleotide sequence selected from the group consisting of nucleotide sequences set forth as SEQ ID NOs: 16 and 20.

4. The antibody-drug conjugate of claim 1, wherein the antibody or antigen-binding fragment thereof is AB-PGI-XG1-006, A13-PGI-XG1-026 or an antigen-binding fragment thereof.

5. The antibody-drug conjugate of claim 1, wherein the antibody or antigen-binding fragment thereof is selected from the group consisting of:
(a) antibodies comprising a heavy chain encoded by a nucleic acid molecule comprising the coding region or regions of a nucleotide sequence selected from the group consisting of nucleotide sequences set forth as SEQ ID NOs: 2 and 3, and a light chain encoded by a nucleic acid molecule comprising the coding region or regions of a nucleotide sequence selected from the group consisting of nucleotide sequences set forth as SEQ ID NOs: 8 and 9, and
antigen-binding fragments thereof;
(b) antibodies comprising a heavy chain encoded by a nucleic acid molecule comprising the coding region or regions of a nucleotide sequence set forth as SEQ ID NO: 2, and a light chain encoded by a nucleic acid molecule comprising the coding region or regions of a nucleotide sequence set forth as SEQ ID NO: 8, and
antigen-binding fragments thereof; and
(c) antibodies comprising a heavy chain encoded by a nucleic acid molecule comprising the coding region or regions of a nucleotide sequence set forth as SEQ ID NO: 3, and a light chain encoded by a nucleic acid molecule comprising the coding region or regions of a nucleotide sequence set forth as SEQ ID NO: 9, and
antigen-binding fragments thereof; and
(d) antibodies comprising a heavy chain variable region encoded by a nucleic acid molecule comprising the coding region or regions of a nucleotide sequence selected from the group consisting of nucleotide sequences set forth as SEQ ID NOs: 14 and 18, and a light chain variable region encoded by a nucleic acid molecule comprising the coding region or regions of a nucleotide sequence selected from the group consisting of nucleotide sequences set forth as SEQ ID NOs: 16 and 20, and
antigen-binding fragments thereof; and
(e) antibodies comprising a heavy chain variable region encoded by a nucleic acid molecule comprising the coding region or regions of a nucleotide sequence set forth as SEQ ID NO: 14, and a light chain variable region encoded by a nucleic acid molecule comprising the coding region or regions of a nucleotide sequence set forth as SEQ ID NO: 16, and
antigen-binding fragments thereof; and
(f) antibodies comprising a heavy chain variable region encoded by a nucleic acid molecule comprising the coding region or regions of a nucleotide sequence set forth as SEQ ID NO: 18, and a light chain variable region encoded by a nucleic acid molecule comprising the coding region or regions of a nucleotide sequence set forth as SEQ ID NO: 20, and
antigen-binding fragments thereof.

6. The antibody-drug conjugate of claim 1, wherein the antibody or antigen-binding fragment thereof is IgG1, IgG2, IgG3, IgG4, IgM, IgA1, IgA2, IgAsec, IgD, IgE or has immunoglobulin constant and/or variable domain of IgG1, IgG2, IgG3, IgG4, IgM, IgA1, IgA2, IgAsec, IgD or IgE.

7. The antibody-drug conjugate of claim 1, wherein the antibody is a monoclonal, humanized, human, recombinant, chimeric, bispecific or multispecific antibody.

8. The antibody-drug conjugate of claim 1, wherein the antigen-binding fragment is a Fab fragment, a F(ab')₂ fragment, a Fv fragment, or a CDR3-containing fragment.

9. The antibody-drug conjugate of claim 1, wherein the PC-3 cell to C4-2 or LNCaP cell selectivity is at least 500, 1000, 2500, 6000 or 13,000.

10. The antibody-drug conjugate of claim 1, wherein the monomethylauristatin norephedrine or monomethylauristatin phenylalanine is conjugated to the antibody or antigen-binding fragment thereof with a compound of the formula:
-Aₙ-Yₘ-Zₘ-Xₙ-Wₙ-
wherein, A is a carboxylic acyl unit; Y is an amino acid; Z is an amino acid; X and W are each a self-immolative spacer; n is an integer of 0 or 1; and m is an integer of 0 or 1, 2, 3, 4, 5 or 6.

11. The antibody-drug conjugate of claim 10, wherein A is
(a) 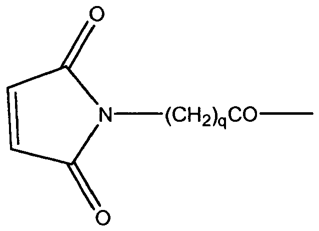 in which q is 1-10,
(b) 4- (N-succinimidomethyl) cyclohexane-1-carbonyl,
(c) m-succinimidobenzoyl,
(d) 4-(p-succinimidophenyl)-butyryl,
(e) 4-(2-acetamido) benzoyl,
(f) 3-thiopropionyl,
(g) 4-(1-thioethyl)-benzoyl,
(h) 6-(3-thiopropionylamido)-hexanoyl, or
(i) maleimide caproyl.

12. The antibody-drug conjugate of claim 10, wherein Y is alanine, valine, leucine, isoleucine, methionine, phenylalanine, tryptophan or proline.

13. The antibody-drug conjugate of claim 10, wherein Z is lysine, lysine protected with acetyl or formyl, arginine, arginine protected with tosyl or nitro groups, histidine, ornithine, ornithine protected with acetyl or formyl, or citrulline.

14. The antibody-drug conjugate of claim 10, wherein Yₘ-Zₘ is valine-citrulline.

15. The antibody-drug conjugate of claim 10, wherein Yₘ-Zₘ is a protein sequence which is selectively cleavable by a protease.

16. The antibody-drug conjugate of claim 10, wherein X is:
(a) a compound having the formula 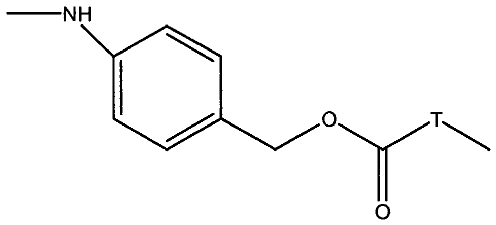 in which T is O, N, or S;
(b) a compound having the formula
-HN-R¹-COT
in which R¹ is C₁-C₅ alkyl, T is O, N or S;
(c) a compound having the formula 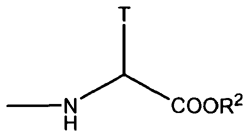 in which T is O, N, or S, R² is H or C₁-C₅ alkyl;
(d) p-aminobenzylcarbamoyloxy;
(e) p-aminobenzylalcohol;
(f) p-aminobenzylcarbamate;
(g) p-aminobenzyloxycarbonyl; or
(h) γ-aminobutyric acid; α,α-dimethyl γ-aminobutyric acid or β,β-dimethyl γ-aminobutyric acid.

17. The antibody-drug conjugate of claim 10, wherein W is 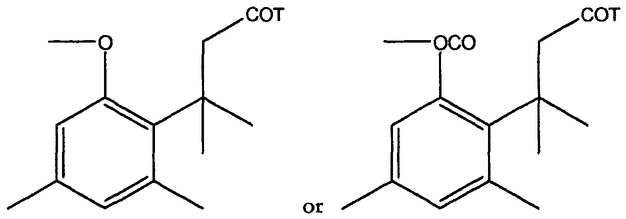 in which T is O, S or N.

18. The antibody-drug conjugate of any of claims 10-17, wherein m and n are 0.

19. The antibody-drug conjugate of claim 1, which is AB-PG1-XG1-006-maleimide caproyl-valine-citrulline-p-aminobenzyloxycarbonyl-monomethylauristatin norephedrine, AB-PG 1-XG1-006-maleimide caproyl-valine-citrulline-p-aminobenzyloxycarbonyl-monomethylauristatin phenylalanine, AB-PG1-XG1-006-maleimide caproyl-monomethylauristatin phenylalanine, AB-PG1-XG1-006-maleimide caproyl-valine-citrulline-p-aminobenzylcarbamate-monomethylauristatin norephedrine, AB-PG1-XG1-006-maleimide caproyl-valine-citrulline-p-aminobenzylcarbamate-monomethylauristatin phenylalanine, AB-PG1-X1-026-maleimide caproyl-valine-citrulline-p-aminobenzyloxycarbonyl-monomethylauristatin norephedrine, AB-PG1-XG1-026-maleimide caproyl-valine-citnzlline-p-aminobenzyloxycarbonyl-monomethylauristatin phenylalanine, or AB-PG1-XG1-026-maleimide caproyl-monomethylauristatin phenylalanine.

20. The antibody-drug conjugate of claim 1, wherein the antibody-drug conjugate binds a tumor cell, preferably a prostate tumor cell.

21. The antibody-drug conjugate of claim 1, wherein the antibody-drug conjugate binds endothelial cells of the neovasculature of a tumor, does not require cell lysis to bind PSMA, leads to cell-cycle arrest, and/or inhibits the growth of PSMA-expressing cells.

22. The antibody-drug conjugate of claim 1, wherein the antibody-drug conjugate mediates specific cell killing of PSMA-expressing cells with an IC₅₀ of:
(a) less than 1X10⁻¹⁰, 1X10⁻¹¹ or 1X10⁻¹²M;
(b) 11-, 42-, 60- or 65 - 208 X10⁻¹²M; or
(c) 11, 42, 60 or 83 X10⁻¹²M.

23. The antibody-drug conjugate of claim 1, wherein the antibody-drug conjugate when administered to mice with a regimen of q4d x 6 at a dose of 6 mg/kg effects a cure rate of at least 20%, 30%, 40% or 50%.

24. The antibody-drug conjugate of claim 1, bound to a label, preferably a fluorescent label, an enzyme label, a radioactive label, a nuclear magnetic resonance active label, a luminescent label or a chromophore label.

25. The antibody-drug conjugate of claim 1, packaged in lyophilized or aqueous form.

26. The antibody-drug conjugate of claim 1, in a sterile form.

27. A composition comprising:
(a) the antibody-drug conjugate of claim 1 and a pharmaceutically acceptable carrier, excipient or stabilizer; or
(b) a combination of two or more different antibody-drug conjugates according to claim 1 and a pharmaceutically acceptable carrier, excipient or stabilizer.

28. The composition of claim 27, further comprising an antitumor agent, an immunostimulatory agent, an immunomodulator, corticosteroid or a combination thereof.

29. The composition of claim 28, wherein the antitumor agent is a cytotoxic agent, an agent that acts on tumor neovasculature or a combination thereof, preferably docetaxel.

30. The composition of claim 28, wherein the immunomodulator is a cytokine, chemokine, adjuvant or a combination thereof.

31. The composition of claim 28, wherein the immunostimulatory agent is interleukin-2, α-interferon, γ-interferon, tumor necrosis factor-α, an immunostimulatory oligonucleotide or a combination thereof.

32. The composition of claim 28, wherein the corticosteroid is prednisone or hydrocortisone.

33. A composition comprising:
one or more antibody-drug conjugates of claim 1 and one or more unconjugated anti-PSMA antibodies.

34. An antibody-drug conjugate as claimed in any of claims 1-23, for use in a method of inhibiting the growth of a PSMA-expressing cell, wherein the conjugate is to be contacted with the PSMA-expressing cell.

35. An antibody-drug conjugate as claimed in claim 34, wherein the PSMA-expressing cell is a prostate tumor cell, a cell of the neovasculature of a non-prostate tumor, an androgen dependent-cell or an androgen-independent cell.

36. An antibody-drug conjugate as claimed in claim 34 wherein, in said method, the PSMA-expressing cell is further to be contacted with an antitumor agent, an immunostimulatory agent, an immunomodulator, corticosteroid or a combination thereof.

37. An *ex vivo* method for inhibiting the growth of a PSMA-expressing cell comprising contacting the PSMA-expressing cell with an amount of an antibody-drug conjugate as claimed in any of claims 1-23, effective to inhibit the growth of the PSMA-expressing cell.

38. A method as claimed in claim 37, wherein the PSMA-expressing cell is a prostate tumor cell, a cell of the neovasculature of a non-prostate tumor, an androgen-dependent cell or an androgen-independent cell.

39. A method as claimed in claim 37, further comprising contacting the PSMA-expressing cell with an anti-tumor agent, an immunostimulatory agent, an immunomodulator, corticosteroid, or a combination thereof.

40. An antibody-drug conjugate as claimed in any of claims 1-23, for use in a method of effecting cell-cycle arrest in a PSMA-expressing cell, wherein the conjugate is to be contacted with the PSMA-expressing cell.

41. An antibody-drug conjugate as claimed in any of claims 1-23, for use in a method of treating cancer, optionally prostate cancer or a non-prostate cancer, and said non-prostate cancer, optionally, is bladder cancer, pancreatic cancer, lung cancer, kidney cancer, sarcoma, breast cancer, brain cancer, neuroendocrine carcinoma, colon cancer, testicular cancer or melanoma.

42. An antibody-drug conjugate for use in a method of treating cancer as claimed in claim 41, wherein, in said method, another therapeutic agent is to be co-administered to treat the cancer, wherein the co-administration is to be before, during or after the administration of the antibody-drug conjugate.

43. An antibody-drug conjugate for use in a method of treating cancer as claimed in claim 42, wherein the other therapeutic agent is an antitumor agent, an immunostimulatory agent, an immunomodulator, corticosteroid or a combination thereof

44. An antibody-drug conjugate for use in a method of inhibiting the growth of a PSMA-expressing cell as claimed in claim 36, an antibody-drug conjugate for use in a method of treating cancer as claimed in claim 43, or a method as claimed in claim 39, wherein the antitumor agent is a cytotoxic agent, an agent that acts on tumor neovasculature or a combination thereof, and, preferably, docetaxel.

45. An antibody-drug conjugate for use in a method of inhibiting the growth of a PSMA-expressing cell as claimed in claim 36, an antibody-drug conjugate for use in a method of treating cancer as claimed in claim 43, or a method as claimed in claim 39, wherein the immunomodulator is a cytokine, chemokine, adjuvant or a combination thereof.

46. An antibody-drug conjugate for use in a method of inhibiting the growth of a PSMA-expressing cell as claimed in claim 36, an antibody-drug conjugate for use in a method of treating cancer as claimed in claim 43, or a method as claimed in claim 39, wherein the immunostimulatory agent is interleukin-2, α-interferon, γ-interferon, tumor necrosis factor-α, an immunostimulatory oligonucleotide, or a combination thereof.

47. An antibody-drug conjugate for use in a method of inhibiting the growth of a PSMA-expressing cell as claimed in claim 36, an antibody-drug conjugate for use in a method of treating cancer as claimed in claim 43, or a method as claimed in claim 39, wherein the corticosteroid is prednisone or hydrocortisone.

48. An antibody-drug conjugate for use in a method of treating cancer as claimed in claim 42, wherein the therapeutic agent is a vaccine, preferably a vaccine that immunizes against PSMA.

49. An antibody-drug conjugate for use in a method of inhibiting the growth of a PSMA-expressing cell as claimed in claim 44, or an antibody-drug conjugate for use in a method of treating cancer as claimed in any one of claims 42-44, wherein, in said method, still another therapeutic agent, preferably prednisone, is to be administered.

50. An antibody-drug conjugate as claimed in any of claims 1-23, for use in a method of inhibiting the growth of a tumor, wherein said conjugate is to be contacted with PSMA-expressing cells of the neovasculature of the tumor, thereby to inhibit the growth of the tumor.

51. An antibody-drug conjugate for use in a method of inhibiting the growth of a tumor as claimed in claim 50 wherein, in said method, the PSMA-expressing cells are to be further contacted with an antitumor agent, an immunostimulatory agent, an immunomodulator, corticosteroid or a combination thereof.

52. An antibody-drug conjugate for use in a method of inhibiting the growth of a tumor as claimed in claim 51, wherein:
(a) the antitumor agent is a cytotoxic agent, an agent that acts on tumor neovasculature or a combination thereof;
(b) the immunomodulator is a cytokine, chemokine, adjuvant or a combination thereof; and/or
(c) the immunostimulatory agent is interleukin-2, α-interferon, γ-interferon, tumor
necrosis factor-α, immunostimulatory oligonucleotides or a combination thereof.

## Patentansprüche

1. Antikörper-Wirkstoff-Konjugat, das Folgendes umfasst:
einen Antikörper oder ein antigenbindendes Fragment davon, der bzw. das an prostataspezifisches Membranantigen (PSMA) bindet und an Monomethylauristatin-Norephedrin oder
Monomethylauristatin-Phenylalanin konjugiert ist, wobei das Antikörper-Wirkstoff-Konjugat eine Selektivität von PC3-Zellen zu C4-2- oder LNCaP-Zellen von mindestens 250 aufweist und 3 oder 4 Moleküle von Monomethylauristatin-Norephedrin oder Monomethylauristatin-Phenylalanin an den Antikörper oder das antigenbindende Fragment davon konjugiert sind.

2. Antikörper-Wirkstoff-Konjugat nach Anspruch 1, wobei der Antikörper oder das antigenbindende Fragment davon ein monoklonaler Antikörper oder ein antigenbindendes Fragment davon ist, der bzw. das spezifisch an Folgendes bindet:
(a) PSMA;
(b) eine extrazelluläre Domäne von PSMA oder
(c) an ein Konformationsepitop von PSMA.

3. Antikörper-Wirkstoff-Konjugat nach Anspruch 1, wobei der Antikörper von einem Nukleinsäuremolekül codiert wird, das eine Nukleotidsequenz umfasst, die zu mindestens 90, 95, 97, 98 oder 99 % zu einer Nukleotidsequenz identisch ist, die einen Antikörper codiert, der aus der Gruppe bestehend aus folgenden ausgewählt ist: AB-PG1-XG1-006, AB-PG1-XG1-026 und Antikörpern,, die Folgendes umfassen:
(a) eine schwere Kette, die von einem Nukleinsäuremolekül codiert wird, das die codierende Region oder Regionen einer Nukleotidsequenz umfasst, die aus der Gruppe bestehend aus als SEQ ID Nr. 2 und 3 dargelegten Nukleotidsequenzen ausgewählt ist, und
(b) eine leichte Kette, die von einem Nukleinsäuremolekül codiert wird, das die codierende Region oder Regionen einer Nukleotidsequenz umfasst, die aus der Gruppe bestehend aus als SEQ ID Nr. 8 und 9 dargelegten Nukleotidsequenzen ausgewählt ist, oder
(c) eine variable Region einer schweren Kette, die von einem Nukleinsäuremolekül codiert wird, das die codierende Region oder Regionen einer Nukleotidsequenz umfasst, die aus der Gruppe bestehend aus als SEQ ID Nr. 14 und 18 dargelegten Nukleotidsequenzen ausgewählt ist, und
(d) eine variable Region einer leichten Kette, die von einem Nukleinsäuremolekül codiert wird, das die codierende Region oder Regionen einer Nukleotidsequenz umfasst, die aus der Gruppe bestehend aus als SEQ ID Nr. 16 und 20 dargelegten Nukleotidsequenzen ausgewählt ist.

4. Antikörper-Wirkstoff-Konjugat nach Anspruch 1, wobei der Antikörper oder das antigenbindende Fragment davon AB-PG1-XG1-006, AB-PG1-XG1-026 oder ein antigenbindendes Fragment davon ist.

5. Antikörper-Wirkstoff-Konjugat nach Anspruch 1, wobei der Antikörper oder das antigenbindende Fragment davon aus der Gruppe bestehend aus folgenden ausgewählt ist:
(a) Antikörpern, die eine schwere Kette, die von einem Nukleinsäuremolekül codiert wird, das die codierende Region oder Regionen einer Nukleotidsequenz umfasst, die aus der Gruppe bestehend aus als SEQ ID Nr. 2 und 3 dargelegten Nukleotidsequenzen ausgewählt ist, und eine leichte Kette umfassen, die von einem Nukleinsäuremolekül codiert wird, das die codierende Region oder Regionen einer Nukleotidsequenz umfasst, die aus der Gruppe bestehend aus als SEQ ID Nr. 8 und 9 dargelegten Nukleotidsequenzen ausgewählt ist, und
antigenbindenden Fragmenten davon;
(b) Antikörpern, die eine schwere Kette, die von einem Nukleinsäuremolekül codiert wird, das die codierende Region oder Regionen einer als SEQ ID Nr. 2 dargelegten Nukleotidsequenz umfasst, und eine leichte Kette umfassen, die von einem Nukleinsäuremolekül codiert wird, das die codierende Region oder Regionen einer als SEQ ID Nr. 8 dargelegten Nukleotidsequenz umfasst, und
antigenbindenden Fragmenten davon; und
(c) Antikörpern, die eine schwere Kette, die von einem Nukleinsäuremolekül codiert wird, das die codierende Region oder Regionen einer als SEQ ID Nr. 3 dargelegten Nukleotidsequenz umfasst, und eine leichte Kette umfassen, die von einem Nukleinsäuremolekül codiert wird, das die codierende Region oder Regionen einer als SEQ ID Nr. 9 dargelegten Nukleotidsequenz umfasst, und
antigenbindenden Fragmenten davon; und
(d) Antikörpern, die eine variable Region einer schweren Kette, die von einem Nukleinsäuremolekül codiert wird, das die codierende Region oder Regionen einer Nukleotidsequenz umfasst, die aus der Gruppe bestehend aus als SEQ ID Nr. 14 und 18 dargelegten Nukleotidsequenzen ausgewählt ist, und eine variable Region einer leichten Kette umfassen, die von einem Nukleinsäuremolekül codiert wird, das die codierende Region oder Regionen einer Nukleotidsequenz umfasst, die aus der Gruppe bestehend aus als SEQ ID Nr. 16 und 20 dargelegten Nukleotidsequenzen ausgewählt ist, und antigenbindenden Fragmenten davon; und
(e) Antikörpern, die eine variable Region einer schweren Kette, die von einem Nukleinsäuremolekül codiert wird, das die codierende Region oder Regionen einer als SEQ ID Nr. 14 dargelegten Nukleotidsequenz umfasst, und eine variable Region einer leichten Kette umfassen, die von einem Nukleinsäuremolekül codiert wird, das die codierende Region oder Regionen einer als SEQ ID Nr. 16 dargelegten Nukleotidsequenz umfasst, und
antigenbindenden Fragmenten davon; und
(f) Antikörpern, die eine variable Region einer schweren Kette, die von einem Nukleinsäuremolekül codiert wird, das die codierende Region oder Regionen einer als SEQ ID Nr. 18 dargelegten Nukleotidsequenz umfasst, und eine variable Region einer leichten Kette umfassen, die von einem Nukleinsäuremolekül codiert wird, das die codierende Region oder Regionen einer als SEQ ID Nr. 20 dargelegten Nukleotidsequenz umfasst, und
antigenbindenden Fragmenten davon.

6. Antikörper-Wirkstoff-Konjugat nach Anspruch 1, wobei der Antikörper oder das antigenbindende Fragment davon IgG1, IgG2, IgG3, IgG4, IgM, IgA1, IgA2, IgAsec, IgD, IgE ist oder eine konstante und/oder variable Immunglobulin-Region von IgG1, IgG2, IgG3, IgG4, IgM, IgA1, IgA2, IgAsec, IgD oder IgE aufweist.

7. Antikörper-Wirkstoff-Konjugat nach Anspruch 1, wobei der Antikörper ein monoklonaler, humanisierter, humaner, rekombinanter, chimärer, bispezifischer oder multispezifischer Antikörper ist.

8. Antikörper-Wirkstoff-Konjugat nach Anspruch 1, wobei das antigenbindende Fragment ein Fab-Fragment, ein F(ab')₂-Fragment, ein Fv-Fragment oder ein CDR3 enthaltendes Fragment ist.

9. Antikörper-Wirkstoff-Konjugat nach Anspruch 1, wobei die Selektivität von PC3-Zellen zu C4-2- oder LNCaP-Zellen mindestens 500, 1000, 2500, 6000 oder 13.000 beträgt.

10. Antikörper-Wirkstoff-Konjugat nach Anspruch 1, wobei das Monomethylauristatin-Norephedrin oder Monomethylauristatin-Phenylalanin an den Antikörper oder das antigenbindende Fragment davon mit einer Verbindung der folgenden Formel konjugiert ist:
-Aₙ-Yₘ-Zₘ-Xₙ-Wₙ-,
wobei A eine Carboxylacyl-Einheit ist; Y eine Aminosäure ist; Z eine Aminosäure ist; X und W jeweils ein selbstimmolativer Spacer sind; n eine ganze Zahl 0 oder 1 ist und m eine ganze Zahl 0 oder 1, 2, 3, 4, 5 oder 6 ist.

11. Antikörper-Wirkstoff-Konjugat nach Anspruch 10, wobei A
(a) 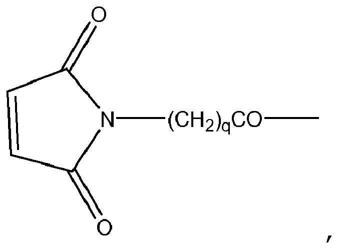 wobei q 1 - 10 ist,
(b) 4-(N-Succinimidomethyl)cyclohexan-1-carbonyl,
(c) m-Succinimidobenzoyl,
(d) 4-(p-Succinimidophenyl)butyryl,
(e) 4-(2-Acetamido)benzoyl,
(f) 3-Thiopropionyl,
(g) 4-(1-Thioethyl)benzoyl,
(h) 6-(3-Thiopropionylamido)hexanoyl oder
(i) Maleinimidcaproyl ist.

12. Antikörper-Wirkstoff-Konjugat nach Anspruch 10, wobei Y Alanin, Valin, Leucin, Isoleucin, Methionin, Phenylalanin, Tryptophan oder Prolin ist.

13. Antikörper-Wirkstoff-Konjugat nach Anspruch 10, wobei Z Lysin, mit Acetyl oder Formyl geschütztes Lysin, Arginin, mit Tosyl oder Nitrogruppen geschütztes Arginin, Histidin, Ornithin, mit Acetyl oder Formyl geschütztes Ornithin oder Citrullin ist.

14. Antikörper-Wirkstoff-Konjugat nach Anspruch 10, wobei Yₘ-Zₘ Valin-Citrullin ist.

15. Antikörper-Wirkstoff-Konjugat nach Anspruch 10, wobei Yₘ-Zₘ eine Proteinsequenz ist, die selektiv durch eine Protease spaltbar ist.

16. Antikörper-Wirkstoff-Konjugat nach Anspruch 10, wobei X Folgendes ist:
(a) eine Verbindung mit der Formel 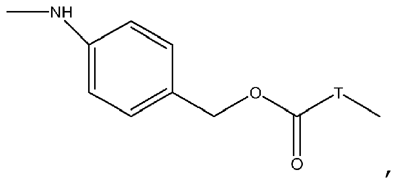 wobei T O, N oder S ist;
(b) eine Verbindung mit der Formel
-HN-R¹-COT,
wobei R¹ C₁-C₅-Alkyl ist, T O, N oder S ist;
(c) eine Verbindung mit der Formel 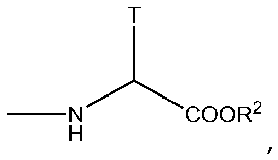 wobei T O, N oder S ist, R² H oder C₁-C₅-Alkyl ist;
(d) p-Aminobenzylcarbamoyloxy;
(e) p-Aminobenzylalkohol;
(f) p-Aminobenzylcarbamat;
(g) p-Aminobenzyloxycarbonyl oder
(h) γ-Aminobuttersäure; α,α-Dimethyl-γ-aminobuttersäure oder β,β-Dimethyl-γ-aminobuttersäure.

17. Antikörper-Wirkstoff-Konjugat nach Anspruch 10, wobei W 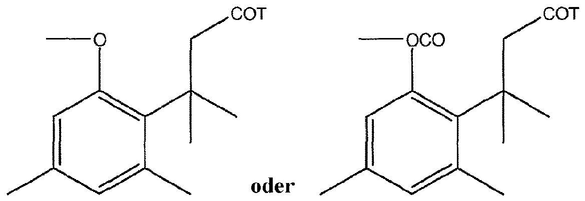 ist, wobei T O, S oder N ist.

18. Antikörper-Wirkstoff-Konjugat nach einem der Ansprüche 10 - 17, wobei m und n 0 sind.

19. Antikörper-Wirkstoff-Konjugat nach Anspruch 1, bei dem es sich um AB-PG1-XG1-006-Maleinimidcaproyl-Valin-Citrullin-p-Aminobenzyloxycarbonyl-Monomethylauristatin-Norephedrin, AB-PG1-XG1-006-Maleinimidcaproyl-Valin-Citrullin-p-Aminobenzyloxycarbonyl-Monomethylauristatin-Phenylalanin, AB-PG1-XG1-006-Maleinimidcaproyl-Monomethylauristatin-Phenylalanin, AB-PG1-XG1-006-Maleinimidcaproyl-Valin-Citrullin-p-Aminobenzylcarbamat-Monomethylauristatin-Norephedrin, AB-PG1-XG1-006-Maleinimidcaproyl-Valin-Citrullin-p-Aminobenzylcarbamat-Monomethylauristatin-Phenylalanin, AB-PG1-XG1-026-Maleinimidcaproyl-Valin-Citrullin-p-Aminobenzyloxycarbonyl-Monomethylauristatin-Norephedrin, AB-PG1-XG1-026-Maleinimidcaproyl-Valin-Citrullin-p-Aminobenzyloxycarbonyl-Monomethylauristatin-Phenylalanin oder AB-PG1-XG1-026-Maleinimidcaproyl-Monomethylauristatin-Phenylalanin handelt.

20. Antikörper-Wirkstoff-Konjugat nach Anspruch 1, wobei das Antikörper-Wirkstoff-Konjugat eine Tumorzelle, vorzugsweise eine Prostatatumorzelle bindet.

21. Antikörper-Wirkstoff-Konjugat nach Anspruch 1, wobei das Antikörper-Wirkstoff-Konjugat Endothelzellen der neuen Blutgefäße eines Tumors bindet, keine Zelllyse benötigt, um PSMA zu binden, zu einem Zellzyklusstopp führt und/oder das Wachstum von PSMA exprimierenden Zellen hemmt.

22. Antikörper-Wirkstoff-Konjugat nach Anspruch 1, wobei das Antikörper-Wirkstoff-Konjugat eine spezifische Zellabtötung von PSMA exprimierenden Zellen mit einem IC₅₀ von:
(a) weniger als 1 x 10⁻¹⁰, 1 x 10⁻¹¹ oder 1 x 10⁻¹² M;
(b) 11 -, 42 -, 60 - oder 65 - 208 x 10⁻¹² M oder
(c) 11, 42, 60 oder 83 x 10⁻¹² M vermittelt.

23. Antikörper-Wirkstoff-Konjugat nach Anspruch 1, wobei das Antikörper-Wirkstoff-Konjugat bei Verabreichung an Mäuse mit einem Therapieplan von 6 x alle 4 Tage mit einer Dosis von 6 mg/kg eine Heilungsrate von mindestens 20 %, 30 %, 40 % oder 50 % bewirkt.

24. Antikörper-Wirkstoff-Konjugat nach Anspruch 1, das an einen Marker, vorzugsweise einen Fluoreszenzmarker, einen Enzymmarker, einen radioaktiven Marker, einen Kernspinresonanz-aktiven Marker, einen Lumineszenzmarker oder einen Chromophormarker gebunden ist.

25. Antikörper-Wirkstoff-Konjugat nach Anspruch 1, das in lyophilisierter oder wässriger Form verpackt ist.

26. Antikörper-Wirkstoff-Konjugat nach Anspruch 1 in steriler Form.

27. Zusammensetzung, die Folgendes umfasst:
(a) das Antikörper-Wirkstoff-Konjugat nach Anspruch 1 und einen pharmazeutisch unbedenklichen Trägerstoff, Hilfsstoff oder Stabilisator oder
(b) eine Kombination von zwei oder mehr unterschiedlichen Antikörper-Wirkstoff-Konjugaten nach Anspruch 1 und einen pharmazeutisch unbedenklichen Trägerstoff, Hilfsstoff oder Stabilisator.

28. Zusammensetzung nach Anspruch 27, die weiterhin eine antineoplastische Substanz, eine immunstimulierende Substanz, einen Immunmodulator, ein Kortikosteroid oder eine Kombination davon umfasst.

29. Zusammensetzung nach Anspruch 28, wobei die antineoplastische Substanz ein Zytotoxikum, eine Substanz, die auf die neuen Blutgefäße des Tumors einwirkt, oder eine Kombination davon, vorzugsweise Docetaxel ist.

30. Zusammensetzung nach Anspruch 28, wobei der Immunmodulator ein Zytokin, ein Chemokin, ein Adjuvans oder eine Kombination davon ist.

31. Zusammensetzung nach Anspruch 28, wobei die immunstimulierende Substanz Interleukin-2, α-Interferon, γ-Interferon, Tumornekrosefaktor-α, ein immunstimulierendes Oligonukleotid oder eine Kombination davon ist.

32. Zusammensetzung nach Anspruch 28, wobei das Kortikosteroid Prednison oder Hydrokortison ist.

33. Zusammensetzung, die Folgendes umfasst:
ein oder mehrere Antikörper-Wirkstoff-Konjugate nach Anspruch 1 und einen oder mehrere unkonjugierte Anti-PSMA-Antikörper.

34. Antikörper-Wirkstoff-Konjugat nach einem der Ansprüche 1 - 23 zur Verwendung in einem Verfahren zum Hemmen des Wachstums einer PSMA exprimierenden Zelle, wobei das Konjugat mit der PSMA exprimierenden Zelle in Kontakt gebracht werden soll.

35. Antikörper-Wirkstoff-Konjugat nach Anspruch 34, wobei die PSMA exprimierende Zelle eine Prostatatumorzelle, eine Zelle der neuen Blutgefäße eines Tumors, bei dem es sich nicht um einen Prostatatumor handelt, eine androgenabhängige Zelle oder eine androgenunabhängige Zelle ist.

36. Antikörper-Wirkstoff-Konjugat nach Anspruch 34, wobei in dem Verfahren die PSMA exprimierende Zelle weiterhin mit einer antineoplastischen Substanz, einer immunstimulierenden Substanz, einem Immunmodulator, einem Kortikosteroid oder einer Kombination davon in Kontakt gebracht werden soll.

37. Ex-vivo-Verfahren zum Hemmen des Wachstums einer PSMA exprimierenden Zelle, das das Inkontaktbringen der PSMA exprimierenden Zelle mit einer Menge eines Antikörper-Wirkstoff-Konjugats nach einem der Ansprüche 1 - 23 umfasst, die dazu wirksam ist, das Wachstum der PSMA exprimierenden Zelle zu hemmen.

38. Verfahren nach Anspruch 37, wobei die PSMA exprimierende Zelle eine Prostatatumorzelle, eine Zelle der neuen Blutgefäße eines Tumors, bei dem es sich nicht um einen Prostatatumor handelt, eine androgenabhängige Zelle oder eine androgenunabhängige Zelle ist.

39. Verfahren nach Anspruch 37, das weiterhin das Inkontaktbringen der PSMA exprimierenden Zelle mit einer antineoplastischen Substanz, einer immunstimulierenden Substanz, einem Immunmodulator, einem Kortikosteroid oder einer Kombination davon umfasst.

40. Antikörper-Wirkstoff-Konjugat nach einem der Ansprüche 1 - 23 zur Verwendung in einem Verfahren zum Bewirken eines Zellzyklusstopps in einer PSMA exprimierenden Zelle, wobei das Konjugat mit der PSMA exprimierenden Zelle in Kontakt gebracht werden soll.

41. Antikörper-Wirkstoff-Konjugat nach einem der Ansprüche 1 - 23 zur Verwendung in einem Verfahren zum Behandeln von Krebs, gegebenenfalls Prostatakrebs oder einer Krebserkrankung, bei der es sich nicht um Prostatakrebs handelt, und wobei die Krebserkrankung, bei der es sich nicht um Prostatakrebs handelt, gegebenenfalls Blasenkrebs, Bauchspeicheldrüsenkrebs, Lungenkrebs, Nierenkrebs, ein Sarkom, Brustkrebs, Hirnkrebs, ein neuroendokrines Karzinom, Darmkrebs, Hodenkrebs oder ein Melanom ist.

42. Antikörper-Wirkstoff-Konjugat zur Verwendung in einem Verfahren zum Behandeln von Krebs nach Anspruch 41, wobei in dem Verfahren ein anderes Therapeutikum in Kombination verabreicht werden soll, um die Krebserkrankung zu behandeln, wobei die Kombinationsverabreichung vor, während oder nach der Verabreichung des Antikörper-Wirkstoff-Konjugats sein soll.

43. Antikörper-Wirkstoff-Konjugat zur Verwendung in einem Verfahren zum Behandeln von Krebs nach Anspruch 42, wobei das andere Therapeutikum eine antineoplastische Substanz, eine immunstimulierende Substanz, ein Immunmodulator, ein Kortikosteroid oder eine Kombination davon ist.

44. Antikörper-Wirkstoff-Konjugat zur Verwendung in einem Verfahren zum Hemmen des Wachstums einer PSMA exprimierenden Zelle nach Anspruch 36, Antikörper-Wirkstoff-Konjugat zur Verwendung in einem Verfahren zum Behandeln von Krebs nach Anspruch 43 oder Verfahren nach Anspruch 39, wobei die antineoplastische Substanz ein Zytotoxikum, eine Substanz, die auf die neuen Blutgefäße des Tumors einwirkt, oder eine Kombination davon, vorzugsweise Docetaxel ist.

45. Antikörper-Wirkstoff-Konjugat zur Verwendung in einem Verfahren zum Hemmen des Wachstums einer PSMA exprimierenden Zelle nach Anspruch 36, Antikörper-Wirkstoff-Konjugat zur Verwendung in einem Verfahren zum Behandeln von Krebs nach Anspruch 43 oder Verfahren nach Anspruch 39, wobei der Immunmodulator ein Zytokin, ein Chemokin, ein Adjuvans oder eine Kombination davon ist.

46. Antikörper-Wirkstoff-Konjugat zur Verwendung in einem Verfahren zum Hemmen des Wachstums einer PSMA exprimierenden Zelle nach Anspruch 36, Antikörper-Wirkstoff-Konjugat zur Verwendung in einem Verfahren zum Behandeln von Krebs nach Anspruch 43 oder Verfahren nach Anspruch 39, wobei die immunstimulierende Substanz Interleukin-2, α-Interferon, γ-Interferon, Tumornekrosefaktor-α, ein immunstimulierendes Oligonukleotid oder eine Kombination davon ist.

47. Antikörper-Wirkstoff-Konjugat zur Verwendung in einem Verfahren zum Hemmen des Wachstums einer PSMA exprimierenden Zelle nach Anspruch 36, Antikörper-Wirkstoff-Konjugat zur Verwendung in einem Verfahren zum Behandeln von Krebs nach Anspruch 43 oder Verfahren nach Anspruch 39, wobei das Kortikosteroid Prednison oder Hydrokortison ist.

48. Antikörper-Wirkstoff-Konjugat zur Verwendung in einem Verfahren zum Behandeln von Krebs nach Anspruch 42, wobei das Therapeutikum ein Impfstoff, vorzugsweise ein Impfstoff, der gegen PSMA immunisiert, ist.

49. Antikörper-Wirkstoff-Konjugat zur Verwendung in einem Verfahren zum Hemmen des Wachstums einer PSMA exprimierenden Zelle nach Anspruch 44 oder Antikörper-Wirkstoff-Konjugat zur Verwendung in einem Verfahren zum Behandeln von Krebs nach einem der Ansprüche 42 - 44, wobei in dem Verfahren noch ein anderes Therapeutikum, vorzugsweise Prednison, verabreicht werden soll.

50. Antikörper-Wirkstoff-Konjugat nach einem der Ansprüche 1 - 23 zur Verwendung in einem Verfahren zum Hemmen des Wachstums eines Tumors, wobei das Konjugat mit PSMA exprimierenden Zellen der neuen Blutgefäße des Tumors in Kontakt gebracht werden soll, um das Wachstum des Tumors zu hemmen.

51. Antikörper-Wirkstoff-Konjugat zur Verwendung in einem Verfahren zum Hemmen des Wachstums eines Tumors nach Anspruch 50, wobei in dem Verfahren die PSMA exprimierenden Zellen weiterhin mit einer antineoplastischen Substanz, einer immunstimulierenden Substanz, einem Immunmodulator, einem Kortikosteroid oder einer Kombination davon in Kontakt gebracht werden sollen.

52. Antikörper-Wirkstoff-Konjugat zur Verwendung in einem Verfahren zum Hemmen des Wachstums eines Tumors nach Anspruch 51, wobei:
(a) die antineoplastische Substanz ein Zytotoxikum, eine Substanz, die auf die neuen Blutgefäße des Tumors einwirkt, oder eine Kombination davon ist;
(b) der Immunmodulator ein Zytokin, ein Chemokin, ein Adjuvans oder eine Kombination davon ist und/oder
(c) die immunstimulierende Substanz Interleukin-2, α-Interferon, γ-Interferon, Tumornekrosefaktor-α, ein immunstimulierende Oligonukleotide oder eine Kombination davon ist.

## Revendications

1. Conjugué anticorps - médicament comprenant :
un anticorps ou un fragment de liaison à l'antigène de celui-ci, qui se lie à l'antigène membranaire spécifique de la prostate (PSMA), conjugué à la monométhylauristatine noréphédrine ou à la monométhylauristatine phénylalanine,
dans lequel le conjugué anticorps - médicament présente une sélectivité des cellules PC3 pour C4-2 ou des cellules LNCaP d'au moins 250, et 3 ou 4 molécules de monométhylauristatine noréphédrine ou de monométhylauristatine phénylalanine sont conjuguées à l'anticorps ou à un fragment de liaison à l'antigène de celui-ci.

2. Conjugué anticorps - médicament selon la revendication 1, dans lequel l'anticorps ou le fragment de liaison à l'antigène de celui-ci est un anticorps monoclonal ou un fragment de liaison à l'antigène de celui-ci qui se lie spécifiquement à :
(a) PSMA ;
(b) un domaine extracellulaire de PSMA ; ou
(c) un épitope conformationnel de PSMA.

3. Conjugué anticorps - médicament selon la revendication 1, dans lequel l'anticorps est codé par une molécule d'acide nucléique comprenant une séquence nucléotidique qui est au moins 90, 95, 97, 98 ou 99 % identique à une séquence nucléotidique codant pour un anticorps sélectionné parmi le groupe constitué de : AB-PG1-XG1-006, AB-PG1-XG1-026 et des anticorps comprenant :
(a) une chaîne lourde codée par une molécule d'acide nucléique comprenant la région ou les régions codante(s) d'une séquence nucléotidique sélectionnée parmi le groupe constitué des séquences nucléotidiques établies comme SEQ ID N° : 2 et 3, et
(b) une chaîne légère codée par une molécule d'acide nucléique comprenant la région ou les régions codante(s) d'une séquence nucléotidique sélectionnée parmi le groupe constitué des séquences nucléotidiques établies comme SEQ ID N° : 8 et 9, ou
(c) une région variable à chaîne lourde codée par une molécule d'acide nucléique comprenant la région ou les régions codante(s) d'une séquence nucléotidique sélectionnée parmi le groupe constitué des séquences nucléotidiques établies comme SEQ ID N° : 14 et 18, et
(d) une région variable à chaîne légère codée par une molécule d'acide nucléique comprenant la région ou les régions codante(s) d'une séquence nucléotidique sélectionnée parmi le groupe constitué des séquences nucléotidiques établies comme SEQ ID N° : 16 et 20.

4. Conjugué anticorps - médicament selon la revendication 1, dans lequel l'anticorps ou le fragment de liaison à l'antigène de celui-ci est AB-PG1-XG1-006, AB-PG1-XG1-026 ou un fragment de liaison à l'antigène de ceux-ci.

5. Conjugué anticorps - médicament selon la revendication 1, dans lequel l'anticorps ou le fragment de liaison à l'antigène de celui-ci est sélectionné parmi le groupe constitué :
(a) d'anticorps comprenant une chaîne lourde codée par une molécule d'acide nucléique comprenant la région ou les régions codante(s) d'une séquence nucléotidique sélectionnée parmi le groupe constitué des séquences nucléotidiques établies comme SEQ ID N° : 2 et 3, et une chaîne légère codée par une molécule d'acide nucléique comprenant la région ou les régions codante(s) d'une séquence nucléotidique sélectionnée parmi le groupe constitué des séquences nucléotidiques établies comme SEQ ID N° : 8 et 9, et
des fragments de liaison à l'antigène de ceux-ci ;
(b) d'anticorps comprenant une chaîne lourde codée par une molécule d'acide nucléique comprenant la région ou les régions codante(s) d'une séquence nucléotidique établie comme SEQ ID N° : 2, et une chaîne légère codée par une molécule d'acide nucléique comprenant la région ou les régions codante(s) d'une séquence nucléotidique établie comme SEQ ID N° : 8, et
des fragments de liaison à l'antigène de ceux-ci ; et
(c) d'anticorps comprenant une chaîne lourde codée par une molécule d'acide nucléique comprenant la région ou les régions codante(s) d'une séquence nucléotidique établie comme SEQ ID N° : 3, et une chaîne légère codée par une molécule d'acide nucléique comprenant la région ou les régions codante(s) d'une séquence nucléotidique établie comme SEQ ID N° : 9, et
des fragments de liaison à l'antigène de ceux-ci ; et
(d) d'anticorps comprenant une région variable à chaîne lourde codée par une molécule d'acide nucléique comprenant la région ou les régions codante(s) d'une séquence nucléotidique sélectionnée parmi le groupe constitué des séquences nucléotidiques établies comme SEQ ID N° : 14 et 18, et une région variable à chaîne légère codée par une molécule d'acide nucléique comprenant la région ou les régions codante(s) d'une séquence nucléotidique sélectionnée parmi le groupe constitué des séquences nucléotidiques établies comme SEQ ID N° : 16 et 20, et
des fragments de liaison à l'antigène de ceux-ci ; et
(e) d'anticorps comprenant une région variable à chaîne lourde codée par une molécule d'acide nucléique comprenant la région ou les régions codante(s) d'une séquence nucléotidique établie comme SEQ ID N° : 14, et une région variable à chaîne légère codée par une molécule d'acide nucléique comprenant la région ou les régions codante(s) d'une séquence nucléotidique établie comme SEQ ID N° : 16, et
des fragments de liaison à l'antigène de ceux-ci ; et
(f) d'anticorps comprenant une région variable à chaîne lourde codée par une molécule d'acide nucléique comprenant la région ou les régions codante(s) d'une séquence nucléotidique établie comme SEQ ID N° : 18, et une région variable à chaîne légère codée par une molécule d'acide nucléique comprenant la région ou les régions codante(s) d'une séquence nucléotidique établie comme SEQ ID N° : 20, et
des fragments de liaison à l'antigène de ceux-ci.

6. Conjugué anticorps - médicament selon la revendication 1, dans lequel l'anticorps ou le fragment de liaison à l'antigène de celui-ci est IgG1, IgG2, IgG3, IgG4, IgM, IgA1, IgA2, IgAsec, IgD, IgE ou présente une constante d'immunoglobuline et / ou un domaine variable d'IgG1, IgG2, IgG3, IgG4, IgM, IgA1, IgA2, IgAsec, IgD ou IgE.

7. Conjugué anticorps - médicament selon la revendication 1, dans lequel l'anticorps est un anticorps monoclonal, humanisé, humain, recombinant, chimère, bispécifique ou multispécifique.

8. Conjugué anticorps - médicament selon la revendication 1, dans lequel le fragment de liaison à l'antigène est un fragment Fab, un fragment F(ab')₂, un fragment Fv, ou un fragment contenant CDR-3.

9. Conjugué anticorps - médicament selon la revendication 1, dans lequel la sélectivité des cellules PC3 pour C4-2 ou des cellules LNCaP est d'au moins 500, 1000, 2500, 6000 ou 13 000.

10. Conjugué anticorps - médicament selon la revendication 1, dans lequel la monométhylauristatine noréphédrine ou la monométhylauristatine phénylalanine est conjuguée à l'anticorps ou au fragment de liaison à l'antigène de celui-ci avec un composé de formule :
-Aₙ-Yₘ-Zₘ-Xₙ-Wₙ-
dans laquelle, A est une unité acyle carboxylique ; Y est un acide aminé ; Z est un acide aminé ; X et W sont chacun un espaceur auto-sacrificiel ; n est un entier de 0 ou 1 ; et m est un entier de 0 ou 1, 2, 3, 4, 5 ou 6.

11. Conjugué anticorps - médicament selon la revendication 10, dans lequel A est
(a) 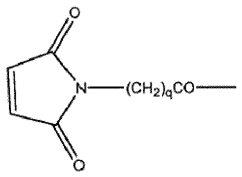 dans laquelle q est 1 - 10,
(b) 4-(N-succinimidométhyl)cyclohexane-1-carbonyle,
(c) m-succinimidobenzoyle,
(d) 4-(p-succinimidophényl)-butyryle,
(e) 4-(2-acétamido)benzoyle,
(f) 3-thiopropionyle,
(g) 4-(1-thioéthyl)-benzoyle,
(h) 6-(3-thiopropionylamido)-hexanoyle, ou
(i) caproyle maléimidique.

12. Conjugué anticorps - médicament selon la revendication 10, dans lequel Y est de l'alanine, de la valine, de la leucine, de l'isoleucine, de la méthionine, de la phénylalanine, du tryptophane ou de la proline.

13. Conjugué anticorps - médicament selon la revendication 10, dans lequel Z est de la lysine, de la lysine protégée avec de l'acétyle ou du formyle, de l'arginine, de l'arginine protégée avec des groupes tosyle ou nitro, de l'histidine, de l'ornithine, de l'ornithine protégée avec de l'acétyle ou du formyle, ou de la citrulline.

14. Conjugué anticorps - médicament selon la revendication 10, dans lequel Yₘ - Zₘ est de la valine - citrulline.

15. Conjugué anticorps - médicament selon la revendication 10, dans lequel Yₘ - Zₘ est une séquence protéinée qui est sélectivement clivable par protéase.

16. Conjugué anticorps - médicament selon la revendication 10, dans lequel X est :
(a) un composé ayant la formule 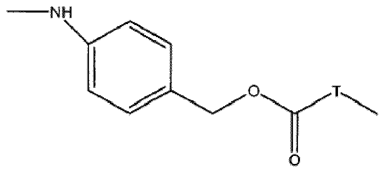 dans laquelle T est O, N ou S ;
(b) un composé ayant la formule -HN-R¹-COT dans laquelle R¹ est alkyle en C₁ - C₅, T est O, N ou S ;
(c) un composé ayant la formule 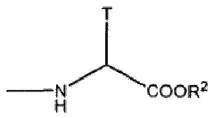 dans laquelle T est O, N, ou S, R² est H ou alkyle en C₁ - C₅ ;
(d) p-aminobenzylcarbamoyloxy ;
(e) p-aminobenzylalcool ;
(f) p-aminobenzylcarbamate ;
(g) p-aminobenzyloxycarbonyle ; ou
(h) γ-acide aminobutyrique ; α,α-diméthyle γ-acide aminobutyrique ou β,β-diméthyle γ-acide aminobutyrique.

17. Conjugué anticorps - médicament selon la revendication 10, dans lequel W est : 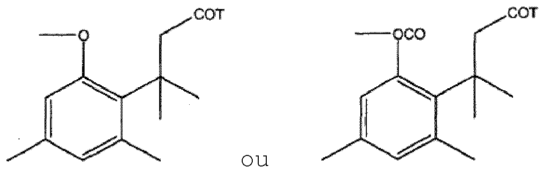 dans lesquelles T est O, S ou N.

18. Conjugué anticorps - médicament selon l'une quelconque des revendications 10 à 17, dans lequel m et n sont 0.

19. Conjugué anticorps - médicament selon la revendication 1, qui est AB-PG1-XG1-006-caproyle maléimidique-valine-citrulline-p-aminobenzyloxycarbonyle-monométhylauristatine noréphédrine, AB-PG1-XG1-006-caproyle maléimidique-valine-citrulline-p-aminobenzyloxycarbonyle-monométhylauristatine phénylalanine, AB-PG1-XG1-006-caproyle maléimidique-monométhylauristatine phénylalanine, AB-PG1-XG1-006-caproyle maléimidique-valine-citrulline-p-aminobenzylcarbamate-monométhylauristatine noréphédrine, AB-PG1-XG1-006-caproyle maléimidique-valine-citrulline-p-aminobenzylcarbamate-monomethylauristatine phénylalanine, AB-PG1-XG1-026-caproyle maléimidique-valine-citrulline-p-aminobenzyloxycarbonyle-monométhylauristatine noréphédrine, AB-PG1-XG1-026-caproyle maléimidique-valine-citrulline-p-aminobenzyloxycarbonyle-monométhylauristatine phénylalanine, ou AB-PG1-XG1-026-caproyle maléimidique-monométhylauristatine phénylalanine.

20. Conjugué anticorps - médicament selon la revendication 1, dans lequel le conjugué anticorps - médicament se lie à une cellule de tumeur, de préférence une cellule de tumeur de prostate.

21. Conjugué anticorps - médicament selon la revendication 1, dans lequel le conjugué anticorps - médicament se lie à des cellules endothéliales de la néovasculature d'une tumeur, ne requiert pas de lyse cellulaire pour se lier à PSMA, mène à un arrêt du cycle cellulaire, et / ou inhibe la croissance des cellules exprimant PSMA.

22. Conjugué anticorps - médicament selon la revendication 1, dans lequel le conjugué anticorps - médicament médie la destruction cellulaire spécifique des cellules exprimant PSMA avec une CI₅₀ de :
(a) moins de 1 X 10⁻¹⁰, 1 X 10⁻¹¹ ou 1 X 10⁻¹²M ;
(b) 11-, 42-, 60- ou 65 - 208 X 10⁻¹²M ; ou
(c) 11, 42, 60 ou 83 X 10⁻¹²M.

23. Conjugué anticorps - médicament selon la revendication 1, dans lequel le conjugué anticorps - médicament quand administré à des souris avec un régime de q4d x 6 à une dose de 6 mg / kg déclenche un taux de guérison d'au moins 20 %, 30 %, 40 % ou 50 %.

24. Conjugué anticorps - médicament selon la revendication 1, lié à un marqueur, de préférence un marqueur fluorescent, un marqueur enzymatique, un marqueur radioactif, un marqueur actif à résonnance magnétique nucléaire, un marqueur luminescent ou un marqueur chromophore.

25. Conjugué anticorps - médicament selon la revendication 1, emballé sous une forme lyophilisée ou aqueuse.

26. Conjugué anticorps - médicament selon la revendication 1, sous une forme stérile.

27. Composition comprenant :
(a) le conjugué anticorps - médicament selon la revendication 1 et un support, excipient ou stabilisant pharmaceutiquement acceptable ; ou
(b) une combinaison de deux conjugués anticorps - médicament différents ou plus selon la revendication 1 et d'un support, excipient ou stabilisant pharmaceutiquement acceptable.

28. Composition selon la revendication 27, comprenant en outre un agent antitumoral, un agent immunostimulateur, un immunomodulateur, un corticostéroïde ou une combinaison de ceux-ci.

29. Composition selon la revendication 28, dans laquelle l'agent antitumoral est un agent cytotoxique, un agent qui agit sur la néovasculature de la tumeur ou une combinaison de ceux-ci, de préférence du docétaxel.

30. Composition selon la revendication 28, dans laquelle l'immunomodulateur est une cytokine, une chimiokine, un adjuvant ou une combinaison de ceux-ci.

31. Composition selon la revendication 28, dans laquelle l'agent immunostimulateur est l'interleukine-2, α-interféron, γ-interféron, le facteur α de nécrose tumorale, un oligonucléotide immunostimulateur ou une combinaison de ceux-ci.

32. Composition selon la revendication 28, dans laquelle le corticostéroïde est la prédnisone ou l'hydrocortisone.

33. Composition comprenant :
un ou plusieurs conjugués anticorps - médicament selon la revendication 1 et un ou plusieurs anticorps anti-PSMA non conjugués.

34. Conjugué anticorps - médicament selon l'une quelconque des revendications 1 à 23, à utiliser dans un procédé d'inhibition de la croissance d'une cellule exprimant PSMA, dans lequel le conjugué est à mettre en contact avec la cellule exprimant PSMA.

35. Conjugué anticorps - médicament selon la revendication 34, dans lequel la cellule exprimant PSMA est une cellule de tumeur de prostate, une cellule de la néovasculature d'une tumeur autre que de la prostate, une cellule dépendante d'un androgène ou une cellule indépendante d'un androgène.

36. Conjugué anticorps - médicament selon la revendication 34, dans lequel, dans ledit procédé, la cellule exprimant PSMA est en outre à mettre en contact avec un agent antitumoral, un agent immunostimulateur, un immunomodulateur, un corticostéroïde ou une combinaison de ceux-ci.

37. Procédé ex-vivo pour inhiber la croissance d'une cellule exprimant PSMA, comprenant l'étape consistant à mettre en contact la cellule exprimant PSMA avec une quantité d'un conjugué anticorps - médicament selon l'une quelconque des revendications 1 à 23, efficace pour inhiber la croissance de la cellule exprimant PSMA.

38. Procédé selon la revendication 37, dans lequel la cellule exprimant PSMA est une cellule de tumeur de prostate, une cellule de la néovasculature d'une tumeur autre que de la prostate, une cellule dépendante d'un androgène ou une cellule indépendante d'un androgène.

39. Procédé selon la revendication 37, comprenant en outre l'étape consistant à mettre en contact la cellule exprimant PSMA avec un agent antitumoral, un agent immunostimulateur, un immunomodulateur, un corticostéroïde ou une combinaison de ceux-ci.

40. Conjugué anticorps - médicament selon l'une quelconque des revendications 1 à 23, à utiliser dans un procédé de déclenchement de l'arrêt du cycle cellulaire dans une cellule exprimant PSMA, dans lequel le conjugué doit être mis en contact avec la cellule exprimant PSMA.

41. Conjugué anticorps - médicament selon l'une quelconque des revendications 1 à 23, à utiliser dans un procédé de traitement du cancer, éventuellement du cancer de la prostate ou d'un cancer autre que celui de la prostate, et ledit cancer autre que celui de la prostate, éventuellement, est un cancer de la vessie, un cancer du pancréas, un cancer du poumon, un cancer du rein, un sarcome, un cancer du sein, un cancer du cerveau, un carcinome neuroendocrinien, un cancer du côlon, un cancer du testicule ou un mélanome.

42. Conjugué anticorps - médicament à utiliser dans un procédé de traitement du cancer selon la revendication 41, dans lequel, dans ledit procédé, un autre agent thérapeutique doit être co-administré pour traiter le cancer, dans lequel la co-administration doit être faite avant, pendant ou après l'administration du conjugué anticorps - médicament.

43. Conjugué anticorps - médicament à utiliser dans un procédé de traitement du cancer selon la revendication 42, dans lequel l'autre agent thérapeutique est un agent antitumoral, un agent immunostimulateur, un immunomodulateur, un corticostéroïde ou une combinaison de ceux-ci.

44. Conjugué anticorps - médicament à utiliser dans un procédé d'inhibition de la croissance d'une cellule exprimant PSMA selon la revendication 36, conjugué anticorps - médicament à utiliser dans un procédé de traitement du cancer selon la revendication 43, ou procédé selon la revendication 39, dans lequel l'agent antitumoral est un agent cytotoxique, un agent qui agit sur la néovasculature de la tumeur ou une combinaison de ceux-ci, et de préférence, du docétaxel.

45. Conjugué anticorps - médicament à utiliser dans un procédé d'inhibition de la croissance d'une cellule exprimant PSMA selon la revendication 36, conjugué anticorps - médicament à utiliser dans un procédé de traitement du cancer selon la revendication 43, ou procédé selon la revendication 39, dans lequel l'immunomodulateur est une cytokine, une chimiokine, un adjuvant ou une combinaison de ceux-ci.

46. Conjugué anticorps - médicament à utiliser dans un procédé d'inhibition de la croissance d'une cellule exprimant PSMA selon la revendication 36, conjugué anticorps - médicament à utiliser dans un procédé de traitement du cancer selon la revendication 43, ou procédé selon la revendication 39, dans lequel l'agent immunostimulateur est l'interleukine-2, α-interféron, γ-interféron, le facteur α de nécrose tumorale, un oligonucléotide immunostimulateur ou une combinaison de ceux-ci.

47. Conjugué anticorps - médicament à utiliser dans un procédé d'inhibition de la croissance d'une cellule exprimant PSMA selon la revendication 36, conjugué anticorps - médicament à utiliser dans un procédé de traitement du cancer selon la revendication 43, ou procédé selon la revendication 39, dans lequel le corticostéroïde est la prédnisone ou l'hydrocortisone.

48. Conjugué anticorps - médicament à utiliser dans un procédé de traitement du cancer selon la revendication 42, dans lequel l'agent thérapeutique est un vaccin, de préférence un vaccin qui immunise contre PSMA.

49. Conjugué anticorps - médicament à utiliser dans un procédé d'inhibition de la croissance d'une cellule exprimant PSMA selon la revendication 44, ou conjugué anticorps - médicament à utiliser dans un procédé de traitement du cancer selon l'une quelconque des revendications 42 - 44, dans lequel, dans ledit procédé, encore un autre agent thérapeutique, de préférence la prédnisone, doit être administré.

50. Conjugué anticorps - médicament selon l'une quelconque des revendications 1 à 23, à utiliser dans un procédé d'inhibition de la croissance d'une tumeur, dans lequel ledit conjugué doit être mis en contact avec des cellules exprimant PSMA de la néovasculature de la tumeur, pour ainsi inhiber la croissance de la tumeur.

51. Conjugué anticorps - médicament à utiliser dans un procédé d'inhibition de la croissance d'une tumeur selon la revendication 50, dans lequel, dans ledit procédé, les cellules exprimant PSMA doivent être mises davantage en contact avec un agent antitumoral, un agent immunostimulateur, un immunomodulateur, un corticostéroïde ou une combinaison de ceux-ci.

52. Conjugué anticorps - médicament à utiliser dans un procédé d'inhibition de la croissance d'une tumeur selon la revendication 51, dans lequel :
(a) l'agent antitumoral est un agent cytotoxique, un agent qui agit sur la néovasculature de la tumeur ou une combinaison de ceux-ci ;
(b) l'immunomodulateur est une cytokine, une chimiokine, un adjuvant ou une combinaison de ceux-ci ; et / ou
(c) l'agent immunostimulateur est l'interleukine-2, α-interféron, γ-interféron, le facteur α de nécrose tumorale, des oligonucléotides immunostimulateurs ou une combinaison de ceux-ci.
